(19)

**Europäisches Patentamt**
**European Patent Office**
**Office européen des brevets**

(11)  **EP 3 921 444 B1**

(12)  # EUROPEAN PATENT SPECIFICATION

(45) Date of publication and mention
of the grant of the patent:
**09.10.2024 Bulletin 2024/41**

(21) Application number: **20752248.3**

(22) Date of filing: **05.02.2020**

(51) International Patent Classification (IPC):
**C12Q 1/6886** (2018.01)     **C12Q 1/6832** (2018.01)

(52) Cooperative Patent Classification (CPC):
(C-Sets available)
**G16B 40/20; C12Q 1/6886; G16B 20/00;**
**G16B 25/20; G16B 30/00;** C12Q 1/6806;
C12Q 1/6834; C12Q 2600/154; C12Q 2600/16

(Cont.)

(86) International application number:
**PCT/US2020/016684**

(87) International publication number:
**WO 2020/163410 (13.08.2020 Gazette 2020/33)**

(54) **DETECTING CANCER, CANCER TISSUE OF ORIGIN, AND/OR A CANCER CELL TYPE**

NACHWEIS VON KREBS, URSPRUNGSKREBSGEWEBE, UND/ODER EINES KREBSZELLENTYPS

DÉTECTION D'UN CANCER, D'UN TISSU CANCÉREUX D'ORIGINE ET/OU D'UN TYPE DE CELLULE CANCÉREUSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.02.2019   US 201962801556 P**
**05.02.2019   US 201962801561 P**
**24.01.2020   US 202062965327 P**
**24.01.2020   US 202062965342 P**
**24.01.2020   PCT/US2020/015082**
**04.02.2020   PCT/US2020/016673**

(43) Date of publication of application:
**15.12.2021   Bulletin 2021/50**

(73) Proprietor: **Grail, Inc.**
**Menlo Park, California 94025 (US)**

(72) Inventors:
• **VENN, Oliver Claude**
**Menlo Park, California 94025 (US)**
• **FIELDS, Alexander P.**
**Menlo Park, California 94025 (US)**
• **GROSS, Samuel S.**
**Menlo Park, California 94025 (US)**
• **LIU, Qinwen**
**Menlo Park, California 94025 (US)**
• **SCHELLENBERGER, Jan**
**Menlo Park, California 94025 (US)**
• **BREDNO, Joerg**
**Menlo Park, California 94025 (US)**
• **BEAUSANG, John F.**
**Menlo Park, California 94025 (US)**
• **SHOJAEE, Seyedmehdi**
**Menlo Park, California 94025 (US)**
• **SAKARYA, Onur**
**Menlo Park, California 94025 (US)**
• **MAHER, M. Cyrus**
**Menlo Park, California 94025 (US)**
• **JAMSHIDI, Arash**
**Menlo Park, California 94025 (US)**

(74) Representative: **Murgitroyd & Company**
**165-169 Scotland Street**
**Glasgow G5 8PL (GB)**

(56) References cited:
**WO-A1-2014/026768     WO-A1-2020/163403**
**WO-A2-2020/154682     US-A1- 2007 161 031**
**US-A1- 2013 129 668**

- **LIU L. ET AL: "Targeted methylation sequencing of plasma cell-free DNA for cancer detection and classification", ANNALS OF ONCOLOGY, vol. 29, no. 6, 1 June 2018 (2018-06-01), NL, pages 1445 - 1453, XP055910054, ISSN: 0923-7534, Retrieved from the Internet <URL:https://www.ncbi.nlm.nih.gov/pmc/article s/PMC6005020/pdf/mdy119.pdf> DOI: 10.1093/annonc/mdy119**
- **ILLUMINA: "Data Sheet: Epigenetics Infinium MethylationEPIC BeadChip", 19 October 2015 (2015-10-19), XP055833502, Retrieved from the Internet <URL:https://filgen.jp/Product/Bioscience/Meth yl/humanmethylationepic-data-sheet-1070-2015- 008.pdf> [retrieved on 20210819]**
- **PIDSLEY RUTH ET AL: "Critical evaluation of the Illumina MethylationEPIC BeadChip microarray for whole-genome DNA methylation profiling", GENOME BIOLOGY, vol. 17, no. 1, 1 December 2016 (2016-12-01), XP055932440, Retrieved from the Internet <URL:https://www.ncbi.nlm.nih.gov/pmc/article s/PMC5055731/pdf/13059_2016_Article_1066.pd f> DOI: 10.1186/s13059-016-1066-1**

- **DATABASE GenBank [online] 25 June 2002 (2002-06-25), XP055728937, Database accession no. AC093151.2**
- **DATABASE 0 [online] 7 March 2003 (2003-03-07), GENBANK ET AL., XP055728940, Database accession no. AC067721**

Remarks:
The complete document including Reference Table(s) and the Sequence Listing(s) can be downloaded from the EPO website

(52) Cooperative Patent Classification (CPC): (Cont.)

C-Sets
C12Q 1/6806, C12Q 2535/122, C12Q 2537/159;
C12Q 1/6834, C12Q 2535/122, C12Q 2537/159

**Description**

**BACKGROUND**

DNA methylation plays an important role in regulating gene expression. Aberrant DNA methylation has been implicated in many disease processes, including cancer. DNA methylation profiling using methylation sequencing (e.g., whole genome bisulfite sequencing (WGBS)) is increasingly recognized as a valuable diagnostic tool for detection, diagnosis, and/or monitoring of cancer. For example, specific patterns of differentially methylated regions may be useful as molecular markers for various diseases.

However, WGBS is not ideally suitable for a product assay. The reason is that the vast majority of the genome is either not differentially methylated in cancer, or the local CpG density is too low to provide a robust signal. Only a few percent of the genome is likely to be useful in classification.

Furthermore, there have been various challenges in identifying differentially methylated regions in various diseases. First off, determining differentially methylated regions in a disease group only holds weight in comparison with a group of control subjects, such that if the control group is small in number, the determination loses confidence with the small control group. Additionally, among a group of control subjects, methylation status can vary which can be difficult to account for when determining whether the regions are differentially methylated in a disease group. On another note, methylation of a cytosine at a CpG site is strongly correlated with methylation at a subsequent CpG site. To encapsulate this dependency is a challenge in itself.

Accordingly, a cost-effective method of accurately diagnosing a disease by detecting differentially methylated regions has not yet been available.

Liu, L. et al "Targeted methylation sequencing of plasma cell-free DNA for cancer detection and classification", ANNALS OF ONCOLOGY, vol. 29, no. 6, 1 June 2018 (2018-06-01), discusses the development of a methylation sequencing assay targeting 9223 CpG sites consistently hypermethylated according to The Cancer Genome Atlas. The study carried out a clinical validation of the method using plasma cfDNA samples from 78 patients with advanced colorectal cancer, non-small-cell lung cancer (NSCLC), breast cancer or melanoma and compared results with patients' outcomes.

Pidsley, R. et al "Critical evaluation of the Illumina MethylationEPIC BeadChip microarray for whole-genome DNA methylation profiling", Genome Biology (2016) 17:208 discusses using the Illumina HumanMethylation450 (HM450) BeadChip platform with whole-genome bisulphite sequencing (WGBS) to perform a critical evaluation of the Illumina MethylationEPIC BeadChip microarray platform.

**SUMMARY**

The scope of the present invention is recited in the appended claims, where there is described:
A method of detecting cells of a cancer type in a subject, the method comprising:

(i) capturing cell-free DNA (cfDNA) fragments from the subject or amplification products thereof with a composition comprising a plurality of different bait oligonucleotides, wherein:

(a) each bait oligonucleotide in the plurality of different bait oligonucleotides has a length of at least 45 nucleotides, optionally 45 to 300 nucleotides;
(b) the bait oligonucleotides collectively hybridize to at least 100 target genomic regions that are differentially methylated in a first cancer type of at least 10 cancer types, relative to a different cancer type of the at least 10 cancer types or relative to non-cancer;

(ii) sequencing the captured cfDNA fragments or amplification products thereof to produce sequencing reads, wherein the cfDNA is converted prior to sequencing by treating the cfDNA to convert unmethylated cytosines to uracils, and
(iii) for each of the at least 10 cancer types, applying a trained classifier to the sequencing reads, wherein the classifier (a) is constrained to the at least 100 target genomic regions of the bait oligonucleotides for the first cancer type, and (b) assigns a score for each of the at least 10 cancer types; wherein the trained classifier is a classifier trained using converted DNA sequences, wherein converted DNA relates to DNA that has been treated to convert unmethylated cytosines to uracils, and
(iv) detecting the cells of the cancer type in the subject as the cancer type assigned the highest score;

wherein the at least 10 cancer types are selected from anorectal cancer, bladder cancer, urothelial cancer, breast cancer, cervical cancer, colorectal cancer, head & neck cancer, liver cancer, bile duct cancer, lung cancer, ovarian cancer, pancreatic cancer, gallbladder cancer, prostate cancer, renal cancer, sarcoma, thyroid cancer, upper GI cancer, and uterine cancer.

Also described herein, but not claimed, are compositions comprising a plurality of different bait oligonucleotides, wherein the plurality of different bait oligonucleotides are configured to collectively hybridize to DNA molecules derived from at least 100 target genomic regions and wherein each genomic region of the at least 100 target genomic regions is differentially methylated in at least one cancer type relative to another cancer type or relative to a non-cancer type. In some arrangements, the at least 100 target genomic regions comprise at least one, at least 5, at least 10, at least 20, at least 50, or at least 100 target genomic regions that are differentially methylated in at least a first cancer type relative to a second cancer type and relative to a non-cancer type. In some arrangements, the at least 100 target genomic regions comprise at least one target genomic region that is differentially methylated in the first cancer type relative to two or more, three or more, four or more, five or more, or ten or more, twelve or more, or fifteen or more other cancer types. In some arrangements, the at least 100 target genomic regions comprise, for all possible pairs between the one cancer type and at least 10, at least 12, at least 15 or at least 18 other cancer types or the non-cancer type, at least one target genomic region that is differentially methylated between the pair of cancer types.

In some arrangements, the plurality of bait oligonucleotides are configured to hybridize to DNA molecules derived from at least 20% of the target genomic regions of any one of Lists 1-49. In some arrangements, the plurality of bait oligonucleotides are configured to hybridize to DNA molecules derived from at least 20% of the target genomic regions of Lists 1-49. In some arrangements, the plurality of bait oligonucleotides are configured to hybridize to DNA molecules derived from at least 20% or at least 40% of the target genomic regions of any one of Lists 1-15. In some arrangements, the plurality of bait oligonucleotides are configured to hybridize to DNA molecules derived from at least 20% or at least 40% of the target genomic regions of Lists 1-15. In some arrangements, the plurality of bait oligonucleotides are configured to hybridize to DNA molecules derived from at least 20% the target genomic regions of any one of Lists 16-32. In some arrangements, the plurality of bait oligonucleotides are configured to hybridize to DNA molecules derived from at least 20% of the target genomic regions of Lists 16-32. In some arrangements, the plurality of bait oligonucleotides are configured to hybridize to DNA molecules derived from at least 20% of the target genomic regions of any one of Lists 33-49. In some arrangements, the plurality of bait oligonucleotides are configured to hybridize to DNA molecules derived from at least 20% of the target genomic regions of Lists 33-49.

Described herein, but not claimed, are compositions comprising a plurality of different bait oligonucleotides configured to hybridize to DNA molecules derived from at least 20% of the target genomic regions of any one of Lists 1-49. In some arrangements, the plurality of bait oligonucleotides are configured to hybridize to DNA molecules derived from at least 20% of the target genomic regions of Lists 1-49. In some arrangements, the plurality of bait oligonucleotides are configured to hybridize to DNA molecules derived from at least 20% or at least 40% of the target genomic regions of any one of Lists 1-15. In some arrangements, the plurality of bait oligonucleotides are configured to hybridize to DNA molecules derived from at least 20% or at least 40% of the target genomic regions of Lists 1-15. In some arrangements, the plurality of bait oligonucleotides are configured to hybridize to DNA molecules derived from at least 20% the target genomic regions of any one of Lists 16-32. In some arrangements, the plurality of bait oligonucleotides are configured to hybridize to DNA molecules derived from at least 20% of the target genomic regions of Lists 16-32. In some arrangements, the plurality of bait oligonucleotides are configured to hybridize to DNA molecules derived from at least 20% of the target genomic regions of any one of Lists 33-49. In some arrangements, the plurality of bait oligonucleotides are configured to hybridize to DNA molecules derived from at least 20% of the target genomic regions of Lists 33-49.

In some arrangements, the plurality of different bait oligonucleotides are configured to hybridize to DNA molecules derived from at least 20% of the target genomic regions of List 1. In some arrangements, the DNA molecules are derived from at least 30%, 40%, 50%, 60%, 70%, or 80% of the target genomic regions of List 1.

In some arrangements, the plurality of different bait oligonucleotides are configured to hybridize to DNA molecules derived from at least 20% of the target genomic regions of List 2. In some arrangements, the DNA molecules are derived from at least 30%, 40%, 50%, 60%, 70%, or 80% of the target genomic regions of List 2.

In some arrangements, the plurality of different bait oligonucleotides are configured to hybridize to DNA molecules derived from at least 20% of the target genomic regions of List 3. In some arrangements, the DNA molecules are derived from at least 30%, 40%, 50%, 60%, 70%, or 80% of the target genomic regions of List 3.

In some arrangements, the plurality of different bait oligonucleotides are configured to hybridize to DNA molecules derived from at least 20% of the target genomic regions of List 4. In some arrangements, the DNA molecules are derived from at least 30%, 40%, 50%, 60%, 70%, or 80% of the target genomic regions of List 4.

In some arrangements, the plurality of different bait oligonucleotides are configured to hybridize to DNA molecules derived from at least 20% of the target genomic regions of List 5. In some arrangements, the DNA molecules are derived from at least 30%, 40%, 50%, 60%, 70%, or 80% of the target genomic regions of List 5.

In some arrangements, the plurality of different bait oligonucleotides are configured to hybridize to DNA molecules derived from at least 20% of the target genomic regions of List 6. In some arrangements, the DNA molecules are derived from at least 30%, 40%, 50%, 60%, 70%, or 80% of the target genomic regions of List 6.

In some arrangements, the plurality of different bait oligonucleotides are configured to hybridize to DNA molecules derived from at least 20% of the target genomic regions of List 7. In some arrangements, the DNA molecules are derived from at least 30%, 40%, 50%, 60%, 70%, or 80% of the target genomic regions of List 7.

In some arrangements, the plurality of different bait oligonucleotides are configured to hybridize to DNA molecules derived from at least 20% of the target genomic regions of List 8. In some arrangements, the DNA molecules are derived from at least 30%, 40%, 50%, 60%, 70%, or 80% of the target genomic regions of List 8.

In some arrangements, the plurality of different bait oligonucleotides are configured to hybridize to DNA molecules derived from at least 20% of the target genomic regions of List 9. In some arrangements, the DNA molecules are derived from at least 30%, 40%, 50%, 60%, 70%, or 80% of the target genomic regions of List 9.

In some arrangements, the plurality of different bait oligonucleotides are configured to hybridize to DNA molecules derived from at least 20% of the target genomic regions of List 10. In some arrangements, the DNA molecules are derived from at least 30%, 40%, 50%, 60%, 70%, or 80% of the target genomic regions of List 10.

In some arrangements, the plurality of different bait oligonucleotides are configured to hybridize to DNA molecules derived from at least 20% of the target genomic regions of List 11. In some arrangements, the DNA molecules are derived from at least 30%, 40%, 50%, 60%, 70%, or 80% of the target genomic regions of List 11.

In some arrangements, the plurality of different bait oligonucleotides are configured to hybridize to DNA molecules derived from at least 20% of the target genomic regions of List 12. In some arrangements, the DNA molecules are derived from at least 30%, 40%, 50%, 60%, 70%, or 80% of the target genomic regions of List 12.

In some arrangements, the plurality of different bait oligonucleotides are configured to hybridize to DNA molecules derived from at least 20% of the target genomic regions of List 13. In some arrangements, the DNA molecules are derived from at least 30%, 40%, 50%, 60%, 70%, or 80% of the target genomic regions of List 13.

In some arrangements, the plurality of different bait oligonucleotides are configured to hybridize to DNA molecules derived from at least 20% of the target genomic regions of List 14. In some arrangements, the DNA molecules are derived from at least 30%, 40%, 50%, 60%, 70%, or 80% of the target genomic regions of List 14.

In some arrangements, the plurality of different bait oligonucleotides are configured to hybridize to DNA molecules derived from at least 20% of the target genomic regions of List 15. In some arrangements, the DNA molecules are derived from at least 30%, 40%, 50%, 60%, 70%, or 80% of the target genomic regions of List 15.

In some arrangements, the plurality of different bait oligonucleotides are configured to hybridize to DNA molecules derived from at least 20% of the target genomic regions of List 16. In some arrangements, the DNA molecules are derived from at least 30%, 40%, 50%, 60%, 70%, or 80% of the target genomic regions of List 16.

In some arrangements, the plurality of different bait oligonucleotides are configured to hybridize to DNA molecules derived from at least 20% of the target genomic regions of List 17. In some arrangements, the DNA molecules are derived from at least 30%, 40%, 50%, 60%, 70%, or 80% of the target genomic regions of List 17.

In some arrangements, the plurality of different bait oligonucleotides are configured to hybridize to DNA molecules derived from at least 20% of the target genomic regions of List 18. In some arrangements, the DNA molecules are derived from at least 30%, 40%, 50%, 60%, 70%, or 80% of the target genomic regions of List 18.

In some arrangements, the plurality of different bait oligonucleotides are configured to hybridize to DNA molecules derived from at least 20% of the target genomic regions of List 19. In some arrangements, the DNA molecules are derived from at least 30%, 40%, 50%, 60%, 70%, or 80% of the target genomic regions of List 19.

In some arrangements, the plurality of different bait oligonucleotides are configured to hybridize to DNA molecules derived from at least 20% of the target genomic regions of List 20. In some arrangements, the DNA molecules are derived from at least 30%, 40%, 50%, 60%, 70%, or 80% of the target genomic regions of List 20.

In some arrangements, the plurality of different bait oligonucleotides are configured to hybridize to DNA molecules derived from at least 20% of the target genomic regions of List 21. In some arrangements, the DNA molecules are derived from at least 30%, 40%, 50%, 60%, 70%, or 80% of the target genomic regions of List 21.

In some arrangements, the plurality of different bait oligonucleotides are configured to hybridize to DNA molecules derived from at least 20% of the target genomic regions of List 22. In some arrangements, the DNA molecules are derived from at least 30%, 40%, 50%, 60%, 70%, or 80% of the target genomic regions of List 22.

In some arrangements, the plurality of different bait oligonucleotides are configured to hybridize to DNA molecules derived from at least 20% of the target genomic regions of List 23. In some arrangements, the DNA molecules are derived from at least 30%, 40%, 50%, 60%, 70%, or 80% of the target genomic regions of List 23.

In some arrangements, the plurality of different bait oligonucleotides are configured to hybridize to DNA molecules derived from at least 20% of the target genomic regions of List 24. In some arrangements, the DNA molecules are derived from at least 30%, 40%, 50%, 60%, 70%, or 80% of the target genomic regions of List 24.

In some arrangements, the plurality of different bait oligonucleotides are configured to hybridize to DNA molecules derived from at least 20% of the target genomic regions of List 25. In some arrangements, the DNA molecules are derived from at least 30%, 40%, 50%, 60%, 70%, or 80% of the target genomic regions of List 25.

In some arrangements, the plurality of different bait oligonucleotides are configured to hybridize to DNA molecules derived from at least 20% of the target genomic regions of List 26. In some arrangements, the DNA molecules are derived from at least 30%, 40%, 50%, 60%, 70%, or 80% of the target genomic regions of List 26.

In some arrangements, the plurality of different bait oligonucleotides are configured to hybridize to DNA molecules derived from at least 20% of the target genomic regions of List 27. In some arrangements, the DNA molecules are derived from at least 30%, 40%, 50%, 60%, 70%, or 80% of the target genomic regions of List 27.

In some arrangements, the plurality of different bait oligonucleotides are configured to hybridize to DNA molecules derived from at least 20% of the target genomic regions of List 28. In some arrangements, the DNA molecules are derived from at least 30%, 40%, 50%, 60%, 70%, or 80% of the target genomic regions of List 28.

In some arrangements, the plurality of different bait oligonucleotides are configured to hybridize to DNA molecules derived from at least 20% of the target genomic regions of List 29. In some arrangements, the DNA molecules are derived from at least 30%, 40%, 50%, 60%, 70%, or 80% of the target genomic regions of List 29.

In some arrangements, the plurality of different bait oligonucleotides are configured to hybridize to DNA molecules derived from at least 20% of the target genomic regions of List 30. In some arrangements, the DNA molecules are derived from at least 30%, 40%, 50%, 60%, 70%, or 80% of the target genomic regions of List 30.

In some arrangements, the plurality of different bait oligonucleotides are configured to hybridize to DNA molecules derived from at least 20% of the target genomic regions of List 31. In some arrangements, the DNA molecules are derived from at least 30%, 40%, 50%, 60%, 70%, or 80% of the target genomic regions of List 31.

In some arrangements, the plurality of different bait oligonucleotides are configured to hybridize to DNA molecules derived from at least 20% of the target genomic regions of List 32. In some arrangements, the DNA molecules are derived from at least 30%, 40%, 50%, 60%, 70%, or 80% of the target genomic regions of List 32.

In some arrangements, the plurality of different bait oligonucleotides are configured to hybridize to DNA molecules derived from at least 20% of the target genomic regions of List 33. In some arrangements, the DNA molecules are derived from at least 30%, 40%, 50%, 60%, 70%, or 80% of the target genomic regions of List 33.

In some arrangements, the plurality of different bait oligonucleotides are configured to hybridize to DNA molecules derived from at least 20% of the target genomic regions of List 34. In some arrangements, the DNA molecules are derived from at least 30%, 40%, 50%, 60%, 70%, or 80% of the target genomic regions of List 34.

In some arrangements, the plurality of different bait oligonucleotides are configured to hybridize to DNA molecules derived from at least 20% of the target genomic regions of List 35. In some arrangements, the DNA molecules are derived from at least 30%, 40%, 50%, 60%, 70%, or 80% of the target genomic regions of List 35.

In some arrangements, the plurality of different bait oligonucleotides are configured to hybridize to DNA molecules derived from at least 20% of the target genomic regions of List 36. In some arrangements, the DNA molecules are derived from at least 30%, 40%, 50%, 60%, 70%, or 80% of the target genomic regions of List 36.

In some arrangements, the plurality of different bait oligonucleotides are configured to hybridize to DNA molecules derived from at least 20% of the target genomic regions of List 37. In some arrangements, the DNA molecules are derived from at least 30%, 40%, 50%, 60%, 70%, or 80% of the target genomic regions of List 37.

In some arrangements, the plurality of different bait oligonucleotides are configured to hybridize to DNA molecules derived from at least 20% of the target genomic regions of List 38. In some arrangements, the DNA molecules are derived from at least 30%, 40%, 50%, 60%, 70%, or 80% of the target genomic regions of List 38.

In some arrangements, the plurality of different bait oligonucleotides are configured to hybridize to DNA molecules derived from at least 20% of the target genomic regions of List 39. In some arrangements, the DNA molecules are derived from at least 30%, 40%, 50%, 60%, 70%, or 80% of the target genomic regions of List 39.

In some arrangements, the plurality of different bait oligonucleotides are configured to hybridize to DNA molecules derived from at least 20% of the target genomic regions of List 40. In some arrangements, the DNA molecules are derived from at least 30%, 40%, 50%, 60%, 70%, or 80% of the target genomic regions of List 40.

In some arrangements, the plurality of different bait oligonucleotides are configured to hybridize to DNA molecules derived from at least 20% of the target genomic regions of List 41. In some arrangements, the DNA molecules are derived from at least 30%, 40%, 50%, 60%, 70%, or 80% of the target genomic regions of List 41.

In some arrangements, the plurality of different bait oligonucleotides are configured to hybridize to DNA molecules derived from at least 20% of the target genomic regions of List 42. In some arrangements, the DNA molecules are derived from at least 30%, 40%, 50%, 60%, 70%, or 80% of the target genomic regions of List 42.

In some arrangements, the plurality of different bait oligonucleotides are configured to hybridize to DNA molecules derived from at least 20% of the target genomic regions of List 43. In some arrangements, the DNA molecules are derived from at least 30%, 40%, 50%, 60%, 70%, or 80% of the target genomic regions of List 43.

In some arrangements, the plurality of different bait oligonucleotides are configured to hybridize to DNA molecules derived from at least 20% of the target genomic regions of List 44. In some arrangements, the DNA molecules are derived from at least 30%, 40%, 50%, 60%, 70%, or 80% of the target genomic regions of List 44.

In some arrangements, the plurality of different bait oligonucleotides are configured to hybridize to DNA molecules derived from at least 20% of the target genomic regions of List 45. In some arrangements, the DNA molecules are derived from at least 30%, 40%, 50%, 60%, 70%, or 80% of the target genomic regions of List 45.

In some arrangements, the plurality of different bait oligonucleotides are configured to hybridize to DNA molecules derived from at least 20% of the target genomic regions of List 46. In some arrangements, the DNA molecules are derived from at least 30%, 40%, 50%, 60%, 70%, or 80% of the target genomic regions of List 46.

In some arrangements, the plurality of different bait oligonucleotides are configured to hybridize to DNA molecules derived from at least 20% of the target genomic regions of List 47. In some arrangements, the DNA molecules are derived from at least 30%, 40%, 50%, 60%, 70%, or 80% of the target genomic regions of List 47.

In some arrangements, the plurality of different bait oligonucleotides are configured to hybridize to DNA molecules derived from at least 20% of the target genomic regions of List 48. In some arrangements, the DNA molecules are derived from at least 30%, 40%, 50%, 60%, 70%, or 80% of the target genomic regions of List 48.

In some arrangements, the plurality of different bait oligonucleotides are configured to hybridize to DNA molecules derived from at least 20% of the target genomic regions of List 49. In some arrangements, the DNA molecules are derived from at least 30%, 40%, 50%, 60%, 70%, or 80% of the target genomic regions of List 49.

In some arrangements, the plurality of different bait oligonucleotides are configured to hybridize to DNA molecules derived from at least 20% of the target genomic regions from any two or more, three or more, four or more, or five or more of Lists 16-32.

In some arrangements, the DNA molecules are derived from at least 30%, 40%, 50%, 60%, 70%, or 80% of the target genomic regions from any two or more, three or more, four or more, or five or more, six or more, seven or more, eight or more, nine or more, or ten or more of Lists 16-32.

In some arrangements, the plurality of different bait oligonucleotides are configured to hybridize to DNA molecules derived from at least 20% of the target genomic regions from any two or more, three or more, four or more, or five or more of Lists 33-49.

In some arrangements, the DNA molecules are derived from at least 30%, 40%, 50%, 60%, 70%, or 80% of the target genomic regions from any two or more, three or more, four or more, five or more, six or more, seven or more, eight or more, nine or more, or ten or more of Lists 33-49.

In some arrangements, the total size of the of the target genomic regions is less than 1100 kb, less than 750 kb, less than 270 kb, less than 200 kb, less than 150 kb, less than 100 kb, or less than 50 kb. In some arrangements, the total number of target genomic regions is less than 1700, less than 1300, less than 900, less than 700 or less than 400.

In some arrangements, the total size of the targeted genomic regions is less than 5,000 bk, 2,500 kb, less than 2,000 kb, less than 1,500 kb, less than 1,000 kb, less than 750 kb, or less than 500 kb. In some arrangements, the total number of targeted genomic regions is less than 20,000, less than 18,000, less than 16,000, less than 14,000, less than 12,000, less than 10,000, less than 8,000, less than 6,000, less than 4,000, or less than 2,000.

In some arrangements, the DNA molecules are converted cfDNA fragments. In the methods of the invention, the cfDNA molecules are converted prior to sequencing. In some arrangements, the target genomic regions are hypermethylated regions, hypomethylated regions, or binary regions that can be either hypermethylated or hypomethylated, as indicated in the sequence listing. In some arrangements, the bait oligonucleotides are configured to hybridize to hypermethylated converted DNA molecules, hypomethylated converted DNA molecules, or both hypermethylated and hypomethylated converted DNA molecules derived from each targeted genomic region, as indicated in the sequence listing.

In some arrangements, the bait oligonucleotides are each conjugated to an affinity moiety. In some arrangements, the affinity moiety is biotin. In some arrangements, the bait oligonucleotides are each conjugated to a solid surface. In some arrangements, the solid surface is a microarray or chip.

In some arrangements, the bait oligonucleotides each have a length of 45 to 300 nucleotide bases, 75-200 nucleotide bases, 100-150 nucleotide bases, or about 120 nucleotide bases. In some arrangements, the bait oligonucleotides comprise a plurality of sets of two or more bait oligonucleotides, wherein each bait oligonucleotide within a set of bait oligonucleotides is configured to bind to the same converted target genomic region or configured to bind to a nucleic acid molecule derived from the target genomic region. In some arrangements, each set of bait oligonucleotides comprises 1 or more pairs of a first bait oligonucleotide and a second bait oligonucleotide, wherein each bait oligonucleotide comprises a 5' end and a 3' end, wherein a sequence of at least X nucleotide bases at the 3' end of the first bait oligonucleotide is identical to a sequence of X nucleotide bases at the 5' end the second bait oligonucleotide, and wherein X is at least 25, 30, 35, 40, 45, 50, 60, 70, 75 or 100. In some arrangements, the first bait oligonucleotide comprises a sequence of at least 31, 40, 50 or 60 nucleotide bases that does not overlap a sequence of the second bait oligonucleotide.

In some arrangements, the composition further comprises converted cfDNA from a test subject. In some arrangements, the cfDNA from the test subject is converted by a process comprising treatment with bisulfite or a cytosine deaminase.

Described herein, but not claimed in isolation, are methods of enriching cfDNA fragments informative of a type of cancer, the method comprising: contacting any one of the bait oligonucleotide compositions described herein with DNA derived from a test subject, and enriching the sample for cfDNA corresponding to genomic regions associated with the type of cancer by hybridization capture.

Described herein, but not claimed in isolation, are methods for obtaining sequence information informative of a presence or absence of a type of cancer, the method comprising (a) enriching converted DNA from a test subject by contacting the DNA with any one of the bait oligonucleotide compositions described herein, and (b) sequencing the enriched converted DNA.

Described herein, and as described in more detail in the claims, are methods for determining that a test subject has a type of cancer, the method comprising (a) capturing cfDNA fragments from the test subject with any one of the bait oligonucleotide compositions described herein, (b) sequencing the captured cfDNA fragments, and (c) applying a trained classifier to the cfDNA sequences to determine that the test subject has the type of cancer.

Described herein, but not claimed in isolation, are methods for determining that a test subject has a type of cancer, the method comprising (a) capturing cfDNA fragments from the test subject with any one of the bait oligonucleotide compositions described herein, (b) detecting the captured cfDNA fragments by DNA microarray, and (c) applying a trained classifier to the DNA fragments hybridized to the DNA microarray to determine that the test subject has the type of cancer.

In some arrangements, the trained classifier is a mixture model classifier. In some arrangements, the classifier was trained on converted DNA sequences derived from at least 1000, at least 2000, or at least 4000 target genomic regions selected from any one of Lists 1-49.

In some arrangements, the trained classifier determines the presence or absence of cancer or a cancer type by: (i) generating a set of features for the sample, wherein each feature in the set of features comprises a numerical value; (ii) inputting the set of features into the classifier, wherein the classifier comprises a multinomial classifier; (iii) based on the set of features, determining, at the classifier, a set of probability scores, wherein the set of probability scores comprises one probability score per cancer type class and per non-cancer type class; and (iv) thresholding the set of probability scores based on one or more values determined during training of the classifier to determine a final cancer classification of the sample. In some arrangements, the set of features comprises a set of binarized features. In some arrangements, the numerical value comprises a single binary value. In some arrangements, the multinomial classifier comprises a multinomial logistic regression ensemble trained to predict a source tissue for the cancer.

In some arrangements, the method further comprises determining the final cancer classification based on a top-two probability score differential relative to a minimum value, wherein the minimum value corresponds to a predefined percentage of training cancer samples that had been assigned the correct cancer type as their highest score during training of the classifier. In some arrangements, (i) in accordance with a determination that the top-two probability score differential exceeds the minimum value, assign a cancer label corresponding to the highest probability score determined by the classifier as the final cancer classification; and (ii) in accordance with a determination that the top-two probability score differential does not exceed the minimum value, assigning an indeterminate cancer label as the final cancer classification. In some embodiments, the type of cancer is selected from the group consisting of anorectal cancer, bladder cancer, bladder and urothelial cancer, breast cancer, cervical cancer, colorectal cancer, head and neck cancer, hepatobiliary cancer, liver and bile duct cancer, lung cancer, melanoma, ovarian cancer, pancreatic cancer, pancreatic and gall bladder cancer, prostate cancer, renal cancer, sarcoma, thyroid cancer, upper GI cancer, and uterine cancer. In some embodiments, the capture cfDNA fragments are converted cfDNA fragments.

Described herein, but not claimed, are cancer assay panels comprising: at least 5 pairs of probes, wherein each pair of the at least 5 pairs comprise two probes configured to overlap each other by an overlapping sequence, wherein the overlapping sequence comprises a sequence of at least 30 nucleotides, wherein the at least 30-nucleotide sequence is configured to hybridize to a converted cfDNA molecule corresponding to, or derived from one or more of genomic regions, wherein each of the genomic regions comprises at least five methylation sites, wherein the at least five methylation sites have an abnormal methylation pattern in first cancerous samples, and wherein each probe of the of the at least 5 pairs of probes comprises a non-overlapping sequence of at least 31 nucleotides. In some arrangements, the cancer assay panels comprise at least 10, at least 20, at least 30, at least 50, at least 100, at least 200, or at least 500 pairs of probes.

In some arrangements, the genomic regions are selected from a List, and the list is List 1 and the first cancerous samples are samples from subject having bladder cancer, the list is List 2 and the first cancerous samples are samples from subject having breast cancer, the list is List 3 and the first cancerous samples are samples from subject having cervical cancer, the list is List 4 and the first cancerous samples are samples from subject having colorectal cancer, the list is List 5 and the first cancerous samples are samples from subject having head and neck cancer, the list is List 6 and the first cancerous samples are samples from subject having hepatobiliary cancer, the list is List 7 and the first cancerous samples are samples from subject having lung cancer, the list is List 8 and the first cancerous samples are samples from subject having melanoma, the list is List 9 and the first cancerous samples are samples from subject having ovarian cancer, the list is List 10 and the first cancerous samples are samples from subject having pancreatic cancer, the list is List 11 and the first cancerous samples are samples from subject having prostate cancer, the list is List 12 and the first cancerous samples are samples from subject having renal cancer, the list is List 13 and the first cancerous samples are samples from subject having thyroid cancer, the list is List 14 and the first cancerous samples are samples from subject having upper gastrointestinal cancer, or the list is List 15 and the first cancerous samples are samples from subject having uterine cancer.

In some arrangements, the genomic regions are selected from a List, and the list is List 16 or List 33 and the first cancerous samples are samples from subject having anorectal cancer, the list is List 17 or List 34 and the first cancerous samples are samples from subject having bladder or urothelial cancer, the list is List 18 or List 35 and the first cancerous samples are samples from subject having breast cancer, the list is List 19 or List 36 and the first cancerous samples are samples from subject having cervical cancer, the list is List 20 or List 37 and the first cancerous samples are samples from subject having colorectal cancer, the list is List 21 or List 38 and the first cancerous samples are samples from subject having head or neck cancer, the list is List 22 or List 39 and the first cancerous samples are samples from subject having liver or bile duct cancer, the list is List 23 or List 40 and the first cancerous samples are samples from subject having lung cancer, the list is List 24 or List 41 and the first cancerous samples are samples from subject having melanoma, the list is List 25 or List 42 and the first cancerous samples are samples from subject having ovarian cancer, the list is List 26 or List 43 and the first cancerous samples are samples from subject having pancreatic or gallbladder cancer, the list is List 27 or List 44 and the first cancerous samples are samples from subject having prostate cancer, the list is List 28 or List 45 and the first cancerous samples are samples from subject having renal cancer, or the list is List 29 or List 46 and the first cancerous samples are samples from subject having sarcoma, the list is List 30 or List 47 and the first cancerous samples are samples from subjects having thyroid cancer, the list is List 31 or List 48 and the first cancerous samples are samples from subjects having upper gastrointestinal tract cancer, or the list is List 32 or List 49 and the first cancerous samples are samples from subjects having uterine cancer.

In some arrangements, the genomic regions comprise at least 20%, 30%, 40%, 50%, 60%, 70%, 80%, 90%, 95%, or 100% of the genomic regions in the List. In some arrangements, the genomic regions comprise at least 30, **Error! Reference source not found., Error! Reference source not found.,** 159, 160, 200, 250, 300, 400, 500, 600, 800, or 1,000 genomic regions in the List. In some arrangements, the converted cfDNA molecules comprise cfDNA molecules treated to covert unmethylated C (cytosine) to U (uracil). In some arrangements, each of the at least 5 pairs of probes is conjugated to a non-nucleotide affinity moiety. In some arrangements, the non-nucleotide affinity moiety is a biotin moiety. In some arrangements, the abnormal methylation pattern has at least a threshold p-value rarity in the first cancerous samples. In some arrangements, each of the probes is designed to have sequence homology or sequence complementarity with less than 20 off-target genomic regions. In some arrangements, the less than 20 off-target genomic regions are identified using a k-mer seeding strategy. In some arrangements, the less than 20 off-target genomic regions are identified using k-mer seeding strategy combined to local alignment at seed locations. In some arrangements, each of the probes comprises at least 61, 75, 100, 120, or 121 nucleotides. In some arrangements, each of the probes comprises less than 300, 250, 200, 160 or 159 nucleotides. In some arrangements, each of the probes comprises 100-159 or 100-160 nucleotides. In some arrangements, each of the probes comprises less than 20, 15, 10, 8, or 6 methylation sites. In some arrangements, at least 80, 85, 90, 92, 95, or 98% of the at least five methylation sites are either methylated or unmethylated in the cancerous samples. In some arrangements, at least 3%, 5%, 10%, 15%, or 20% of the probes comprise no G (Guanine). In some arrangements, each of the probes comprise multiple binding sites to the methylation sites of the converted cfDNA molecule, wherein at least 80, 85, 90, 92, 95, or 98% of the multiple binding sites comprise exclusively either CpG or CpA. In some arrangements, each of the probes is configured to have sequence homology or sequence complementarity with less than 15, 10 or 8 off-target genomic regions.

In some arrangements, at least 30% of the genomic regions are in exons or introns. In some arrangements, at least 15% of the genomic regions are in exons. In some arrangements, at least 20% of the genomic regions are in exons. In some arrangements, less than 10% of the genomic regions are in intergenic regions. In some arrangements, the cancer assay panel comprises at least 100, 200, 300, 400, 500, 600, 700, 800, 900, 1000, 1,200, 1,400, 1,600, 1,800, 2,000, 2,200, 2,400, 2,600, 2,800, 3,000, 3,200, 4,000, 4,500, 5,000, 5,500, 6,000, 6,500, 7,000, 7,500, 8,000, 8,500, 9,000, 10,000, 15,000, or 20,000 probes. In some arrangements, the at least 5 pairs of probes together comprise at least 10,000, 20,000, 30,000, 40,000, 50,000, 60,000, 70,000, 80,000, 90,000, 100,000, 120,000, 140,000, 160,000, 180,000, 200,000, 240,000, 260,000, 280,000, 300,000, 320,000, 400,000, 450,000, 500,000, 550,000, 600,000, 650,000, 700,000, 750,000, 800,000, 850,000, 900,000, 1 million, 1.5 million, 2 million, 2.5 million, or 3 million, nucleotides.

Described herein are methods of detecting cancer and/or a cancer tissue of origin (TOO), comprising: (a) receiving a sample comprising a plurality of cfDNA molecules; (b) treating the plurality of cfDNA molecules to convert unmethylated C (cytosine) to U (uracil), thereby obtaining a plurality of converted cfDNA molecules; (c) applying any one of the cancer assay panels described herein to the plurality of converted cfDNA molecules, thereby enriching a subset of the converted cfDNA molecules; and (d) sequencing the enriched subset of the converted cfDNA molecule, thereby providing a set of sequence reads.

Described herein are methods of detecting cancer and/or a cancer tissue of origin (TOO), comprising: (a) receiving a sample comprising a plurality of cfDNA molecules; (b) treating the plurality of cfDNA molecules to convert unmethylated C (cytosine) to U (uracil), thereby obtaining a plurality of converted cfDNA molecules; (c) applying any one of the cancer assay panels described herein to the plurality of converted cfDNA molecules, thereby enriching a subset of the converted cfDNA molecules; and (d) detecting the enriched subset of the converted cfDNA molecule by hybridization to a DNA microarray.

In some arrangements, the method further comprises the step of: determining a health condition by evaluating the set of sequence reads, wherein the health condition is (a) a presence or absence of cancer; (b) a stage of cancer; (c) a presence or absence of a cancer tissue of origin (TOO); (d) a presence or absence of a cancer cell type; or (e) a presence or absence of at least 2, 3, 4, 5, 6, 7, 8, 9, 10, 11, 12, 13, 14, or 15 different types of cancer. In some arrangements, the sample comprising a plurality of cfDNA molecules was obtained from a human subject.

Described herein are methods for detecting cancer, comprising the steps of: (a) obtaining a set of sequence reads by sequencing a set of nucleic acid fragments from a subject, wherein each of the nucleic acid fragments correspond to, or are derived from a plurality of genomic regions selected from one or more of Lists 1 to 15; one or more of Lists 16 to 32; or one or more of Lists 33 to 49 (b) for each of the sequence reads, determining methylation status at a plurality of CpG sites; and (c) determining that cancer has been detected in the subject by evaluating the methylation status for the sequence reads, wherein the detection of cancer comprises one or more of: (i) a presence or absence of cancer; (ii) a stage of cancer; (iii) a presence or absence of a cancer tissue of origin (TOO); (iv) a presence or absence of a cancer cell type; and (v) a presence or absence of at least 2, 3, 4, 5, 6, 7, 8, 9, 10, 11, 12, 13, 14, or 15 different types of cancer.

In some arrangements, (a) the plurality of genomic regions are selected from List 1 and the detection of cancer comprises a detection of bladder cancer; (b) the plurality of genomic regions are selected from List 2 and the detection of cancer comprises a detection of breast cancer; (c) the plurality of genomic regions are selected from List 3 and the detection of cancer comprises a detection of cervical cancer; (d) the plurality of genomic regions are selected from List 4 and the detection of cancer comprises a detection of colorectal cancer; (e) the plurality of genomic regions are selected from List 5 and the detection of cancer comprises a detection of head and neck cancer; (f) the plurality of genomic regions are selected from List 6 and the detection of cancer comprises a detection of hepatobiliary cancer; (g) the plurality of genomic regions are selected from List 7 and the detection of cancer comprises a detection of lung cancer; (h) the plurality of genomic regions are selected from List 8 and the detection of cancer comprises a detection of melanoma; (i) the plurality of genomic regions are selected from List 9 and the detection of cancer comprises a detection of ovarian cancer; (j) the plurality of genomic regions are selected from List 10 and the detection of cancer comprises a detection of pancreatic cancer; (k) the plurality of genomic regions are selected from List 11 and the detection of cancer comprises a presence or detection prostate cancer; (l) the plurality of genomic regions are selected from List 12 and the detection of cancer comprises a detection of renal cancer; (m) the plurality of genomic regions are selected from List 13 and the detection of cancer comprises a detection of thyroid cancer; (n) the plurality of genomic regions are selected from List 14 and the detection of cancer comprises a detection of upper gastrointestinal cancer; or (o) the plurality of genomic regions are selected from List 15 and the detection of cancer comprises a detection of uterine cancer.

In some arrangements, (a) the plurality of genomic regions are selected from List 16 or List 33 and the detection of cancer comprises a detection of anorectal cancer; the plurality of genomic regions are selected from List 17 or List 34 and the detection of cancer comprises a detection of bladder or urothelial cancer; the plurality of genomic regions are selected from List 18 or List 35 and the detection of cancer comprises a detection of breast cancer; the plurality of genomic regions are selected from List 19 or List 36 and the detection of cancer comprises a detection of cervical cancer; the plurality of genomic regions are selected from List 20 or List 37 and the detection of cancer comprises a detection of colorectal cancer; the plurality of genomic regions are selected from List 21 or List 38 and the detection of cancer comprises a detection of head and neck cancer; the plurality of genomic regions are selected from List 22 or List 39 and the detection of cancer comprises a detection of liver or bile duct cancer; the plurality of genomic regions are selected from List 23 or List 40 and the detection of cancer comprises a detection of lung cancer; the plurality of genomic regions are selected from List 24 or List 41 and the detection of cancer comprises a detection of melanoma; the plurality of genomic regions are selected from List 25 or List 42 and the detection of cancer comprises a detection of ovarian cancer; the plurality of genomic regions are selected from List 26 or List 43 and the detection of cancer comprises a presence or detection pancreatic or gallbladder cancer; the plurality of genomic regions are selected from List 27 or List 44 and the detection of cancer comprises a detection of prostate cancer; the plurality of genomic regions are selected from List 28 or List 45 and the detection of cancer comprises a detection of renal cancer; the plurality of genomic regions are selected from List 29 or List 46 and the detection of cancer comprises a detection of sarcoma; the plurality of genomic regions are selected from List 30 or List 47 and the detection of cancer comprises a detection of thyroid cancer; the plurality of genomic regions are selected from List 31 or List 48 and the detection of cancer comprises a detection of upper gastrointestinal tract cancer; or the plurality of genomic regions are selected from List 32 or List 49 and the detection of cancer comprises a detection of uterine cancer.

In some arrangements, the plurality of genomic regions comprises at least 20%, 30%, 40%, 50%, 60%, 70%, 80%, 90%, 95%, or 100% of the genomic regions of the List. In some arrangements, the plurality of genomic regions comprises at least 30, 50, 100, 150, 200, 250, or 300 of the genomic regions of the List. In some arrangements, the plurality of genomic regions comprises less than 90%, 80%, 70%, 60%, 50%, 40%, 30% or 20% of the genomic regions of the List. In some arrangements, the plurality of genomic regions comprises less than 25000, 20000, 15000, 10000, 7500, 5000, or 2500 of the genomic regions of the List. In some arrangements, the plurality of genomic regions comprises less than 1000, 500, 400, 300, 200, or 100 of the genomic regions of the List.

Described herein are cancer assay panels comprising a plurality of probes, wherein each of the plurality of probes is configured to hybridize to a converted cfDNA molecule corresponding to one or more of a plurality of genomic regions selected from one or more of Lists 1 to 15. In some arrangements, the converted cfDNA molecules comprise cfDNA molecules treated to convert unmethylated cytosines to uracils. In some arrangements, wherein the plurality of probes are configured to hybridize to nucleic acid molecules corresponding to, or derived from at least 20%, 30%, 40%, 50%, 60%, 70%, 80%, 90%, 95%, or 100% of the genomic regions of a List and the List is one or more of Lists 1 to 15. In some arrangements, the plurality of probes are configured to hybridize to nucleic acid molecules corresponding to, or derived from at least 30, 50, 100, 159, 171, 200, 250, 300, 400, 500, 600, 800, or 1,000 of the genomic regions of a List and the List is one or more of Lists 1 to 15. In some arrangements, at least 3%, 5%, 10%, 15%, or 20% of the probes comprise no G (Guanine). In some arrangements, each of the probes comprise multiple binding sites to methylation sites of the converted cfDNA molecule, wherein at least 80, 85, 90, 92, 95, or 98% of the multiple binding sites comprise exclusively either CpG or CpA. In some arrangements, each of the probes is conjugated to a non-nucleotide affinity moiety. In some arrangements, the non-nucleotide affinity moiety is a biotin moiety.

Described herein are methods of determining a presence or absence of cancer in a subject, the method comprising: (i) capturing cfDNA fragments from the subject with a composition comprising a plurality of different oligonucleotide baits; (ii) sequencing the captured cfDNA fragments, and (iii) applying a trained classifier to the cfDNA sequences to determine the presence or absence of cancer. In some arrangements, the likelihood of a false positive determination of a presence or absence of cancer is less than 1% and the likelihood of an accurate determination of a presence or absence of cancer is at least 40%. In some arrangements, the cancer is a stage I cancer, the likelihood of a false positive determination of a presence or absence of cancer is less than 1%, and the likelihood of an accurate determination of a presence or absence of cancer is at least 9%. In some arrangements, the cfDNA fragments are converted cfDNA fragments.

Described herein are methods of detecting a cancer type comprising: (i) capturing cfDNA fragments from a subject with a composition comprising a plurality of different oligonucleotide baits, (ii) sequencing the captured cfDNA fragments, and (iii) applying a trained classifier to the cfDNA sequences to determine a cancer type; wherein the oligonucleotide baits are configured to hybridize to cfDNA fragments derived from a plurality of target genomic regions, wherein the plurality of target genomic regions is differentially methylated in one or more cancer types relative to a different cancer type or a non-cancer type, wherein the likelihood of a false-positive determination of cancer is less than 1%, and wherein the likelihood of an accurate assignment of a cancer type is at least 75%, at least 80%, at least 85% or at least 89%, or at least 90%. In some arrangements, the method further comprises applying the trained classifier to the cfDNA sequences to determine a presence of cancer before determining the cancer type. In some arrangements, the cfDNA fragments are converted cfDNA fragments.

In some arrangements, the cancer type is selected from uterine cancer, upper GI squamous cancer, all other upper GI cancers, thyroid cancer, sarcoma, urothelial renal cancer, all other renal cancers, prostate cancer, pancreatic cancer, ovarian cancer, neuroendocrine cancer, multiple myeloma, melanoma, lymphoma, small cell lung cancer, lung adenocarcinoma, all other lung cancers, leukemia, hepatobiliary carcinoma, hepatobiliary biliary, head and neck cancer, colorectal cancer, cervical cancer, breast cancer, bladder cancer, and anorectal cancer. In some arrangements, the cancer type is selected from anal cancer, bladder cancer, colorectal cancer, esophageal cancer, head and neck cancer, liver/bile-duct cancer, lung cancer, lymphoma, ovarian cancer, pancreatic cancer, plasma cell neoplasm, and stomach cancer. In some arrangements, the cancer type is selected from thyroid cancer, melanoma, sarcoma, myeloid neoplasm, renal cancer, prostate cancer, breast cancer, uterine cancer, ovarian cancer, bladder cancer, urothelial cancer, cervical cancer, anorectal cancer, head & neck cancer, colorectal cancer, liver cancer, bile duct cancer, pancreatic cancer, gallbladder cancer, upper GI cancer, multiple myeloma, lymphoid neoplasm, and lung cancer.

In some arrangements, the cancer type is a stage I cancer type, and the likelihood of an accurate assignment is at least 70% or at least 75%. In some arrangements, the cancer type is a stage II cancer type, and the likelihood of an accurate assignment is at least 85%.

In some arrangements, the cancer type is anorectal cancer, the target genomic regions are selected from Lists 16 or 33, and the accuracy of detecting anorectal cancer among samples with detected cancer is at least 80% or 88%. In some arrangements, the cancer type is stage I or stage II anorectal cancer, the target genomic regions are selected from Lists 16 or 33, and the accuracy of detecting stage I or stage II anorectal cancer among samples with detected cancer is at least 75% or 85%.

In some arrangements, the cancer type is bladder & urothelial cancer, the target genomic regions are selected from Lists 1, 17 or 34, and the accuracy of detecting bladder & urothelial cancer among samples with detected cancer is at least 80% or 90%. In some arrangements, the cancer type is stage I or stage II bladder & urothelial cancer, the target genomic regions are selected from Lists 1, 17 or 34, and the accuracy of stage I or stage II detecting bladder & urothelial cancer among samples with detected cancer is at least 75% or 85%.

In some arrangements, the cancer type is breast cancer, the target genomic regions are selected from Lists 2, 18 or 35, and the accuracy of detecting breast cancer among samples with detected cancer is at least 80% or 88%. In some arrangements, the cancer type is stage I or stage II breast cancer, the target genomic regions are selected from Lists 2, 18 or 35, and the accuracy of detecting stage I or stage II breast cancer among samples with detected cancer is at least 75% or 84%.

In some arrangements, the cancer type is cervical cancer, the target genomic regions are selected from Lists 3, 19 or 36, and the accuracy of detecting cervical cancer among samples with detected cancer is at least 80% or 88%. In some arrangements, the cancer type is stage I or stage II cervical cancer, the target genomic regions are selected from Lists 3, 19 or 36, and the accuracy of detecting stage I or stage II cervical cancer among samples with detected cancer is at least 75% or 85%.

In some arrangements, the cancer type is colorectal cancer, the target genomic regions are selected from Lists 4, 20 or 37, and the accuracy of detecting colorectal cancer among samples with detected cancer is at least 80% or 88%. In some arrangements, the cancer type is stage I or stage II colorectal cancer, the target genomic regions are selected from Lists 4, 20 or 37, and the accuracy of detecting stage I or stage II colorectal cancer among samples with detected cancer is at least 75% or 85%.

In some arrangements, the cancer type is head & neck cancer, the target genomic regions are selected from Lists 5, 21 or 38, and the accuracy of detecting head & neck cancer among samples with detected cancer is at least 80% or 88%. In some arrangements, the cancer type is stage I or stage II head & neck cancer, the target genomic regions are selected from Lists 5, 21 or 38, and the accuracy of detecting stage I or stage II head & neck cancer among samples with detected cancer is at least 75% or 85%.

In some arrangements, the cancer type is liver & bile duct cancer, the target genomic regions are selected from Lists 6, 22, or 39, and the accuracy of detecting liver & bile duct cancer among samples with detected cancer is at least 80% or 88%. In some arrangements, the cancer type is stage I or stage II liver & bile duct cancer, the target genomic regions are selected from Lists 6, 22, or 39, and the accuracy of detecting stage I or stage II liver & bile duct cancer among samples with detected cancer is at least 75% or 85%.

In some arrangements, the cancer type is lung cancer, the target genomic regions are selected from Lists 7, 23 or 40, and the accuracy of detecting lung cancer among samples with detected cancer is at least 80% or 88%. In some arrangements, the cancer type is stage I or stage II lung cancer, the target genomic regions are selected from Lists 7, 23 or 40, and the accuracy of detecting stage I or stage II lung cancer among samples with detected cancer is at least 75% or 85%.

In some arrangements, the cancer type is melanoma, the target genomic regions are selected from Lists 8, 24 or 41, and the accuracy of detecting melanoma among samples with detected cancer is at least 80% or 88%. In some arrangements, the cancer type is stage I or stage II melanoma, the target genomic regions are selected from Lists 8, 24 or 41, and the accuracy of detecting stage I or stage II melanoma among samples with detected cancer is at least 75% or 84%.

In some arrangements, the cancer type is ovarian cancer, the target genomic regions are selected from Lists 9, 25 or 42, and the accuracy of detecting ovarian cancer among samples with detected cancer is at least 80% or 88%. In some arrangements, the cancer type is stage I or stage II ovarian cancer, the target genomic regions are selected from Lists 9, 25 or 42, and the accuracy of detecting stage I or stage II ovarian cancer among samples with detected cancer is at least 75% or 85%.

In some arrangements, the cancer type is pancreas & gallbladder cancer, the target genomic regions are selected from Lists 10, 26 or 43, and the accuracy of detecting pancreas & gallbladder cancer among samples with detected cancer is at least 80% or 88%. In some arrangements, the cancer type is stage I or stage II pancreas & gallbladder cancer, the target genomic regions are selected from Lists 10, 26 or 43, and the accuracy of detecting stage I or stage II pancreas & gallbladder cancer among samples with detected cancer is at least 75%, 81% or 83%.

In some arrangements, the cancer type is prostate cancer, the target genomic regions are selected from Lists 11, 27 or 44, and the accuracy of detecting prostate cancer among samples with detected cancer is at least 80% or 88%. In some arrangements, the cancer type is stage I or stage II prostate cancer, the target genomic regions are selected from Lists 11, 27 or 44, and the accuracy of detecting stage I or stage II prostate cancer among samples with detected cancer is at least 75% or 83%.

In some arrangements, the cancer type is renal cancer, the target genomic regions are selected from Lists 12, 28 or 45, and the accuracy of detecting renal cancer among samples with detected cancer is at least 80% or 88%. In some arrangements, the cancer type is stage I or stage II renal cancer, the target genomic regions are selected from Lists 12, 28 or 45, and the accuracy of detecting stage I or stage II renal cancer among samples with detected cancer is at least 75% or 85%.

In some arrangements, the cancer type is sarcoma, the target genomic regions are selected from Lists 29 or 46, and the accuracy of detecting sarcoma among samples with detected cancer is at least 80% or 88%. In some arrangements, the cancer type is stage I or stage II sarcoma, the target genomic regions are selected from Lists 29 or 46, and the accuracy of detecting stage I or stage II sarcoma among samples with detected cancer is at least 75% or 83%.

In some arrangements, the cancer type is thyroid cancer, the target genomic regions are selected from Lists 13, 30 or 47, and the accuracy of detecting thyroid cancer among samples with detected cancer is at least 80% or 88%. In some arrangements, the cancer type is stage I or stage II thyroid cancer, the target genomic regions are selected from Lists 13, 30 or 47, and the accuracy of detecting stage I or stage II thyroid cancer among samples with detected cancer is at least 75% or 87%.

In some arrangements, the cancer type is upper gastrointestinal tract cancer, the target genomic regions are selected from Lists 14, 31 or 48, and the accuracy of detecting upper gastrointestinal tract cancer among samples with detected cancer is at least 80% or 88%. In some arrangements, the cancer type is stage I or stage II upper gastrointestinal tract cancer, the target genomic regions are selected from Lists 14, 31 or 48, and the accuracy of detecting stage I or stage II upper gastrointestinal tract cancer among samples with detected cancer is at least 75% or 83%.

In some arrangements, the cancer type is uterine cancer, the target genomic regions are selected from Lists 15, 32 or 49, and the accuracy of detecting uterine cancer among samples with detected cancer is at least 80% or 88%. In some arrangements, the cancer type is stage I or stage II uterine cancer, the target genomic regions are selected from Lists 16 or 33, and the accuracy of detecting stage I or stage II uterine cancer among samples with detected cancer is at least 75% or 85%.

In some arrangements, the cancer type is anorectal cancer, the target genomic regions are selected from Lists 16 or 33, and the sensitivity for anorectal cancer is at least 65% or 75%. In some arrangements, the cancer type is stage I or stage II anorectal cancer, the target genomic regions are selected from Lists 16 or 33, and the sensitivity for stage I or stage II anorectal cancer is at least 65% or 55%.

In some arrangements, the cancer type is bladder & urothelial cancer, the target genomic regions are selected from Lists 1, 17 or 34, and the sensitivity for bladder & urothelial cancer is at least 50% or 40%. In some arrangements, the cancer type is stage I or stage II bladder & urothelial cancer, the target genomic regions are selected from Lists 1, 17 or 34, and the accuracy of stage I or stage II detecting bladder & urothelial cancer is at least 40% or 50%.

In some arrangements, the cancer type is breast cancer, the target genomic regions are selected from Lists 2, 18 or 35, and the sensitivity for breast cancer is at least 20% or 25%. In some arrangements, the cancer type is stage I or stage II breast cancer, the target genomic regions are selected from Lists 2, 18 or 35, and the sensitivity for stage I or stage II breast cancer is at least 15% or 18%.

In some arrangements, the cancer type is cervical cancer, the target genomic regions are selected from Lists 3, 19 or 36, and the sensitivity for cervical cancer is at least 25% or 35%. In some arrangements, the cancer type is stage I or stage II cervical cancer, the target genomic regions are selected from Lists 3, 19 or 36, and the sensitivity for stage I or stage II cervical cancer is at least 17% or 22%.

In some arrangements, the cancer type is colorectal cancer, the target genomic regions are selected from Lists 4, 20 or 37, and the sensitivity for colorectal cancer is at least 55% or 65%. In some arrangements, the cancer type is stage I or stage II colorectal cancer, the target genomic regions are selected from Lists 4, 20 or 37, and the sensitivity for stage I or stage II colorectal cancer is at least 25%, 29% or 34%.

In some arrangements, the cancer type is head & neck cancer, the target genomic regions are selected from Lists 5, 21 or 38, and the sensitivity for head & neck cancer is at least 70% or 80%. In some arrangements, the cancer type is stage I or stage II head & neck cancer, the target genomic regions are selected from Lists 5, 21 or 38, and the sensitivity for stage I or stage II head & neck cancer is at least 70% or 79%.

In some arrangements, the cancer type is liver & bile duct cancer, the target genomic regions are selected from Lists 6, 22, or 39, and the sensitivity for liver & bile duct cancer is at least 75% or 85%. In some arrangements, the cancer type is stage I or stage II liver & bile duct cancer, the target genomic regions are selected from Lists 6, 22, or 39, and the sensitivity for stage I or stage II liver & bile duct cancer is at least 65% or 75%.

In some arrangements, the cancer type is lung cancer, the target genomic regions are selected from Lists 7, 23 or 40, and the sensitivity for lung cancer is at least 55% or 62%. In some arrangements, the cancer type is stage I or stage II lung cancer, the target genomic regions are selected from Lists 7, 23 or 40, and the sensitivity for stage I or stage II lung cancer is at least 20% or 25%.

In some arrangements, the cancer type is melanoma, the target genomic regions are selected from Lists 8, 24 or 41, and the sensitivity for melanoma is at least 40% or 30%.

In some arrangements, the cancer type is ovarian cancer, the target genomic regions are selected from Lists 9, 25 or 42, and the sensitivity for ovarian cancer is at least 70% or 80%.

In some arrangements, the cancer type is pancreas & gallbladder cancer, the target genomic regions are selected from Lists 10, 26 or 43, and the sensitivity for pancreas & gallbladder cancer is at least 60%, 70% or 74%. In some arrangements, the cancer type is stage I or stage II pancreas & gallbladder cancer, the target genomic regions are selected from Lists 10, 26 or 43, and the sensitivity for stage I or stage II pancreas & gallbladder cancer is at least 40% or 50%.

In some arrangements, the cancer type is sarcoma, the target genomic regions are selected from Lists 29 or 46, and the sensitivity for sarcoma is at least 40% or 50%.

In some arrangements, the cancer type is upper gastrointestinal tract cancer, the target genomic regions are selected from Lists 14, 31 or 48, and the sensitivity for upper gastrointestinal tract cancer is at least 70% or 60%. In some arrangements, the cancer type is stage I or stage II upper gastrointestinal tract cancer, the target genomic regions are selected from Lists 14, 31 or 48, and the sensitivity for stage I or stage II upper gastrointestinal tract cancer is at least 35% or 45%.

In some arrangements, the composition comprising oligonucleotide baits is the composition of any one of the compositions described herein or any one of the cancer assay panels described herein. In some arrangements, the plurality of genomic regions comprises no more than 1700, 1300, 900, 700 or 400 genomic regions. In some arrangements, the total size of the plurality of genomic regions is less than 4 MB, less than 2 MB, less than 1100 kb, less than 750 kb, less than 270 kb, less than 200 kb, less than 150 kb, less than 100 kb, or less than 50 kb. In some arrangements, the subject has an elevated risk of one or more cancer types. In some arrangements, the subject manifests symptoms associated with one or more cancer types. In some arrangements, the subject has not been diagnosed with a cancer.

In some arrangements, the classifier was trained on converted DNA sequences derived from a least 100 subjects with a first cancer type, at least 100 subjects with a second cancer type, and at least 100 subjects with no cancer. In some arrangements, the first cancer type is ovarian cancer. In some arrangements, the first cancer type is colorectal cancer. In some arrangements, the first cancer type is selected from thyroid cancer, melanoma, sarcoma, myeloid neoplasm, renal cancer, prostate cancer, breast cancer, uterine cancer, ovarian cancer, bladder cancer, urothecal cancer, cervical cancer, anorectal cancer head & neck cancer, colorectal cancer, liver cancer, pancreatic cancer, gallbladder cancer, esophageal cancer, stomach cancer, multiple myeloma, lymphoid neoplasm, lung cancer, or leukemia. In some arrangements, the classifier was trained on converted DNA sequences derived from at least 1000, at least 2000, or at least 4000 target genomic regions selected from any one of Lists 1-49.

In some arrangements, the trained classifier determines the presence or absence of cancer or a cancer type by: (i) generating a set of features for the sample, wherein each feature in the set of features comprises a numerical value; (ii) inputting the set of features into the classifier, wherein the classifier comprises a multinomial classifier; (iii) based on the set of features, determining, at the classifier, a set of probability scores, wherein the set of probability scores comprises one probability score per cancer type class and per non-cancer type class; and (iv) thresholding the set of probability scores based on one or more values determined during training of the classifier to determine a final cancer classification of the sample. In some arrangements, the set of features comprises a set of binarized features. In some arrangements, the numerical value comprises a single binary value. In some arrangements, the multinomial classifier comprises a multinomial logistic regression ensemble trained to predict a source tissue for the cancer. In some arrangements, the method further comprises determining the final cancer classification based on a top-two probability score differential relative to a minimum value, wherein the minimum value corresponds to a predefined percentage of training cancer samples that had been assigned the correct cancer type as their highest score during training of the classifier.

In some arrangements, (i) in accordance with a determination that the top-two probability score differential exceeds the minimum value, assign a cancer label corresponding to the highest probability score determined by the classifier as the final cancer classification; and (ii) in accordance with a determination that the top-two probability score differential does not exceed the minimum value, assigning an indeterminate cancer label as the final cancer classification.

Described herein, but not claimed, are methods of treating a type of cancer in a subject in need thereof, the method comprising: (i) detecting the type of cancer by any of the method described herein, and (ii) administering an anti-cancer therapeutic agent to the subject. In some arrangements, the anti-cancer agent is a chemotherapeutic agent selected from the group consisting of alkylating agents, antimetabolites, anthracyclines, anti-tumor antibiotics, cytoskeletal disruptors (taxans), topoisomerase inhibitors, mitotic inhibitors, corticosteroids, kinase inhibitors, nucleotide analogs, and platinum-based agents.

## BRIEF DESCRIPTION OF THE DRAWINGS

A better understanding of the features and advantages of the present disclosure will be obtained by reference to the following detailed description that sets forth illustrative arrangements, in which the principles of the disclosure are utilized, and the accompanying drawings of which:

**FIG. 1A** illustrates a 2x tiled probe design, with three probes targeting a small target region, where each base in a target region (boxed in the dotted rectangle) is covered by at least two probes.
**FIG. 1B** illustrates a 2x tiled probe design, with more than three probes targeting a larger target region, where each base in a target region (boxed in the dotted rectangle) is covered by at least two probes.

**FIG. 1C** illustrates probe design targeting hypomethylated and/or hypermethylated fragments in genomic regions.

**FIG. 2** illustrates a process of generating a cancer assay panel.

**FIG. 3A** is a flowchart describing a process of creating a data structure for a control group.

**FIG. 3B** is a flowchart describing an additional step of validating the data structure for the control group of **FIG. 3A.**

**FIG. 4** is a flowchart describing a process for selecting genomic regions for designing probes for a cancer assay panel.

**FIG. 5** is an illustration of an example p-value score calculation.

**FIG. 6A** is a flowchart describing a process of training a classifier based on hypomethylated and hypermethylated fragments indicative of cancer.

**FIG. 6B** is a flowchart describing a process of identifying fragments indicative of cancer determined by probabilistic models.

**FIG. 7A** is a flowchart describing a process of sequencing a fragment of cell-free (cf) DNA.

**FIG. 7B** is an illustration of the process of **FIG. 7A** of sequencing a fragment of cell-free (cf) DNA to obtain a methylation state vector.

**FIG. 8A** illustrates extent of bisulfite conversion (upper panel) and mean coverage/sequencing depth (lower panel) across varying stages of cancer.

**FIG. 8B** illustrates concentration of cfDNA per sample across varying stages of cancer.

**FIG. 9** is a graph of the amounts of DNA fragments binding to probes depending on the sizes of overlaps between the DNA fragments and the probes.

**FIG. 10A** illustrates a flowchart of devices for sequencing nucleic acid samples. **FIG. 10B** illustrates an analytic system that analyzes methylation status of cfDNA.

**FIG. 11** is a color-coded graph presenting numbers of genomic regions selected for differentiating each target TOO (x-axis) from a contrast TOO (y-axis).

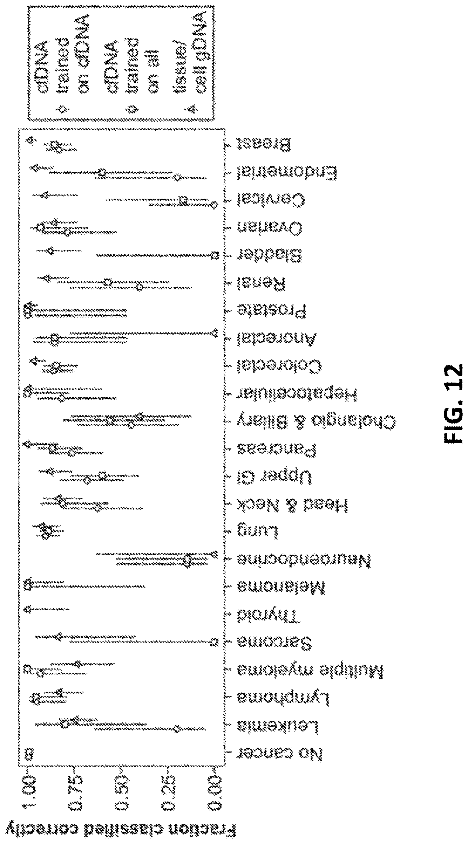

**FIG. 12** provides data for verifying selected genomic regions using cfDNA and WBG gDNA. Fractions (y-axis) classifying each TOO (x-axis) correctly are provided.

**FIG. 13** is a receiver operator curve comparing the true positive rate and false positive rate of cancer detection by a trained classifier utilizing methylation status information from the target genomic regions of list 23 (optimized for lung cancer).

## DETAILED DESCRIPTION

### Definitions

Unless defined otherwise, all technical and scientific terms used herein have the meaning commonly understood by a person skilled in the art to which this description belongs. As used herein, the following terms have the meanings ascribed to them below.

As used herein any reference to "one embodiment" or "an embodiment" means that a particular element, feature, structure, or characteristic described in connection with the embodiment is included in at least one embodiment. The appearances of the phrase "in one embodiment" in various places in the specification are not necessarily all referring to the same embodiment, thereby providing a framework for various possibilities of described embodiments to function together.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In addition, use of the "a" or "an" are employed to describe elements and components of the embodiments herein. This is done merely for convenience and to give a general sense of the description. This description should be read to include one or at least one and the singular also includes the plural unless it is obvious that it is meant otherwise.

As used herein, ranges and amounts can be expressed as "about" a particular value or range. About also includes the exact amount. Hence "about 5 μg" means "about 5 μg" and also "5 μg." Generally, the term "about" includes an amount that would be expected to be within experimental error. In some arrangements, "about" refers to the number or value recited, "+" or "-" 20%, 10%, or 5% of the number or value. Additionally, ranges recited herein are understood to be shorthand for all of the values within the range, inclusive of the recited endpoints. For example, a range of 1 to 50 is understood to include any number, combination of numbers, or sub-range from the group consisting of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50.

The term "methylation" as used herein refers to a process by which a methyl group is added to a DNA molecule. For example, a hydrogen atom on the pyrimidine ring of a cytosine base can be converted to a methyl group, forming 5-methylcytosine. The term also refers to a process by which a hydroxymethyl group is added to a DNA molecule, for example by oxidation of a methyl group on the pyrimidine ring of a cytosine base. Methylation and hydroxymethylation tend to occur at dinucleotides of cytosine and guanine referred to herein as "CpG sites."

The term "methylation" can also refer to the methylation status of a CpG site. A CpG site with a 5-methylcytosine moiety is methylated. A CpG site with a hydrogen atom on the pyrimidine ring of the cytosine base is unmethylated.

In such arrangements, the wet laboratory assay used to detect methylation may vary from those described herein as is well known in the art.

The term "methylation site" as used herein refers to a region of a DNA molecule where a methyl group can be added. "CpG" sites are the most common methylation site, but methylation sites are not limited to CpG sites. For example, DNA methylation may occur in cytosines in CHG and CHH, where H is adenine, cytosine or thymine. Cytosine methylation in the form of 5-hydroxymethylcytosine may also assessed (see, e.g., WO 2010/037001 and WO 2011/127136), and features thereof, using the methods and procedures disclosed herein.

The term "CpG site" as used herein refers to a region of a DNA molecule where a cytosine nucleotide is followed by a guanine nucleotide in the linear sequence of bases along its 5' to 3' direction. "CpG" is a shorthand for 5'-C-phosphate-G-3' that is cytosine and guanine separated by only one phosphate group. Cytosines in CpG dinucleotides can be methylated to form 5-methylcytosine.

The term "CpG detection site" as used herein refers to a region in a probe that is configured to hybridize to a CpG site of a target DNA molecule. The CpG site on the target DNA molecule can comprise cytosine and guanine separated by one phosphate group, where cytosine is methylated or unmethylated. The CpG site on the target DNA molecule can comprise uracil and guanine separated by one phosphate group, where the uracil is generated by the conversion of unmethylated cytosine.

The term "UpG" is a shorthand for 5'-U-phosphate-G-3' that is uracil and guanine separated by only one phosphate group. UpG can be generated by a bisulfite treatment of a DNA that converts unmethylated cytosines to uracils. Cytosines can be converted to uracils by other methods known in the art, such as chemical modification, synthesis, or enzymatic conversion.

The term "hypomethylated" or "hypermethylated" as used herein refers to a methylation status of a DNA molecule containing multiple CpG sites (e.g., more than 3, 4, 5, 6, 7, 8, 9, 10, etc.) where a high percentage of the CpG sites (e.g., more than 80%, 85%, 90%, or 95%, or any other percentage within the range of 50%-100%) are unmethylated or methylated, respectively.

The terms "methylation state vector" or "methylation status vector" as used herein refers to a vector comprising multiple elements, where each element indicates the methylation status of a methylation site in a DNA molecule comprising multiple methylation sites, in the order they appear from 5' to 3' in the DNA molecule. For example, $< M_x, M_{x+1}, M_{x+2} >, < M_x, M_{x+1}, U_{x+2} >, ..., < U_x, U_{x+1}, U_{x+2} >$ can be methylation vectors for DNA molecules comprising three methylation sites, where M represents a methylated methylation site and U represents an unmethylated methylation site.

The term "abnormal methylation pattern" or "anomalous methylation pattern" as used herein refers to the methylation pattern of a DNA molecule or a methylation state vector that is expected to be found in a sample less frequently than a threshold value. In one arrangement provided herein, the expectedness of finding a specific methylation state vector in a healthy control group comprising healthy individuals is represented by a p-value. A low p-value score generally corresponds to a methylation state vector which is relatively unexpected in comparison to other methylation state vectors within samples from healthy individuals. A high p-value score generally corresponds to a methylation state vector which is relatively more expected in comparison to other methylation state vectors found in samples from healthy individuals in the healthy control group. A methylation state vector having a p-value lower than a threshold value (e.g., 0.1, 0.01, 0.001, 0.0001, etc.) can be defined as an abnormal/anomalous methylation pattern. Various methods known in the art can be used to calculate a p-value or expectedness of a methylation pattern or a methylation state vector. Exemplary methods provided herein involve use of a Markov chain probability that assumes methylation statuses of CpG sites to be dependent on methylation statuses of neighboring CpG sites. Alternate methods provided herein calculate the expectedness of observing a specific methylation state vector in healthy individuals by utilizing a mixture model including multiple mixture components, each being an independent-sites model where methylation at each CpG site is assumed to be independent of methylation statuses at other CpG sites.

The term "cancerous sample" as used herein refers to a sample comprising genomic DNAs from an individual diagnosed with cancer. The genomic DNAs can be, but are not limited to, cfDNA fragments or chromosomal DNAs from a subject with cancer. The genomic DNAs can be sequenced (or otherwise detected) and their methylation status can be assessed by methods known in the art, for example, bisulfite sequencing. When genomic sequences are obtained from public database (e.g., The Cancer Genome Atlas (TCGA)) or experimentally obtained by sequencing a genome of an individual diagnosed with cancer, cancerous sample can refer to genomic DNAs or cfDNA fragments having the genomic sequences. The term "cancerous samples" as a plural refers to samples comprising genomic DNAs from multiple individuals, each individual diagnosed with cancer. In various embodiments, cancerous samples from more than 100, 300, 500, 1,000, 2,000, 5,000, 10,000, 20,000, 40,000, 50,000, or more individuals diagnosed with cancer are used.

The term "non-cancerous sample" or "healthy sample" as used herein refers to a sample comprising genomic DNAs from an individual not diagnosed with cancer. The genomic DNAs can be, but are not limited to, cfDNA fragments or chromosomal DNAs from a subject without cancer. The genomic DNAs can be sequenced (or otherwise detected) and their methylation status can be assessed by methods known in the art, for example, bisulfite sequencing. When genomic sequences are obtained from public database (e.g., The Cancer Genome Atlas (TCGA)) or experimentally obtained by sequencing a genome of an individual without cancer, non-cancerous sample can refer to genomic DNAs or cfDNA fragments having the genomic sequences. The term "non-cancerous samples" as a plural refers to samples comprising genomic DNAs from multiple individuals, each individual is without cancer. In various arrangements, healthy samples from more than 100, 300, 500, 1,000, 2,000, 5,000, 10,000, 20,000, 40,000, 50,000, or more individuals without cancer are used.

The term "training sample" as used herein refers to a sample used to train a classifier described herein and/or to select one or more genomic regions for cancer detection or detecting a cancer tissue of origin or cancer cell-type. The training samples can comprise genomic DNAs or a modification there of, from one or more healthy subjects and from one or more subjects having a disease condition (e.g., cancer, a specific type of cancer, a specific stage of cancer, etc.). The genomic DNAs can be, but are not limited to, cfDNA fragments or chromosomal DNAs. The genomic DNAs can be sequenced (or otherwise detected) and their methylation status can be assessed by methods known in the art, for example, bisulfite sequencing. When genomic sequences are obtained from public database (e.g., The Cancer Genome Atlas (TCGA)) or experimentally obtained by sequencing a genome of an individual, a training sample can refer to genomic DNAs or cfDNA fragments having the genomic sequences.

The term "test sample" as used herein refers to a sample from a subject, whose health condition was, has been or will be tested using a classifier and/or an assay panel described herein. The test sample can comprise genomic DNAs or a modification there of. The genomic DNAs can be, but are not limited to, cfDNA fragments or chromosomal DNAs.

The term "target genomic region" as used herein refers to a region in a genome selected for analysis in test samples. An assay panel is generated with probes designed to hybridize to (and optionally pull down) nucleic acid fragments derived from the target genomic region or a fragment thereof. A nucleic acid fragment derived from the target genomic region refers to a nucleic acid fragment generated by degradation, cleavage, bisulfite conversion, or other processing of the DNA from the target genomic region.

Various target genomic regions are described according to their chromosomal location in the sequence listing filed herewith. The sequence listing includes the following information: (1) the chromosome on which the region is located, along with the start and stop position of the genomic region, (2) whether the region is hypo or hypermethylated in cancer (or "binary" if the both the hypomethylated and hypermethylated are informative). The chromosome numbers and the start and stop positions are provided relative to a known human reference genome, hg19. The sequence of the human reference genome, hg19, is available from Genome Reference Consortium with a reference number, GRCh37/hg19, and also available from Genome Browser provided by Santa Cruz Genomics Institute. Chromosomal DNA is double-stranded, so a target genomic region includes two DNA strands: one with the sequence provided in the listing and a second that is a reverse complement to the sequence in the listing. Probes can be designed to hybridize to one or both sequences. Optionally, probes hybridize to converted sequences resulting from, for example, treatment with sodium bisulfite.

The term "off-target genomic region" as used herein refers to a region in a genome which has not been selected for analysis in test samples, but has sufficient homology to a target genomic region to potentially be bound and pulled down by a probe designed to target the target genomic region. In one arrangement, an off-target genomic region is a genomic region that aligns to a probe along at least 45 bp with at least a 90% match rate.

The terms "converted DNA molecules," "converted cfDNA molecules," and "modified fragment obtained from processing of the cfDNA molecules" refer to DNA molecules obtained by processing DNA or cfDNA molecules in a sample for the purpose of differentiating a methylated nucleotide and an unmethylated nucleotide in the DNA or cfDNA molecules. For example, in one arrangement, the sample can be treated with bisulfite ion (e.g., using sodium bisulfite), as is well-known in the art, to convert unmethylated cytosines ("C") to uracils ("U"). In another arrangement, the conversion of unmethylated cytosines to uracils is accomplished using an enzymatic conversion reaction, for example, using a cytidine deaminase (such as APOBEC). After treatment, converted DNA molecules or cfDNA molecules include additional uracils which are not present in the original cfDNA sample. Replication by DNA polymerase of a DNA strand comprising a uracil results in addition of an adenine to the nascent complementary strand instead of the guanine normally added as the complement to a cytosine or methylcytosine.

The terms "cell free nucleic acid," "cell free DNA," or "cfDNA" refers to nucleic acid fragments that circulate in an individual's body (e.g., bloodstream) and originate from one or more healthy cells and/or from one or more cancerous cells. Additionally, cfDNA may come from other sources such as viruses, fetuses, etc.

The term "circulating tumor DNA" or "ctDNA" refers to nucleic acid fragments that originate from tumor cells, which may be released into an individual's bloodstream as result of biological processes such as apoptosis or necrosis of dying cells or actively released by viable tumor cells.

The term "fragment" as used herein can refer to a fragment of a nucleic acid molecule. For example, in one arrangement, a fragment can refer to a cfDNA molecule in a blood or plasma sample, or a cfDNA molecule that has been extracted from a blood or plasma sample. An amplification product of a cfDNA molecule may also be referred to as a "fragment." In another arrangement, the term "fragment" refers to a sequence read, or set of sequence reads, that have been processed for subsequent analysis (e.g., for in machine-learning based classification), as described herein. For example, as is well known in the art, raw sequence reads can be aligned to a reference genome and matching paired end sequence reads assembled into a longer fragment for subsequent analysis.

The term "individual" refers to a human individual. The term "healthy individual" refers to an individual presumed not to have a cancer or disease.

The term "subject" refers to an individual whose DNA is being analyzed. A subject may be a test subject whose DNA is be evaluated using a targeted panel as described herein to evaluate whether the person has cancer or another disease. A subject may also be part of a control group known not to have cancer or another disease. A subject may also be part of a cancer or other disease group known to have cancer or another disease. Control and cancer/disease groups may be used to assist in designing or validating the targeted panel.

The term "sequence reads" as used herein refers to nucleotide sequences reads from a sample. Sequence reads can be obtained through various methods provided herein or as known in the art.

The term "sequencing depth" as used herein refers to the count of the number of times a given target nucleic acid within a sample has been sequenced (e.g., the count of sequence reads at a given target region). Increasing sequencing depth can reduce required amounts of nucleic acids required to assess a disease state (e.g., cancer or cancer tissue of origin).

The term "tissue of origin" or "TOO" as used herein refers to the organ, organ group, body region or cell type that a cancer arises or originates from. The identification of a tissue of origin or cancer cell type typically allows for identification of the most appropriate next steps in the care continuum of cancer to further diagnose, stage and decide on treatment.

The term "transition" generally refers to changes in base composition from one purine to another purine, or from one pyrimidine to another pyrimidine. For instance, the following changes are transitions: C4→U, U→C, G→A, A→G, C→T, and T→C.

"An entirety of probes" of a panel or bait set or "an entirety of polynucleotide-containing probes" of a panel or bait set generally refers to all of the probes delivered with a specified panel or bait set. For instance, in some arrangements, a panel or bait set may include both (1) probes having features specified herein (e.g., probes for binding to cell-free DNA fragments corresponding to or derived from genomic regions set forth herein in one or more Lists) and (2) additional probes that do not contain such feature(s). The entirety of probes of a panel generally refers to all probes delivered with the panel or bait set, including such probes that do not contain the specified feature(s).

## Cancer assay panel

In a first arrangement, the present description provides a cancer assay panel comprising a plurality of probes or a plurality of probe pairs. The assay panels described herein can alternatively be referred to as bait sets or as compositions comprising bait oligonucleotides. The probes are specifically designed to target one or more nucleic acid molecules corresponding to, or derived from genomic regions differentially methylated between cancer and non-cancer samples, between different cancer tissue of origin (TOO) types, between different cancer cell type, or between samples of different stages of cancer, as identified by methods provided herein. In some arrangements, probes target genomic regions (or nucleic acid molecules derived therefrom) having methylation patterns specific to a cancer type, e.g., (1) bladder cancer, (2) breast cancer, (3) cervical cancer, (4) colorectal cancer, (5) head and neck cancer, (6) hepatobiliary cancer, (7) lung cancer, (8) melanoma, (9) ovarian cancer, (10) pancreatic cancer, (11) prostate cancer, (12) renal cancer, (13) thyroid cancer, (14) upper gastrointestinal cancer, or (15) uterine cancer. In some arrangements, the panel includes probes targeting genomic regions specific to a single cancer type. In some arrangements, the panel includes probes specific to 2, 3, 4, 5, 6, 7, 8, ,9, 10, 11, 12, 13, 14, 15 or more cancer types. In some arrangements, the target genomic regions are selected to maximize classification accuracy, subject to a size budget (which is determined by sequencing budget and desired depth of sequencing).

For designing the cancer assay panel, an analytics system may collect samples corresponding to various outcomes under consideration, e.g., samples known to have cancer, samples considered to be healthy, samples from a known tissue of origin, etc. The sources of the cfDNA and/or ctDNA used to select target genomic regions can vary depending on the purpose of the assay. For example, different sources may be desirable for an assay intended to diagnose cancer generally, a specific type of cancer, a cancer stage, or a tissue of origin. These samples may be processed using one or more methods known in the art to determine the methylation status of CpG sites (e.g., with whole-genome bisulfite sequencing (WGBS)), or the information may be obtained from a public database (e.g., TCGA). The analytics system may be any generic computing system with a computer processor and a computer-readable storage medium with instructions for executing the computer processor to perform any or all operations described in this present disclosure.

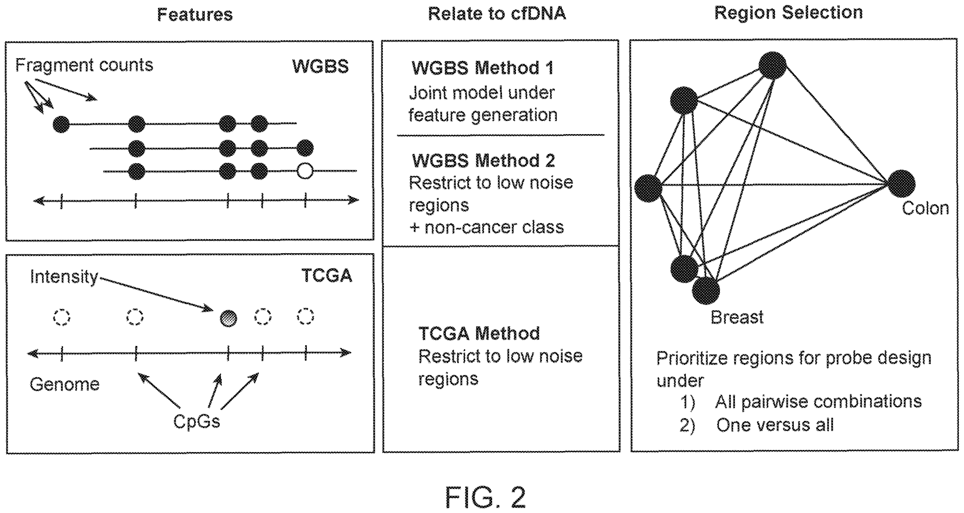

The cancer assay panel's design and utility is generally described in **FIG. 2.** For designing the cancer assay panel, an analytics system collects samples corresponding to various outcomes under consideration, e.g., samples known to have cancer, samples considered to be healthy, samples from a known TOO, etc. These samples may be processed with whole-genome bisulfite sequencing (WGBS) or obtained from public database (e.g., TCGA). The analytics system may be any generic computing system with a computer processor and a computer-readable storage medium with instructions for executing the computer processor to perform any or all operations described in this present disclosure. With the samples, the analytics system determines methylation statuses at CpG sites for each fragment in the sample.

The analytics system may then select target genomic regions for inclusion in a cancer assay panel based on methylation patterns of nucleic acid fragments. One approach considers pairwise distinguishability between pairs of outcomes (e.g., one cancer type vs. a second cancer type) for selection of targeted regions. Another approach considers distinguishability for target genomic regions when considering each outcome against the remaining outcomes (e.g., one cancer type vs. all other cancer types). From the selected target genomic regions with high distinguishability power, the analytics system may design probes to target nucleic acid fragments inclusive of, or derived from, the selected genomic regions. The analytics system may generate variable sizes of the cancer assay panel, e.g., where a small sized cancer assay panel includes probes targeting the most informative genomic region, a medium sized cancer assay panel includes probes from the small sized cancer assay panel and additional probes targeting a second tier of informative genomic regions, and a large sized cancer assay panel includes probes from the small sized and the medium sized cancer assay panels and even more probes targeting a third tier of informative genomic regions. With data obtained such cancer assay panels (e.g., the methylation status on nucleic acids derived from the cancer assay panels), the analytics system may train classifiers with various classification techniques to predict a sample's likelihood of having a particular outcome or state, e.g., cancer, specific cancer type, other disorder, other disease, etc.

Exemplary methodology for designing a cancer assay panel is generally described in **FIG. 2.** For instance, to design a cancer assay panel, an analytics system may collect information on the methylation status of CpG sites of nucleic acid fragments from samples corresponding to various outcomes under consideration, e.g., samples known to have cancer, samples considered to be healthy, samples from a known TOO, etc. These samples may be processed (e.g., with whole-genome bisulfite sequencing (WGBS)) to determine the methylation status of CpG sites, or the information may be obtained from TCGA. The analytics system may be any generic computing system with a computer processor and a computer-readable storage medium with instructions for executing the computer processor to perform any or all operations described in this present disclosure.

In some arrangements, the cancer assay panel comprises at least 500 pairs of probes, wherein each pair of the at least 500 pairs comprises two probes configured to overlap each other by an overlapping sequence, wherein the overlapping sequence comprises at least 30-nucleotides, and wherein each probe is configured to hybridize to a converted DNA (e.g., a cfDNA) molecule corresponding to one or more genomic regions. In some arrangements, each of the genomic regions comprises at least five methylation sites, and wherein the at least five methylation sites have an abnormal methylation pattern in cancerous samples or a different methylation pattern between samples of a different TOO. For example, in one arrangement, the at least five methylation sites are differentially methylated either between cancerous and non-cancerous samples or between one or more pairs of samples from cancers with different tissue of origin. In some arrangements, each pair of probes comprises a first probe and a second probe, wherein the second probe differs from the first probe. The second probe can overlap with the first probe by an overlapping sequence that is at least 30, at least 40, at least 50, or at least 60 nucleotides in length.

The target genomic regions can be selected from any one of Lists 1-49 **(TABLE 1)**. In some arrangements, the cancer assay panel comprises a plurality of probes, wherein each of the plurality of probes is configured to hybridize to a converted cfDNA molecule corresponding to one or more of the genomic regions in any one of Lists 1-49 or any combination of lists thereof. In some arrangements, the plurality of different bait oligonucleotides are configured to hybridize to DNA molecules derived from at least 20% of the target genomic regions of any one of Lists 1-49. In some arrangements, the plurality of different bait oligonucleotides are configured to hybridize to DNA molecules derived from at least 30%, 40%, 50%, 60%, 70%, or 80% of the target genomic regions of any one of Lists 1-49.

The target genomic regions can be selected from List 1. In some arrangements, a method for detecting bladder cancer comprises evaluating the methylation status for sequencing reads derived from the target genomic regions of List 1. The target genomic regions can be selected from List 2. In some arrangements, a method for detecting breast cancer comprises evaluating the methylation status for sequencing reads derived from the target genomic regions of List 2. The target genomic regions can be selected from List 3. In some arrangements, a method for detecting cervical cancer comprises evaluating the methylation status for sequencing reads derived from the target genomic regions of List 3. The target genomic regions can be selected from List 4. In some arrangements, a method for detecting colorectal cancer comprises evaluating the methylation status for sequencing reads derived from the target genomic regions of List 4. The target genomic regions can be selected from List 5. In some arrangements, a method for detecting head and neck cancer comprises evaluating the methylation status for sequencing reads derived from the target genomic regions of List 5. The target genomic regions can be selected from List 6. In some arrangements, a method for detecting hepatobiliary cancer comprises evaluating the methylation status for sequencing reads derived from the target genomic regions of List 6. The target genomic regions can be selected from List 7. In some arrangements, a method for detecting lung cancer comprises evaluating the methylation status for sequencing reads derived from the target genomic regions of List 7. The target genomic regions can be selected from List 8. In some arrangements, a method for detecting melanoma comprises evaluating the methylation status for sequencing reads derived from the target genomic regions of List 8. The target genomic regions can be selected from List 9. In some arrangements, a method for detecting ovarian cancer comprises evaluating the methylation status for sequencing reads derived from the target genomic regions of List 9. The target genomic regions can be selected from List 10. In some arrangements, a method for detecting pancreatic cancer comprises evaluating the methylation status for sequencing reads derived from the target genomic regions of List 10. The target genomic regions can be selected from List 11. In some arrangements, a method for detecting prostate cancer comprises evaluating the methylation status for sequencing reads derived from the target genomic regions of List 11. The target genomic regions can be selected from List 12. In some arrangements, a method for detecting renal cancer comprises evaluating the methylation status for sequencing reads derived from the target genomic regions of List 12. The target genomic regions can be selected from List 13. In some arrangements, a method for detecting thyroid cancer comprises evaluating the methylation status for sequencing reads derived from the target genomic regions of List 13. The target genomic regions can be selected from List 14. In some arrangements, a method for detecting upper gastrointestinal cancer comprises evaluating the methylation status for sequencing reads derived from the target genomic regions of List 14. The target genomic regions can be selected from List 15. In some arrangements, a method for detecting uterine cancer comprises evaluating the methylation status for sequencing reads derived from the target genomic regions of List 15.

The target genomic regions can be selected from List 16. In some arrangements, a method for detecting anorectal cancer comprises evaluating the methylation status for sequencing reads derived from the target genomic regions of List 16. The target genomic regions can be selected from List 17. In some arrangements, a method for detecting bladder and urothelial cancers comprises evaluating the methylation status for sequencing reads derived from the target genomic regions of List 17. The target genomic regions can be selected from List 18. In some arrangements, a method for detecting breast cancer comprises evaluating the methylation status for sequencing reads derived from the target genomic regions of List 18. The target genomic regions can be selected from List 19. In some arrangements, a method for detecting cervical cancer comprises evaluating the methylation status for sequencing reads derived from the target genomic regions of List 19. The target genomic regions can be selected from List 20. In some arrangements, a method for detecting colorectal cancer comprises evaluating the methylation status for sequencing reads derived from the target genomic regions of List 20. The target genomic regions can be selected from List 21. In some arrangements, a method for detecting head and neck cancer comprises evaluating the methylation status for sequencing reads derived from the target genomic regions of List 21. The target genomic regions can be selected from List 22. In some arrangements, a method for detecting liver and bile duct cancers comprises evaluating the methylation status for sequencing reads derived from the target genomic regions of List 22. The target genomic regions can be selected from List 23. In some arrangements, a method for detecting lung cancer comprises evaluating the methylation status for sequencing reads derived from the target genomic regions of List 23. The target genomic regions can be selected from List 24. In some arrangements, a method for detecting melanoma comprises evaluating the methylation status for sequencing reads derived from the target genomic regions of List 24. The target genomic regions can be selected from List 25. In some arrangements, a method for detecting ovarian cancer comprises evaluating the methylation status for sequencing reads derived from the target genomic regions of List 25. The target genomic regions can be selected from List 26. In some arrangements, a method for detecting pancreatic and gallbladder cancers comprises evaluating the methylation status for sequencing reads derived from the target genomic regions of List 26. The target genomic regions can be selected from List 27. In some arrangements, a method for detecting prostate cancer comprises evaluating the methylation status for sequencing reads derived from the target genomic regions of List 27. The target genomic regions can be selected from List 28. In some arrangements, a method for detecting renal cancer comprises evaluating the methylation status for sequencing reads derived from the target genomic regions of List 28. The target genomic regions can be selected from List 29. In some arrangements, a method for detecting sarcoma comprises evaluating the methylation status for sequencing reads derived from the target genomic regions of List 29. The target genomic regions can be selected from List 30. In some arrangements, a method for detecting thyroid cancer comprises evaluating the methylation status for sequencing reads derived from the target genomic regions of List 30. The target genomic regions can be selected from List 31. In some arrangements, a method for detecting upper gastrointestinal cancer comprises evaluating the methylation status for sequencing reads derived from the target genomic regions of List 31. The target genomic regions can be selected from List 32. In some arrangements, a method for detecting uterine cancer comprises evaluating the methylation status for sequencing reads derived from the target genomic regions of List 32.

The target genomic regions can be selected from List 33. In some arrangements, a method for detecting anorectal cancer comprises evaluating the methylation status for sequencing reads derived from the target genomic regions of List 33. The target genomic regions can be selected from List 34. In some arrangements, a method for detecting bladder and urothelial cancers comprises evaluating the methylation status for sequencing reads derived from the target genomic regions of List 34. The target genomic regions can be selected from List 35. In some arrangements, a method for detecting breast cancer comprises evaluating the methylation status for sequencing reads derived from the target genomic regions of List 35. The target genomic regions can be selected from List 36. In some arrangements, a method for detecting cervical cancer comprises evaluating the methylation status for sequencing reads derived from the target genomic regions of List 36. The target genomic regions can be selected from List 37. In some arrangements, a method for detecting colorectal cancer comprises evaluating the methylation status for sequencing reads derived from the target genomic regions of List 37. The target genomic regions can be selected from List 38. In some arrangements, a method for detecting head and neck cancer comprises evaluating the methylation status for sequencing reads derived from the target genomic regions of List 38. The target genomic regions can be selected from List 39. In some arrangements, a method for detecting liver and bile duct cancers comprises evaluating the methylation status for sequencing reads derived from the target genomic regions of List 39. The target genomic regions can be selected from List 40. In some arrangements, a method for detecting lung cancer comprises evaluating the methylation status for sequencing reads derived from the target genomic regions of List 40. The target genomic regions can be selected from List 41. In some arrangements, a method for detecting melanoma comprises evaluating the methylation status for sequencing reads derived from the target genomic regions of List 41. The target genomic regions can be selected from List 42. In some arrangements, a method for detecting ovarian cancer comprises evaluating the methylation status for sequencing reads derived from the target genomic regions of List 42. The target genomic regions can be selected from List 43. In some arrangements, a method for detecting pancreatic and gallbladder cancers comprises evaluating the methylation status for sequencing reads derived from the target genomic regions of List 43. The target genomic regions can be selected from List 44. In some arrangements, a method for detecting prostate cancer comprises evaluating the methylation status for sequencing reads derived from the target genomic regions of List 44. The target genomic regions can be selected from List 45. In some arrangements, a method for detecting renal cancer comprises evaluating the methylation status for sequencing reads derived from the target genomic regions of List 45. The target genomic regions can be selected from List 46. In some arrangements, a method for detecting sarcoma comprises evaluating the methylation status for sequencing reads derived from the target genomic regions of List 46. The target genomic regions can be selected from List 47. In some arrangements, a method for detecting thyroid comprises evaluating the methylation status for sequencing reads derived from the target genomic regions of List 47. The target genomic regions can be selected from List 48. In some arrangements, a method for detecting upper gastrointestinal cancer comprises evaluating the methylation status for sequencing reads derived from the target genomic regions of List 48. The target genomic regions can be selected from List 49. In some arrangements, a method for detecting uterine cancer comprises evaluating the methylation status for sequencing reads derived from the target genomic regions of List 49.

Since the probes are configured to hybridize to a converted DNA or cfDNA molecule corresponding to, or derived from, one or more genomic regions, the probes can have a sequence different from the targeted genomic region. For example, a DNA containing unmethylated CpG site will be converted to include UpG instead of CpG because unmethylated cytosines are converted to uracils by a conversion reaction (e.g., bisulfite treatment). As a result, a probe is configured to hybridize to a sequence including UpG instead of a naturally existing unmethylated CpG. Accordingly, a complementary site in the probe to the unmethylation site can comprise CpA instead of CpG, and some probes targeting a hypomethylated site where all methylation sites are unmethylated can have no guanine (G) bases. In some arrangements, at least 3%, 5%, 10%, 15%, or 20% of the probes comprise no CpG sequences.

The cancer assay panel can be used to detect the presence or absence of cancer generally and/or provide a cancer classification such as cancer type, stage of cancer such as I, II, III, or IV, or provide the TOO where the cancer is believed to originate. The panel may include probes targeting genomic regions differentially methylated between general cancerous (pan-cancer) samples and non-cancerous samples, or only in cancerous samples with a specific cancer type (e.g., lung cancer-specific targets). For example, in some arrangements, a cancer assay panel is designed to include differentially methylated genomic regions based on bisulfite sequencing data generated from the cfDNA from cancer and non-cancer individuals.

Each of the probes (or probe pairs) is designed to target one or more target genomic regions. The target genomic regions are selected based on several criteria designed to increase selective enriching of informative cfDNA fragments while decreasing noise and non-specific bindings.

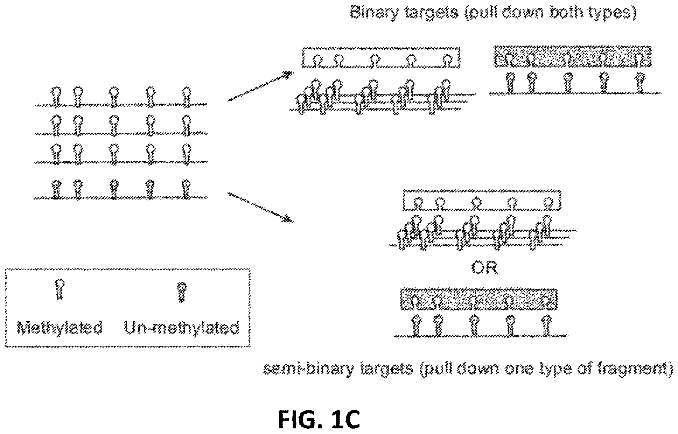

In one example, a panel can include probes that can selectively bind and optionally enrich cfDNA fragments that are differentially methylated in cancerous samples. In this case, sequence from the enriched fragments can provide information relevant to detection of cancer. Furthermore, the probes are designed to target genomic regions that are determined to have an abnormal methylation pattern in cancer samples, or in sample from certain tissue types or cell types. In one arrangement, probes are designed to target genomic regions determined to be hypermethylated or hypomethylated in certain cancers, or cancer tissue of origins, to provide additional selectivity and specificity of the detection. In some arrangements, a panel comprises probes targeting hypomethylated fragments. In some arrangements, a panel comprises probes targeting hypermethylated fragments. In some arrangements, a panel comprises both a first set of probes targeting hypermethylated fragments and a second set of probes targeting hypomethylated fragments. **(FIG. 1C)** In some arrangements, the ratio between the first set of probes targeting hypermethylated fragments and the second set of probes targeting hypomethylated fragments (Hyper:Hypo ratio) ranges between 0.4 and 2, between 0.5 and 1.8, between 0.5 and 1.6, between 1.4 and 1.6, between 1.2 and 1.4, between 1 and 1.2, between 0.8 and 1, between 0.6 and 0.8 or between 0.4 and 0.6. Methods of identifying genomic regions (i.e., genomic regions giving rise to differentially methylated DNA molecules or anomalously methylated DNA molecules between cancer and non-cancer samples, between different cancer tissue of origin (TOO) types, between different cancer cell type, or between samples from different stages of cancer) are provided in detail herein and methods of identifying anomalously methylated DNA molecules or fragments that are identified as indicative of cancer are also provided in detail herein.

In a second example, genomic regions can be selected when the genomic regions give rise to anomalously methylated DNA molecules in cancer samples or samples with known cancer tissue of origin (TOO) types. For example, as described herein, a Markov model trained on a set of non-cancerous samples can be used to identify genomic regions that give rise to anomalously methylated DNA molecules (i.e., DNA molecules having a methylation pattern below a p-value threshold).

Each of the probes can target a genomic region comprising at least 30bp, 35bp, 40bp, 45bp, 50bp, 60bp, 70bp, 80bp, 90bp, 100bp or more. In some arrangements, the genomic regions can be selected to have less than 30, 25, 20, 15, 12, 10, 8, or 6 methylation sites.

The genomic regions can be selected when at least 80, 85, 90, 92, 95, or 98% of the at least five methylation (e.g., CpG) sites within the region are either methylated or unmethylated in non-cancerous or cancerous samples, or in cancer samples from a tissue of origin (TOO).

Genomic regions may be further filtered to select only those that are likely to be informative based on their methylation patterns, for example, CpG sites that are differentially methylated between cancerous and non-cancerous samples (e.g., abnormally methylated or unmethylated in cancer versus non-cancer), between cancerous samples of a TOO and cancerous samples of a different TOO, or CpG sites that are differentially methylated only in cancerous samples of a specific TOO. For the selection, calculation can be performed with respect to each CpG or a plurality of CpG sites. For example, a first count is determined that is the number of cancer-containing samples (cancer count) that include a fragment overlapping that CpG, and a second count is determined that is the number of total samples containing fragments overlapping that CpG site (total). Genomic regions can be selected based on criteria positively correlated to the number of cancer-containing samples (cancer count) that include a fragment indicative of cancer overlapping that CpG site, and inversely correlated with the number of total samples containing fragments indicative of cancer overlapping that CpG site (total). In one arrangement, the number of non-cancerous samples ($n_{non-cancer}$) and the number of cancerous samples ($n_{cancer}$) having a fragment overlapping a CpG site are counted. Then the probability that a sample is cancer is estimated, for example as ($n_{cancer}$ + 1) / ($n_{cancer}$ + $n_{non-cancer}$ + 2).

CpG sites scored by this metric are ranked and greedily added to a panel until the panel size budget is exhausted. The process of selecting genomic regions indicative of cancer is further detailed herein. In some arrangements, depending on whether the assay is intended to be a pan-cancer assay or a single-cancer assay, or depending on what kind of flexibility is desired when picking which CpG sites are contributing to the panel. A panel for detecting a specific cancer type can be designed using a similar process. In this arrangement, for each cancer type, and for each CpG site, the information gain is computed to determine whether to include a probe targeting that CpG site. The information gain may be computed for samples with a given cancer type of a TOO compared to all other samples. For example, consider two random variables, "AF" and "CT". "AF" is a binary variable that indicates whether there is an abnormal fragment overlapping a particular CpG site in a particular sample (yes or no). "CT" is a binary random variable indicating whether the cancer is of a particular type (e.g., lung cancer or cancer other than lung). One can compute the mutual information with respect to "CT" given "AF." That is, how many bits of information about the cancer type (lung vs. non-lung in the example) are gained if one knows whether there is an anomalous fragment overlapping a particular CpG site. This can be used to rank CpG's based on how lung-specific they are. This procedure is repeated for a plurality of cancer types. If a particular region is commonly differentially methylated only in lung cancer (and not other cancer types or non-cancer), CpG's in that region would tend to have high information gains for lung cancer. For each cancer type, CpG sites are ranked by this information gain metric, and then greedily added to a panel until the size budget for that cancer type is exhausted.

Further filtration can be performed to select probes with high specificity for enrichment (i.e., high binding efficiency) of nucleic acids derived from targeted genomic regions. Probes can be filtered to reduce non-specific binding (or off-target binding) to nucleic acids derived from non-targeted genomic regions. For example, probes can be filtered to select only those probes having less than a set threshold of off-target binding events. In one arrangement, probes can be aligned to a reference genome (e.g., a human reference genome) to select probes that align to less than a set threshold of regions across the genome. For example, probes can be selected that align to less than 25, 24, 23, 22, 21, 20, 19, 18, 17, 16, 15, 14, 13, 12, 11, 10, 9 or 8 off-target regions across the reference genome. In other cases, filtration is performed to remove genomic regions when the sequence of the target genomic regions appears more than 5, 10, 15, 20, 21, 22, 23, 24, 25, 26, 27, 28, 29, 30, 31, 32, 33, 34 or 35 times in a genome. Further filtration can be performed to select target genomic regions when a probe sequence, or a set of probe sequences that are 90%, 91%, 92%, 93%, 94%, 95%, 96%, 97%, 98% or 99% homologous to the target genomic regions, appear less than 25, 24, 23, 22, 21, 20, 19, 18, 17, 16, 15, 14, 13, 12, 11, 10, 9 or 8 times in a reference genome, or to remove target genomic regions when the probe sequence, or a set of probe sequences designed to enrich for the targeted genomic region are 90%, 91%, 92%, 93%, 94%, 95%, 96%, 97%, 98% or 99% homologous to the target genomic regions, appear more than 5, 10, 15, 20, 21, 22, 23, 24, 25, 26, 27, 28, 29, 30, 31, 32, 33, 34 or 35 times in a reference genome. This is for excluding repetitive probes that can pull down off-target fragments, which are not desired and can impact assay efficiency.

In some arrangements, a fragment-probe overlap of at least 45 bp was demonstrated to be effective for achieving a non-negligible amount of pulldown (though as one of skill in the art would appreciate this number can very) as provided in Example 1. In some arrangements, more than a 10% mismatch rate between the probe and fragment sequences in the region of overlap is sufficient to greatly disrupt binding, and thus pulldown efficiency. Therefore, sequences that can align to the probe along at least 45 bp with at least a 90% match rate can be candidates for off-target pulldown. Thus, in one arrangement, the number of such regions are scored. The best probes have a score of 1, meaning they match in only one place (the intended target region). Probes with an intermediate score (say, less than 5 or 10) may in some instances be accepted, and in some instances any probes above a particular score are discarded. Other cutoff values can be used for specific samples.

Once the probes hybridize and capture DNA fragments corresponding to, or derived from, a target genomic region, the hybridized probe-DNA fragment intermediates are pulled down (or isolated), and the targeted DNA is amplified and its methylation status is determined by, for example, sequencing or hybridization to a microarray, etc. The sequence read provides information relevant for detection of cancer. For this end, a panel is designed to include a plurality of probes that can capture fragments that can together provide information relevant to detection of cancer. In some arrangements, a panel includes at least 5, 50, 100, 200, 300, 400, 500, 600, 700, 800, 900, 1000, 1,200, 1,400, 1,600, 1,800, 2,000, 2,200, 2,400, 2,600, 2,800, 3,000, 3,200, 4,000, 4,500, 5,000, 5,500, 6,000, 6,500, 7,000, 7,500, 8,000, 8,500, 9,000, or 10,000 pairs of probes. In other arrangements, a panel includes at least 100, 200, 300, 400, 500, 600, 700, 800, 900, 1000, 1,200, 1,400, 1,600, 1,800, 2,000, 2,200, 2,400, 2,600, 2,800, 3,000, 3,200, 4,000, 4,500, 5,000, 5,500, 6,000, 6,500, 7,000, 7,500, 8,000, 8,500, 9,000, 10,000, 15,000, or 20,000 probes. The plurality of probes together can comprise at least 10,000, 20,000, 30,000, 40,000, 50,000, 60,000, 70,000, 80,000, 90,000, 100,000, 120,000, 140,000, 160,000, 180,000, 200,000, 240,000, 260,000, 280,000, 300,000, 320,000, 400,000, 450,000, 500,000, 550,000, 600,000, 650,000, 700,000, 750,000, 800,000, 850,000, 900,000, 1 million, 1.5million, 2 million, 2.5 million, or 3 million nucleotides.

The selected target genomic regions can be located in various positions in a genome, including but not limited to exons, introns, intergenic regions, and other parts. In some arrangements, probes targeting non-human genomic regions, such as those targeting viral genomic regions, can be added.

In some instances, primers may be used to specifically amplify targets/biomarkers of interest (e.g., by PCR), thereby enriching the sample for desired targets/biomarkers (optionally without hybridization capture). For example, forward and reverse primers can be prepared for each genomic region of interest and used to amplify fragments that correspond to or are derived from the desired genomic region. Thus, while the present disclosure pays particular attention to cancer assay panels and bait sets for hybridization capture, the disclosure is broad enough to encompass other methods for enrichment of cell-free DNA. Accordingly, a skilled artisan, with the benefit of this disclosure, will recognize that methods analogous to those described herein in connection with hybridization capture can alternatively be accomplished by replacing hybridization capture with some other enrichment strategy, such as PCR amplification of cell-free DNA fragments that correspond with genomic regions of interest. Such methods are not claimed. In some arrangements, bisulfite padlock probe capture is used to enrich regions of interest, such as is described in Zhang et al. (US 2016/0340740). In some arrangements, additional or alternative methods are used for enrichment (e.g., non-targeted enrichment) such as reduced representation bisulfite sequencing, methylation restriction enzyme sequencing, methylation DNA immunoprecipitation sequencing, methyl-CpG-binding domain protein sequencing, methyl DNA capture sequencing, or microdroplet PCR.

*Probes*

The cancer assay panel provided herein is a panel including a set of hybridization probes (also referred to herein as "probes") designed to, during enrichment, target and pull down nucleic acid fragments of interest for the assay. In some arrangements, the probes are designed to hybridize and enrich DNA or cfDNA molecules from cancerous samples that have been treated to convert unmethylated cytosines (C) to uracils (U). In other arrangements, the probes are designed to hybridize and enrich DNA or cfDNA molecules from cancerous samples of a TOO that have been treated to convert unmethylated cytosines (C) to uracils (U). The probes can be designed to anneal (or hybridize) to a target (complementary) strand of DNA or RNA. The target strand can be the "positive" strand (e.g., the strand transcribed into mRNA, and subsequently translated into a protein) or the complementary "negative" strand. In a particular arrangement, a cancer assay panel may include sets of two probes, one probe targeting the positive strand and the other probe targeting the negative strand of a target genomic region.

For each target genomic region, four possible probe sequences can be designed. DNA molecules corresponding to, or derived from, each target region is double-stranded, as such, a probe or probe set can target either the "positive" or forward strand or its reverse complement (the "negative" strand). Additionally, in some arrangements, the probes or probe sets are designed to enrich DNA molecules or fragments that have been treated to convert unmethylated cytosines (C) to uracils (U). Because the probes or probe sets are designed to enrich DNA molecules corresponding to, or derived from the targeted regions after conversion, the probe's sequence can be designed to enrich DNA molecules of fragments where unmethylated C's have been converted to U's (by utilizing A's in place of G's at sites that are unmethylated cytosines in DNA molecules or fragments corresponding to, or derived from, the targeted region). In one arrangement, probes are designed to bind to, or hybridize to, DNA molecules or fragments from genomic regions known to contain cancer-specific methylation patterns (e.g., hypermethylated or hypomethylated DNA molecules), thereby enriching (or detecting) cancer-specific DNA molecules or fragments. Targeting genomic regions, or cancer-specific methylation patterns, can be advantageous allowing one to specifically enrich for DNA molecules or fragments identified as informative for cancer or cancer TOO, and thus, lowering detection needs and costs (e.g., lowering sequencing costs). In other arrangements, two probe sequences can be designed per a target genomic region (one for each DNA strand). In still other cases, probes are designed to enrich for all DNA molecules or fragments corresponding to, or derived from, a targeted region (i.e., regardless of strand or methylation status). This might be because the cancer methylation status is not highly methylated or unmethylated, or because the probes are designed to target small mutations or other variations rather than methylation changes, with these other variations similarly indicative of the presence or absence of a cancer or the presence or absence of a cancer of one or more TOOs. In that case, all four possible probe sequences can be included per a target genomic region.

The probes can range in length from 10s, 100s, 200s, or 300s of base pairs. The probes can comprise at least 50, 75, 100, or 120 nucleotides. The probes can comprise less than 300, 250, 200, or 150 nucleotides. In an arrangement, the probes comprise 100-150 nucleotides. In one particular arrangement, the probes comprise 120 nucleotides.

In some arrangements, the probes are designed in a "2× tiled" fashion to cover overlapping portions of a target region. Each probe optionally overlaps in coverage at least partially with another probe in the library. In such arrangements, the panel contains multiple pairs of probes, with each probe in a pair overlapping the other by at least 25, 30, 35, 40, 45, 50, 60, 70, 75 or 100 nucleotides. In some arrangements, the overlapping sequence can be designed to be complementary to a target genomic region (or cfDNA derived therefrom) or to be complementary to a sequence with homology to a target region or cfDNA. Thus, in some arrangements, at least two probes are complementary to the same sequence within a target genomic region, and a nucleotide fragment corresponding to or derived from the target genomic region can be bound and pulled down by at least one of the probes. Other levels of tiling are possible, such as 3× tiling, 4× tiling, etc., wherein each nucleotide in a target region can bind to more than two probes.

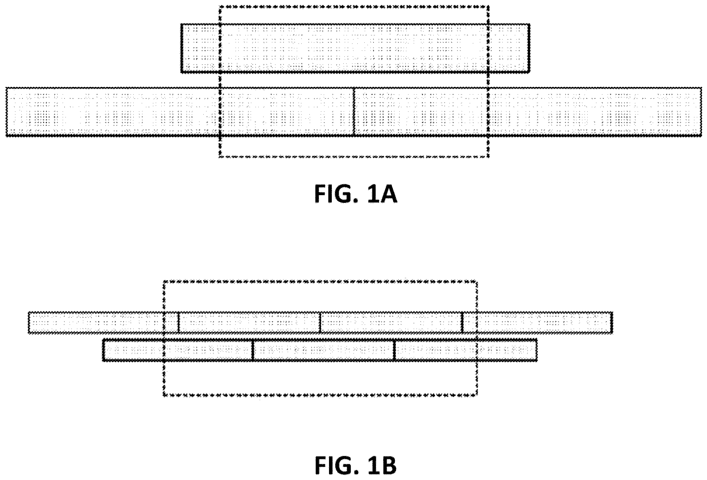

In one arrangement, each base in a target genomic region is overlapped by exactly two probes, as illustrated in **FIG. 1A.** A single pair of probes is enough to pull down a genomic region if the overlap between the two probes is longer than the target genomic region and extends beyond both ends of the target genomic region. In some instances, even relatively small target regions may be targeted with three probes (see **FIG. 1A).** A probe set comprising three or more probes is optionally used to capture a larger genomic region (see **FIG. 1B).** In some arrangements, subsets of probes will collectively extend across an entire genomic region (e.g., may be complementary to non-converted or converted fragments from the genomic region). A tiled probe set optionally comprises probes that collectively include at least two probes that overlap every nucleotide in the genomic region. This is done to ensure that cfDNAs comprising a small portion of a target genomic region at one end will have a substantial overlap extending into the adjacent non-targeted genomic region with at least one probe, to provide for efficient capture.

For example, a 100 bp cfDNA fragment comprising a 30 nt target genomic region can be guaranteed to have at least 65 bp overlap with at least one of the overlapping probes. Other levels of tiling are possible. For example, to increase target size and add more probes in a panel, probes can be designed to expand a 30 bp target region by at least 70 bp, 65 bp, 60 bp, 55 bp, or 50 bp. To capture any fragment that overlaps the target region at all (even if by only 1bp), the probes can be designed to extend past the ends of the target region on either side.

The probes are designed to analyze methylation status of target genomic regions (e.g., of the human or another organism) that are suspected to correlate with the presence or absence of cancer generally, presence or absence of certain types of cancers, cancer stage, or presence or absence of other types of diseases.

Furthermore, the probes are designed to effectively hybridize to and optionally pull down cfDNA fragments containing a target genomic region. In some arrangements, the probes are designed to cover overlapping portions of a target region, so that each probe is "tiled" in coverage such that each probe overlaps in coverage at least partially with another probe in the library. In such arrangements, the panel contains multiple pairs of probes, where each pair comprises at least two probes overlapping each other by an overlapping sequence of at least 25, 30, 35, 40, 45, 50, 60, 70, 75 or 100 nucleotides. In some arrangements, the overlapping sequence can be designed to be complementary to a target genomic region (or a converted version thereof), thus a nucleotide fragment derived from or containing the target genomic region can be bound and optionally pulled down by at least one of the probes.

In one arrangement, the smallest target genomic region is 30bp. When a new target region is added to the panel (based on the greedy selection as described above), the new target region of 30bp can be centered on a specific CpG site of interest. Then, it is checked whether each edge of this new target is close enough to other targets such that they can be merged. This is based on a "merge distance" parameter which can be 200bp by default but can be tuned. This allows close but distinct target regions to be enriched with overlapping probes. Depending on whether close enough targets exist to the left or right of the new target, the new target can be merged with nothing (increasing the number of panel targets by one), merged with just one target either to the left or the right (not changing the number of panel targets), or merged with existing targets both to the left and right (reducing the number of panel targets by one).

### Methods of selecting target genomic regions

In another arrangement, methods of selecting target genomic regions for detecting cancer and/or a TOO are provided. The targeted genomic regions can be used to design and manufacture probes for a cancer assay panel. Methylation status of DNA or cfDNA molecules corresponding to, or derived from, the target genomic regions can be screened using the cancer assay panel. Alternative methods, for example by WGBS or other methods known in the art, can be also implemented to detect methylation status of DNA molecules or fragments corresponding to, or derived from, the target genomic regions.

*Sample processing*

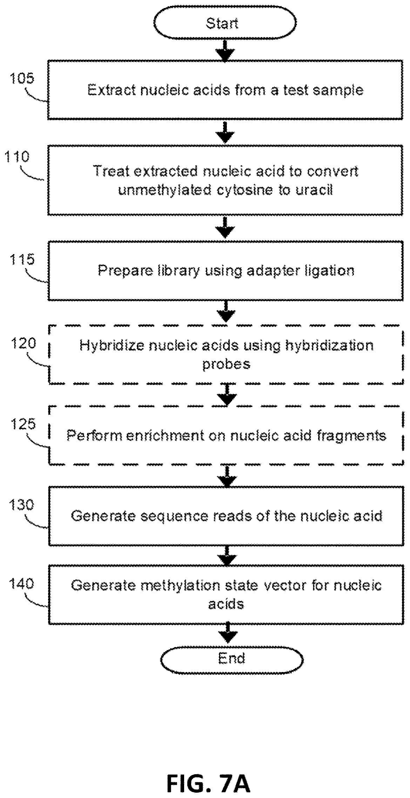

FIG. 7A is a flowchart of a process 100 for processing a nucleic acid sample and generating methylation state vectors for DNA fragments, according to one arrangement. While the present disclosure pays particular attention to sequencing based approaches for detecting nucleic acids and determining methylation status, the disclosure is broad enough to encompass other methods for determining methylation status of nucleic acid sequences (such as methylation-aware sequencing approaches described in WO 2014/043763). As described in FIG. 7A, the method includes, but is not limited to, the following steps. For example, any step of the method may comprise a quantitation sub-step for quality control or other laboratory assay procedures known to one skilled in the art.

In step 105, a nucleic acid sample (DNA or RNA) is extracted from a subject. In the present disclosure, DNA and RNA may be used interchangeably unless otherwise indicated. That is, the arrangementsdescribed herein may be applicable to both DNA and RNA types of nucleic acid sequences. However, the examples described herein may focus on DNA for purposes of clarity and explanation. The sample may be any subset of the human genome, including the whole genome. The sample may include blood, plasma, serum, urine, fecal, saliva, other types of bodily fluids, or any combination thereof. In some arrangements, methods for drawing a blood sample (e.g., syringe or finger prick) may be less invasive than procedures for obtaining a tissue biopsy, which may require surgery. The extracted sample may comprise cfDNA and/or ctDNA. For healthy individuals, the human body may naturally clear out cfDNA and other cellular debris. If a subject has a cancer or disease, cfDNA and/or ctDNA in an extracted sample may be present at a detectable level for detecting the cancer or disease.

In step 110, the cfDNA fragments are treated to convert unmethylated cytosines to uracils. In one arrangement, the method uses a bisulfite treatment of the DNA which converts the unmethylated cytosines to uracils without converting the methylated cytosines. For example, a commercial kit such as the EZ DNA Methylation™ - Gold, EZ DNA Methylation™ - Direct or an EZ DNA Methylation™ - Lightning kit (available from Zymo Research Corp (Irvine, CA)) is used for the bisulfite conversion. In another arrangement, the conversion of unmethylated cytosines to uracils is accomplished using an enzymatic reaction. For example, the conversion can use a commercially available kit for conversion of unmethylated cytosines to uracils, such as APOBEC-Seq (NEBiolabs, Ipswich, MA).

In step 115, a sequencing library is prepared. In a first step, a ssDNA adapter is added to the 3'-OH end of a bisulfite-converted ssDNA molecule using a ssDNA ligation reaction. In one arrangement, the ssDNA ligation reaction uses CircLigase II (Epicentre) to ligate the ssDNA adapter to the 3'-OH end of a bisulfite-converted ssDNA molecule, wherein the 5'-end of the adapter is phosphorylated and the bisulfite-converted ssDNA has been dephosphorylated (i.e., the 3' end has a hydroxyl group). In another arrangement, the ssDNA ligation reaction uses Thermostable 5' AppDNA/RNA ligase (available from New England BioLabs (Ipswich, MA)) to ligate the ssDNA adapter to the 3'-OH end of a bisulfite-converted ssDNA molecule. In this example, the first UMI adapter is adenylated at the 5'-end and blocked at the 3'-end.

In another arrangement, the ssDNA ligation reaction uses a T4 RNA ligase (available from New England BioLabs) to ligate the ssDNA adapter to the 3'-OH end of a bisulfite-converted ssDNA molecule. In a second step, a second strand DNA is synthesized in an extension reaction. For example, an extension primer, that hybridizes to a primer sequence included in the ssDNA adapter, is used in a primer extension reaction to form a double-stranded bisulfite-converted DNA molecule. Optionally, in one arrangement, the extension reaction uses an enzyme that is able to read through uracil residues in the bisulfite-converted template strand. Optionally, in a third step, a dsDNA adapter is added to the double-stranded bisulfite-converted DNA molecule. Finally, the double-stranded bisulfite-converted DNA is amplified to add sequencing adapters. For example, PCR amplification using a forward primer that includes a P5 sequence and a reverse primer that includes a P7 sequence is used to add P5 and P7 sequences to the bisulfite-converted DNA. Optionally, during library preparation, unique molecular identifiers (UMI) may be added to the nucleic acid molecules (e.g., DNA molecules) through adapter ligation. The UMIs are short nucleic acid sequences (e.g., 4-10 base pairs) that are added to ends of DNA fragments during adapter ligation. In some arrangements, UMIs are degenerate base pairs that serve as a unique tag that can be used to identify sequence reads originating from a specific DNA fragment. During PCR amplification following adapter ligation, the UMIs are replicated along with the attached DNA fragment, which provides a way to identify sequence reads that came from the same original fragment in downstream analysis.

In step 120, targeted DNA sequences may be enriched from the library. This is used, for example, where a targeted panel assay is being performed on the samples. During enrichment, hybridization probes (also referred to herein as "probes") are used to target, and pull down, nucleic acid fragments informative for the presence or absence of cancer (or disease), cancer status, or a cancer classification (e.g., cancer type or tissue of origin). For a given workflow, the probes may be designed to anneal (or hybridize) to a target (complementary) strand of DNA or RNA. The target strand may be the "positive" strand (e.g., the strand transcribed into mRNA, and subsequently translated into a protein) or the complementary "negative" strand. The probes may range in length from 10s, 100s, or 1000s of base pairs. Moreover, the probes may cover overlapping portions of a target region.

After a hybridization step 120, the hybridized nucleic acid fragments are captured and may also be amplified using PCR (enrichment 125). For example, the target sequences can be enriched to obtain enriched sequences that can be subsequently sequenced. In general, any known method in the art can be used to isolate, and enrich for, probe-hybridized target nucleic acids. For example, as is well known in the art, a biotin moiety can be added to the 5'-end of the probes (i.e., biotinylated) to facilitate isolation of target nucleic acids hybridized to probes using a streptavidin-coated surface (e.g., streptavidin-coated beads).

In step 130, sequence reads are generated from the enriched DNA sequences, e.g., enriched sequences. Sequence data may be acquired from the enriched DNA sequences by known means in the art. For example, the method may include next generation sequencing (NGS) techniques including synthesis technology (Illumina), pyrosequencing (454 Life Sciences), ion semiconductor technology (Ion Torrent sequencing), single-molecule real-time sequencing (Pacific Biosciences), sequencing by ligation (SOLiD sequencing), nanopore sequencing (Oxford Nanopore Technologies), or paired-end sequencing. In some arrangements, massively parallel sequencing is performed using sequencing-by-synthesis with reversible dye terminators. In other arrangements, as would be readily understood by one of skill in the art, any known means for detecting nucleic acids and determining methylations status can be used. For example, sequences can be detected, and methylation status determined, using known methylation-aware sequencing (see e.g., WO 2014/043763), a DNA microarray (e.g., with labeled probes adhered or conjugated to a solid surface or DNA array chip), etc.

In step 140, methylation state vectors are generated from the sequence reads. To do so, a sequence read is aligned to a reference genome. The reference genome helps provide the context as to what position in a human genome the fragment cfDNA originates from. In a simplified example, the sequence read is aligned such that the three CpG sites correlate to CpG sites 23, 24, and 25 (arbitrary reference identifiers used for convenience of description). After alignment, there is information both on methylation status of all CpG sites on the cfDNA fragment and which position in the human genome the CpG sites map to. With the methylation status and location, a methylation state vector may be generated for the fragment cfDNA.

*Generation of data structure*

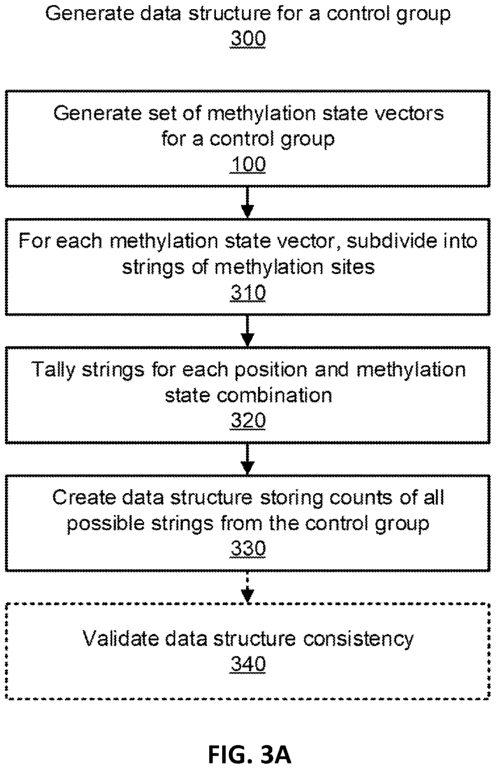

**FIG. 3A** is a flowchart describing a process 300 of generating a data structure for a healthy control group, according to an arrangement. To create a healthy control group data structure, the analytics system obtains information related to methylation status of a plurality of CpG sites on sequence reads derived from a plurality of DNA molecules or fragments from a plurality of healthy subjects. The method provided herein for creating a healthy control group data structure can be performed similarly for subjects with cancer, subjects with cancer of a TOO, subjects with a known cancer type, or subjects with another known disease state. A methylation state vector is generated for each DNA molecule or fragment, for example via the process 100.

With each fragment's methylation state vector, the analytics system subdivides 310 the methylation state vector into strings of CpG sites. In one arrangement, the analytics system subdivides 310 the methylation state vector such that the resulting strings are all less than a given length. For example, a methylation state vector of length 11 may be subdivided into strings of length less than or equal to 3 would result in 9 strings of length 3, 10 strings of length 2, and 11 strings of length 1. In another example, a methylation state vector of length 7 being subdivided into strings of length less than or equal to 4 would result in 4 strings of length 4, 5 strings of length 3, 6 strings of length 2, and 7 strings of length 1. If a methylation state vector is shorter than or the same length as the specified string length, then the methylation state vector may be converted into a single string containing all of the CpG sites of the vector.

The analytics system tallies 320 the strings by counting, for each possible CpG site and possibility of methylation states in the vector, the number of strings present in the control group having the specified CpG site as the first CpG site in the string and having that possibility of methylation states. For example, at a given CpG site and considering string lengths of 3, there are 2^3 or 8 possible string configurations. At that given CpG site, for each of the 8 possible string configurations, the analytics system tallies 320 how many occurrences of each methylation state vector possibility come up in the control group. Continuing this example, this may involve tallying the following quantities: $< M_x, M_{x+1}, M_{x+2} >, < M_x, M_{x+1}, U_{x+2} >, ..., < U_x, U_{x+1}, U_{x+2} >$ for each starting CpG site x in the reference genome. The analytics system creates 330 the data structure storing the tallied counts for each starting CpG site and string possibility.

There are several benefits to setting an upper limit on string length. First, depending on the maximum length for a string, the size of the data structure created by the analytics system can dramatically increase in size. For instance, maximum string length of 4 means that every CpG site has at the very least 2^4 numbers to tally for strings of length 4. Increasing the maximum string length to 5 means that every CpG site has an additional 2^4 or 16 numbers to tally, doubling the numbers to tally (and computer memory required) compared to the prior string length. Reducing string size helps keep the data structure creation and performance (e.g., use for later accessing as described below), in terms of computational and storage, reasonable. Second, a statistical consideration to limiting the maximum string length is to avoid overfitting downstream models that use the string counts. If long strings of CpG sites do not, biologically, have a strong effect on the outcome (e.g., predictions of anomalousness that predictive of the presence of cancer), calculating probabilities based on large strings of CpG sites can be problematic as it requires a significant amount of data that may not be available, and thus would be too sparse for a model to perform appropriately. For example, calculating a probability of anomalousness/cancer conditioned on the prior 100 CpG sites would require counts of strings in the data structure of length 100, ideally some matching exactly the prior 100 methylation states. If only sparse counts of strings of length 100 are available, there will be insufficient data to determine whether a given string of length of 100 in a test sample is anomalous or not.

*Validation of data structure*

Once the data structure has been created, the analytics system may seek to validate 340 the data structure and/or any downstream models making use of the data structure. One type of validation checks consistency within the control group's data structure. For example, if there are any outlier subjects, samples, and/or fragments within a control group, then the analytics system may perform various calculations to determine whether to exclude any fragments from one of those categories. In a representative example, the healthy control group may contain a sample that is undiagnosed but cancerous such that the sample contains anomalously methylated fragments. This first type of validation ensures that potential cancerous samples are removed from the healthy control group so as to not affect the control group's purity.

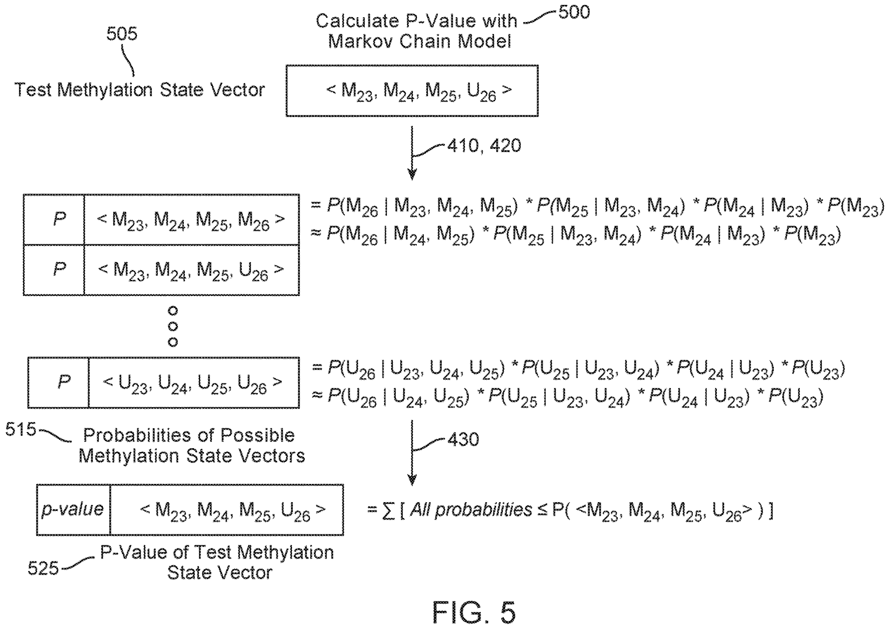

A second type of validation checks the probabilistic model used to calculate p-values with the counts from the data structure itself (i.e., from the healthy control group). A process for p-value calculation is described below in conjunction with **FIG. 5.** Once the analytics system generates a p-value for the methylation state vectors in the validation group, the analytics system builds a cumulative density function (CDF) with the p-values. With the CDF, the analytics system may perform various calculations on the CDF to validate the control group's data structure. One test uses the fact that the CDF should ideally be at or below an identity function, such that $CDF(x) \leq x$. On the converse, being above the identity function reveals some deficiency within the probabilistic model used for the control group's data structure. For example, if 1/100 of fragments have a p-value score of 1/1000 meaning CDF(1/1000) = 1/100 > 1/1000, then the second type of validation fails indicating an issue with the probabilistic model.

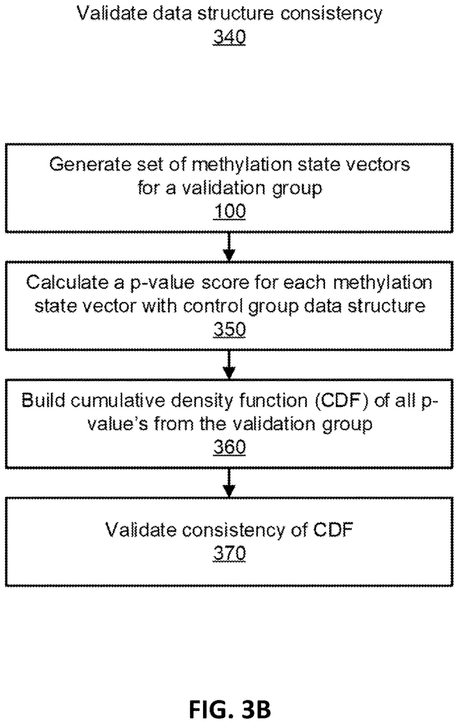

A third type of validation uses a healthy set of validation samples separate from those used to build the data structure, which tests if the data structure is properly built and the model works. An example process for carrying out this type of validation is described below in conjunction with **FIG. 3B.** The third type of validation can quantify how well the healthy control group generalizes the distribution of healthy samples. If the third type of validation fails, then the healthy control group does not generalize well to the healthy distribution.

A fourth type of validation tests with samples from a non-healthy validation group. The analytics system calculates p-values and builds the CDF for the non-healthy validation group. With a non-healthy validation group, the analytics system expects to see the CDF(x) > x for at least some samples or, stated differently, the converse of what was expected in the second type of validation and the third type of validation with the healthy control group and the healthy validation group. If the fourth type of validation fails, then this is indicative that the model is not appropriately identifying the anomalousness that it was designed to identify.

**FIG. 3B** is a flowchart describing the additional step 340 of validating the data structure for the control group of **FIG. 3A,** according to an arrangement. In this arrangement of the step 340 of validating the data structure, the analytics system performs the fourth type of validation test as described above which utilizes a validation group with a supposedly similar composition of subj ects, samples, and/or fragments as the control group. For example, if the analytics system selected healthy subjects without cancer for the control group, then the analytics system also uses healthy subjects without cancer in the validation group.

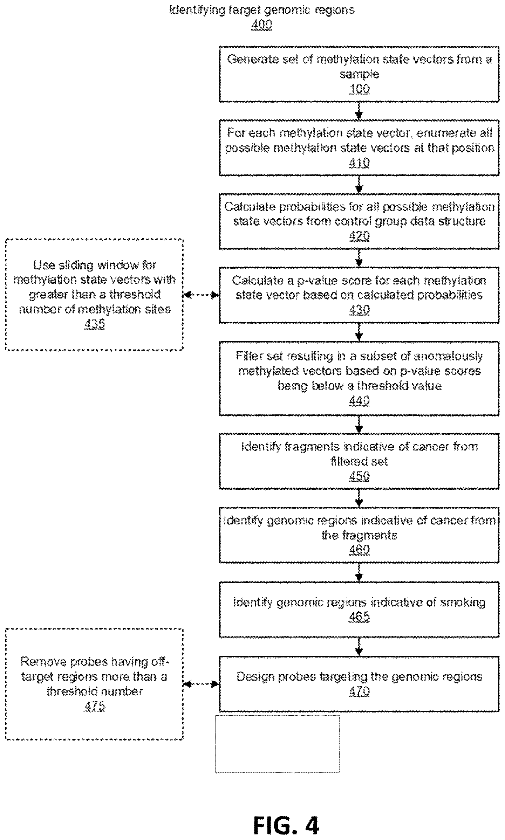

The analytics system takes the validation group and generates 100 a set of methylation state vectors as described in FIG. 3A. The analytics system performs a p-value calculation for each methylation state vector from the validation group. The p-value calculation process will be further described in conjunction with **FIGS. 4-5.** For each possibility of methylation state vector, the analytics system calculates a probability from the control group's data structure. Once the probabilities are calculated for the possibilities of methylation state vectors, the analytics system calculates 350 a p-value score for that methylation state vector based on the calculated probabilities. The p-value score represents an expectedness of finding that specific methylation state vector and other possible methylation state vectors having even lower probabilities in the control group. A low p-value score, thereby, generally corresponds to a methylation state vector which is relatively unexpected in comparison to other methylation state vectors within the control group, where a high p-value score generally corresponds to a methylation state vector which is relatively more expected in comparison to other methylation state vectors found in the control group. Once the analytics system generates a p-value score for the methylation state vectors in the validation group, the analytics system builds 360 a cumulative density function (CDF) with the p-value scores from the validation group. The analytics system validates 370 consistency of the CDF as described above in the fourth type of validation tests.

*Anomalously methylated fragments*

Anomalously methylated fragments having abnormal methylation patterns in cancer patient samples, subject with cancer of a TOO, subjects with a known cancer type, or subjects with another known disease state, are selected as target genomic regions, according to an arrangement as outlined in **FIG. 4.** Exemplary processes of selected anomalously methylated fragments 440 are visually illustrated in **FIG. 5,** and is further described below the description of **FIG. 4.** In process 400, the analytics system generates 100 methylation state vectors from cfDNA fragments of the sample. The analytics system handles each methylation state vector as follows.

For a given methylation state vector, the analytics system enumerates 410 all possibilities of methylation state vectors having the same starting CpG site and same length (i.e., set of CpG sites) in the methylation state vector. As each methylation state may be methylated or unmethylated there are only two possible states at each CpG site, and thus the count of distinct possibilities of methylation state vectors depends on a power of 2, such that a methylation state vector of length n would be associated with 2n possibilities of methylation state vectors.

The analytics system calculates 420 the probability of observing each possibility of methylation state vector for the identified starting CpG site / methylation state vector length by accessing the healthy control group data structure. In one arrangement, calculating the probability of observing a given possibility uses a Markov chain probability to model the joint probability calculation which will be described in greater detail with respect to **FIG. 5** below. In other arrangements, calculation methods other than Markov chain probabilities are used to determine the probability of observing each possibility of methylation state vector.

The analytics system calculates 430 a p-value score for the methylation state vector using the calculated probabilities for each possibility. In one arrangement, this includes identifying the calculated probability corresponding to the possibility that matches the methylation state vector in question. Specifically, this is the possibility having the same set of CpG sites, or similarly the same starting CpG site and length as the methylation state vector. The analytics system sums the calculated probabilities of any possibilities having probabilities less than or equal to the identified probability to generate the p-value score.

This p-value represents the probability of observing the methylation state vector of the fragment or other methylation state vectors even less probable in the healthy control group. A low p-value score, thereby, generally corresponds to a methylation state vector which is rare in a healthy subject, and which causes the fragment to be labeled abnormally methylated, relative to the healthy control group. A high p-value score generally relates to a methylation state vector is expected to be present, in a relative sense, in a healthy subject. If the healthy control group is a non-cancerous group, for example, a low p-value indicates that the fragment is abnormally methylated relative to the non-cancer group, and therefore possibly indicative of the presence of cancer in the test subject.

As above, the analytics system calculates p-value scores for each of a plurality of methylation state vectors, each representing a cfDNA fragment in the test sample. To identify which of the fragments are abnormally methylated, the analytics system may filter 440 the set of methylation state vectors based on their p-value scores. In one embodiment, filtering is performed by comparing the p-values scores against a threshold and keeping only those fragments below the threshold. This threshold p-value score could be on the order of 0.1, 0.01, 0.001, 0.0001, or similar.

*P-value score calculation*

FIG. 5 is an illustration 500 of an example p-value score calculation, according to an arrangement. To calculate a p-value score given a test methylation state vector 505, the analytics system takes that test methylation state vector 505 and enumerates 410 possibilities of methylation state vectors. In this illustrative example, the test methylation state vector 505 is < M23, M24, M25, U26 >. As the length of the test methylation state vector 505 is 4, there are 2^4 possibilities of methylation state vectors encompassing CpG sites 23 - 26. In a generic example, the number of possibilities of methylation state vectors is 2^n, where n is the length of the test methylation state vector or alternatively the length of the sliding window (described further below).

The analytics system calculates 420 probabilities 515 for the enumerated possibilities of methylation state vectors. As methylation is conditionally dependent on methylation status of nearby CpG sites, one way to calculate the probability of observing a given methylation state vector possibility is to use Markov chain model. Generally, a methylation state vector such as $<S_1, S_2, ..., S_n>$, where S denotes the methylation state whether methylated (denoted as M), unmethylated (denoted as U), or indeterminate (denoted as I), has a joint probability that can be expanded using the chain rule of probabilities as:

$$P(< S_1, S_2, ..., S_n >) = P(S_n | S_1, ..., S_{n-1} >) * P(S_{n-1} | S_1, ..., S_{n-2} >) * \\ ... * P(S_2 | S_1) * P(S_1) \tag{1}$$

Markov chain model can be used to make the calculation of the conditional probabilities of each possibility more efficient. In one arrangement, the analytics system selects a Markov chain order k which corresponds to how many prior CpG sites in the vector (or window) to consider in the conditional probability calculation, such that the conditional probability is modeled as $P(S_n | S_1, ..., S_{n-1}) \sim P(S_n | S_{n-k-2}, ..., S_{n-1})$.

To calculate each Markov modeled probability for a possibility of methylation state vector, the analytics system accesses the control group's data structure, specifically the counts of various strings of CpG sites and states. To calculate $P(M_n | S_{n-k-2}, ..., S_{n-1})$, the analytics system takes a ratio of the stored count of the number of strings from the data structure matching

$$< S_{n-k-2}, ..., S_{n-1}, M_n > \text{ divided by the sum of the stored count of the number of strings} \tag{2}$$

from the data structure matching $< S_{n-k-2}, ..., S_{n-1}, M_n >$ and $< S_{n-k-2}, ..., S_{n-1}, U_n >$. Thus, $P(M_n | S_{n-k-2}, ..., S_{n-1})$, is calculated ratio having the form:

$$\frac{\# \text{ of } < S_{n-k-2}, ..., S_{n-1}, M_n >}{\# \text{ of } < S_{n-k-2}, ..., S_{n-1}, M_n > + \# \text{ of } < S_{n-k-2}, ..., S_{n-1}, U_n >}$$

The calculation may additionally implement a smoothing of the counts by applying a prior distribution. In one arrangement, the prior distribution is a uniform prior as in Laplace smoothing. As an example of this, a constant is added to the numerator and another constant (e.g., twice the constant in the numerator) is added to the denominator of the above equation. In other arrangements, an algorithmic technique such as Knesser-Ney smoothing is used.

In the illustration, the above denoted formulas are applied to the test methylation state vector 505 covering sites 23 - 26. Once the calculated probabilities 515 are completed, the analytics system calculates 430 a p-value score 525 that sums the probabilities that are less than or equal to the probability of possibility of methylation state vector matching the test methylation state vector 505.

In one arrangement, the computational burden of calculating probabilities and/or p-value scores may be further reduced by caching at least some calculations. For example, the analytic system may cache in transitory or persistent memory calculations of probabilities for possibilities of methylation state vectors (or windows thereof). If other fragments have the same CpG sites, caching the possibility probabilities allows for efficient calculation of p-value scores without needing to re-calculate the underlying possibility probabilities. Equivalently, the analytics system may calculate p-value scores for each of the possibilities of methylation state vectors associated with a set of CpG sites from vector (or window thereof). The analytics system may cache the p-value scores for use in determining the p-value scores of other fragments including the same CpG sites. Generally, the p-value scores of possibilities of methylation state vectors having the same CpG sites may be used to determine the p-value score of a different one of the possibilities from the same set of CpG sites.

*Sliding window*

In one arrangement, the analytics system uses 435 a sliding window to determine possibilities of methylation state vectors and calculate p-values. Rather than enumerating possibilities and calculating p-values for entire methylation state vectors, the analytics system enumerates possibilities and calculates p-values for only a window of sequential CpG sites, where the window is shorter in length (of CpG sites) than at least some fragments (otherwise, the window would serve no purpose). The window length may be static, user determined, dynamic, or otherwise selected.

In calculating p-values for a methylation state vector larger than the window, the window identifies the sequential set of CpG sites from the vector within the window starting from the first CpG site in the vector. The analytic system calculates a p-value score for the window including the first CpG site. The analytics system then "slides" the window to the second CpG site in the vector, and calculates another p-value score for the second window. Thus, for a window size $l$ and methylation vector length $m$, each methylation state vector will generate $m-l+l$ p-value scores. After completing the p-value calculations for each portion of the vector, the lowest p-value score from all sliding windows is taken as the overall p-value score for the methylation state vector. In another arrangement, the analytics system aggregates the p-value scores for the methylation state vectors to generate an overall p-value score.

Using the sliding window helps to reduce the number of enumerated possibilities of methylation state vectors and their corresponding probability calculations that would otherwise need to be performed. Example probability calculations are shown in **FIG. 5**, but generally the number of possibilities of methylation state vectors increases exponentially by a factor of 2 with the size of the methylation state vector. To give a realistic example, it is possible for fragments to have upwards of 54 CpG sites. Instead of computing probabilities for $2^{54} (\sim 1.8 \times 10^{16})$ possibilities to generate a single p-value, the analytics system can instead use a window of size 5 (for example) which results in 50 p-value calculations for each of the 50 windows of the methylation state vector for that fragment. Each of the 50 calculations enumerates $2^5$ (32) possibilities of methylation state vectors, which total results in $50 \times 2^5 (1.6 \times 10^3)$ probability calculations. This results in a vast reduction of calculations to be performed, with no meaningful hit to the accurate identification of anomalous fragments. This additional step can also be applied when validating 340 the control group with the validation group's methylation state vectors.

*Identifying fragments indicative of cancer*

The analytics system identifies 450 DNA fragments indicative of cancer from the filtered set of anomalously methylated fragments.

*Hypomethylated and hypermethylated fragments*

According to a first method, the analytics system may identify DNA fragments that are deemed hypomethylated or hypermethylated as fragments indicative of cancer from the filtered set of anomalously methylated fragments. Hypomethylated and hypermethylated fragments can be defined as fragments of a certain length of CpG sites (e.g., more than 3, 4, 5, 6, 7, 8, 9, 10, etc.) with a high percentage of methylated CpG sites (e.g., more than 80%, 85%, 90%, or 95%, or any other percentage within the range of 50%-100%) or a high percentage of unmethylated CpG sites (e.g., more than 80%, 85%, 90%, or 95%, or any other percentage within the range of 50%-100%).

*Probabilistic models*

According to a method described herein, the analytics system identifies fragments indicative of cancer utilizing probabilistic models of methylation patterns fitted to each cancer type and non-cancer type. The analytics system calculates log-likelihood ratios for a sample using DNA fragments in the genomic regions considering the various cancer types with the fitted probabilistic models for each cancer type and non-cancer type. The analytics system may determine a DNA fragment to be indicative of cancer based on whether at least one of the log-likelihood ratios considered against the various cancer types is above a threshold value.

In one arrangement of partitioning the genome, the analytics system partitions the genome into regions by multiple stages. In a first stage, the analytics system separates the genome into blocks of CpG sites. Each block is defined when there is a separation between two adjacent CpG sites that exceeds some threshold, e.g., greater than 200 bp, 300 bp, 400 bp, 500 bp, 600 bp, 700 bp, 800 bp, 900 bp, or 1,000 bp. From each block, the analytics system subdivides at a second stage each block into regions of a certain length, e.g., 500 bp, 600 bp, 700 bp, 800 bp, 900 bp, 1,000 bp, 1,100 bp, 1,200 bp, 1,300 bp, 1,400 bp, or 1,500 bp. The analytics system may further overlap adjacent regions by a percentage of the length, e.g., 10%, 20%, 30%, 40%, 50%, or 60%.

The analytics system analyzes sequence reads derived from DNA fragments for each region. The analytics system may process samples from tissue and/or high-signal cfDNA. High-signal cfDNA samples may be determined by a binary classification model, by cancer stage, or by another metric.

For each cancer type and non-cancer, the analytics system fits a separate probabilistic model for fragments. In one example, each probabilistic model is mixture model comprising a combination of a plurality of mixture components with each mixture component being an independent-sites model where methylation at each CpG site is assumed to be independent of methylation statuses at other CpG sites.

In alternate arrangements, calculation is performed with respect to each CpG site. Specifically, a first count is determined that is the number of cancerous samples (cancer count) that include an anomalously methylated DNA fragment overlapping that CpG, and a second count is determined that is the total number of samples containing fragments overlapping that CpG (total) in the set. Genomic regions can be selected based on the numbers, for example, based on criteria positively correlated to the number of cancerous samples (cancer count) that include a DNA fragment overlapping that CpG, and inversely correlated to the total number of samples containing fragments overlapping that CpG (total) in the set.

Cancer of various types having different TOO can be selected from the group consisting of breast cancer, uterine cancer, cervical cancer, ovarian cancer, bladder cancer, urothelial cancer of renal pelvis, renal cancer other than urothelial, prostate cancer, anorectal cancer, anal cancer, colorectal cancer, hepatobiliary cancer arising from hepatocytes, hepatobiliary cancer arising from cells other than hepatocytes, liver/bile-duct cancer, esophageal cancer, pancreatic cancer, squamous cell cancer of the upper gastrointestinal tract, upper gastrointestinal cancer other than squamous, head and neck cancer, lung cancer, lung adenocarcinoma, small cell lung cancer, squamous cell lung cancer and cancer other than adenocarcinoma or small cell lung cancer, neuroendocrine cancer, melanoma, thyroid cancer, sarcoma, plasma cell neoplasm, multiple myeloma, myeloid neoplasm, lymphoma, and leukemia.

In some arrangements, various cancer types can be classified and labeled using classification methods available in the art, such as the International Classification of Diseases for Oncology (ICD-O-3) (codes.iarc.fr) or the Surveillance, Epidemiology, and End Results Program (SEER) (seer.cancer.gov). In other arrangements, cancer types are classified in three orthogonal codes, (i) topographical codes, (ii) morphological codes, or (iii) behavioral codes. Under behavioral codes, benign tumor is 0, uncertain behavior is 1, carcinoma in situ is 2, malignant, primary site is 3 and malignant, metastatic site is 6.

In some arrangements, a cancer TOO can be selected from a group defined by the guideline that will be used to stage a detected cancer. For example, the reference, Amin, M.B., Edge, S., Greene, F., Byrd, D.R., Brookland, R.K., Washington, M.K., Gershenwald, J.E., Compton, C.C., Hess, K.R., Sullivan, D.C., Jessup, J.M., Brierley, J.D., Gaspar, L.E., Schilsky, R.L., Balch, C.M., Winchester, D.P., Asare, E.A., Madera, M., Gress, D.M., Meyer, L.R. (Eds.), AJCC Cancer Staging Manual, 8th edition, Springer, 2017, identifies groups of different cancers that are staged together following standard guidelines, typically, such staging is a next step in cancer management following its detection and diagnosis.

The analytics system can further calculate log-likelihood ratios ("R") for a fragment indicating a likelihood of the fragment being indicative of cancer considering the various cancer types with the fitted probabilistic models for each cancer type and non-cancer type, or for a cancer TOO. The two probabilities may be taken from probabilistic models fitted for each of the cancer types and the non-cancer type, the probabilistic models defined to calculate a likelihood of observing a methylation pattern on a fragment given each of the cancer types and the non-cancer type. For example, the probabilistic models may be defined fitted for each of the cancer types and the non-cancer type.

*Selection of genomic regions indicative of cancer*

The analytics system identifies 460 genomic regions indicative of cancer. To identify these informative regions, the analytics system calculates an information gain for each genomic region or more specifically each CpG site that describes an ability to distinguish between various outcomes.

A method for identifying genomic regions capable of distinguishing between cancer type and non-cancer type utilizes a trained classification model that can be applied on the set of anomalously methylated DNA molecules or fragments corresponding to, or derived from a cancerous or non-cancerous group. The trained classification model can be trained to identify any condition of interest that can be identified from the methylation state vectors.

In one arrangement, the trained classification model is a binary classifier trained based on methylation states for cfDNA fragments or genomic sequences obtained from a subject cohort with cancer or a cancer TOO, and a healthy subject cohort without cancer, and is then used to classify a test subject probability of having cancer, a cancer TOO, or not having cancer, based on anomalously methylation state vectors. In other arrangements, different classifiers may be trained using subject cohorts known to have particular cancer (e.g., breast, lung, prostrate, etc.); known to have cancer of particular TOO where the cancer is believed to originate; or known to have different stages of particular cancer (e.g., breast, lung, prostrate, etc.). In these arrangements, different classifiers may be trained using sequence reads obtained from samples enriched for tumor cells from subject cohorts known to have particular cancer (e.g., breast, lung, prostrate, etc.). Each genomic region's ability to distinguish between cancer type and non-cancer type in the classification model is used to rank the genomic regions from most informative to least informative in classification performance. The analytics system may identify genomic regions from the ranking according to information gain in classification between non-cancer type and cancer type.

*Computing information gain from hypomethylated and hypermethylated fragments indicative of cancer*

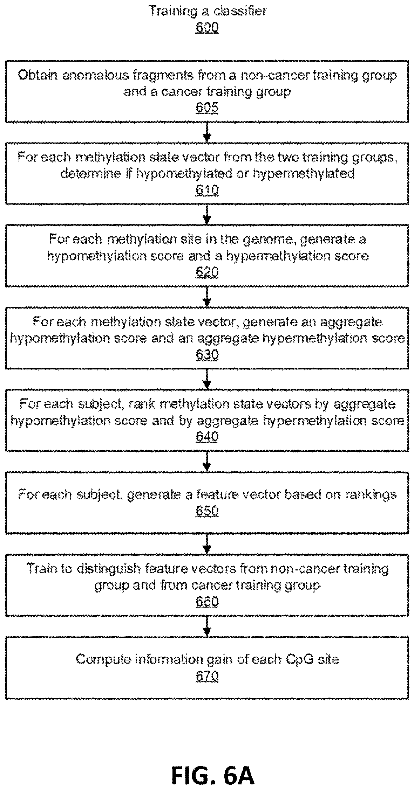

With fragments indicative of cancer, the analytics system may train a classifier according to a process 600 illustrated in **FIG. 6A,** according to an arrangement. The process 600 accesses two training groups of samples - a non-cancer group and a cancer group - and obtains 605 a non-cancer set of methylation state vectors and a cancer set of methylation state vectors comprising anomalously methylated fragments, e.g., via step 440 from the process 400.

The analytics system determines 610, for each methylation state vector, whether the methylation state vector is indicative of cancer. Here, fragments indicative of cancer may be defined as hypermethylated or hypomethylated fragments determined if at least some number of CpG sites have a particular state (methylated or unmethylated, respectively) and/or have a threshold percentage of sites that are the particular state (again, methylated or unmethylated, respectively). In one example, cfDNA fragments are identified as hypomethylated or hypermethylated, respectively, if the fragment overlaps at least 5 CpG sites, and at least 80%, 90%, or 100% of its CpG sites are methylated or at least 80%, 90%, or 100% are unmethylated.

In an alternate arrangement, the process considers portions of the methylation state vector and determines whether the portion is hypomethylated or hypermethylated, and may distinguish that portion to be hypomethylated or hypermethylated. This alternative resolves missing methylation state vectors which are large in size but contain at least one region of dense hypomethylation or hypermethylation. This process of defining hypomethylation and hypermethylation can be applied in step 450 of **FIG. 4.** In another arrangement, the fragments indicative of cancer may be defined according to likelihoods outputted from trained probabilistic models.

In one arrangement, the analytics system generates 620 a hypomethylation score ($P_{hypo}$) and a hypermethylation score ($P_{hyper}$) per CpG site in the genome. To generate either score at a given CpG site, the classifier takes four counts at that CpG site - (1) count of (methylations state) vectors of the cancer set labeled hypomethylated that overlap the CpG site; (2) count of vectors of the cancer set labeled hypermethylated that overlap the CpG site; (3) count of vectors of the non-cancer set labeled hypomethylated that overlap the CpG site; and (4) count of vectors of the non-cancer set labeled hypermethylated that overlap the CpG site. Additionally, the process may normalize these counts for each group to account for variance in group size between the non-cancer group and the cancer group. In alternative arrangements wherein fragments indicative of cancer are more generally used, the scores may be more broadly defined as counts of fragments indicative of cancer at each genomic region and/or CpG site.

In one arrangement, to generate 620 the hypomethylation score at a given CpG site, the process takes a ratio of (1) over (1) summed with (3). Similarly, the hypermethylation score is calculated by taking a ratio of (2) over (2) and (4). Additionally, these ratios may be calculated with an additional smoothing technique as discussed above. The hypomethylation score and the hypermethylation score relate to an estimate of cancer probability given the presence of hypomethylation or hypermethylation of fragments from the cancer set.

The analytics system generates 630 an aggregate hypomethylation score and an aggregate hypermethylation score for each anomalous methylation state vector. The aggregate hyper and hypo methylation scores, are determined based on the hyper and hypo methylation scores of the CpG sites in the methylation state vector. In one arrangement, the aggregate hyper and hypo methylation scores are assigned as the largest hyper and hypo methylation scores of the sites in each state vector, respectively. However, in alternate arrangements, the aggregate scores could be based on means, medians, or other calculations that use the hyper/hypo methylation scores of the sites in each vector.

The analytics system ranks 640 all of that subject's methylation state vectors by their aggregate hypomethylation score and by their aggregate hypermethylation score, resulting in two rankings per subject. The process selects aggregate hypomethylation scores from the hypomethylation ranking and aggregate hypermethylation scores from the hypermethylation ranking. With the selected scores, the classifier generates 650 a single feature vector for each subject. In one arrangement, the scores selected from either ranking are selected with a fixed order that is the same for each generated feature vector for each subject in each of the training groups. As an example, in one arrangement the classifier selects the first, the second, the fourth, and the eighth aggregate hyper methylation score, and similarly for each aggregate hypo methylation score, from each ranking and writes those scores in the feature vector for that subject.

The analytics system trains 660 a binary classifier to distinguish feature vectors between the cancer and non-cancer training groups. Generally, any one of a number of classification techniques may be used. In one arrangement the classifier is a non-linear classifier. In a specific arrangement, the classifier is a non-linear classifier utilizing a L2-regularized kernel logistic regression with a Gaussian radial basis function (RBF) kernel.

Specifically, in one arrangement, the number of non-cancer samples or different cancer type(s) ($n_{other}$) and the

number of cancer samples or cancer type(s) ($n_{cancer}$) having an anomalously methylated fragment overlapping a CpG site are counted. Then the probability that a sample is cancer is estimated by a score ("S") that positively correlates to $n_{cancer}$ and inversely correlated to $n_{other}$. The score can be calculated using the equation: $(n_{cancer}+ 1) / (n_{cancer} + n_{other}+ 2)$ or $(n_{cancer}) / (n_{cancer}+ n_{other})$. The analytics system computes 670 an information gain for each cancer type and for each genomic region or CpG site to determine whether the genomic region or CpG site is indicative of cancer. The information gain is computed for training samples with a given cancer type compared to all other samples. For example, two random variables 'anomalous fragment' ('AF') and 'cancer type' ('CT') are used. In one arrangement, AF is a binary variable indicating whether there is an anomalous fragment overlapping a given CpG site in a given samples as determined for the anomaly score / feature vector above. CT is a random variable indicating whether the cancer is of a particular type. The analytics system computes the mutual information with respect to CT given AF. That is, how many bits of information about the cancer type are gained if it is known whether there is an anomalous fragment overlapping a particular CpG site.

For a given cancer type, the analytics system uses this information to rank CpG sites based on how cancer specific they are. This procedure is repeated for all cancer types under consideration. If a particular region is commonly anomalously methylated in training samples of a given cancer but not in training samples of other cancer types or in healthy training samples, then CpG sites overlapped by those anomalous fragments will tend to have high information gains for the given cancer type. The ranked CpG sites for each cancer type are greedily added (selected) to a selected set of CpG sites based on their rank for use in the cancer classifier.

*Computing pairwise information gain from fragments indicative of cancer identified from probabilistic models*

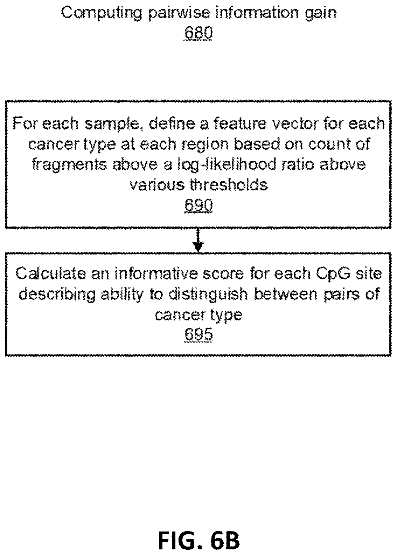

With fragments indicative of cancer identified according to a method described herein., the analytics may identify genomic regions according to the process 680 in **FIG. 6B.** The analytics system defines 690 a feature vector for each sample, for each region, for each cancer type by a count of DNA fragments that have a calculated log-likelihood ratio that the fragment is indicative of cancer above a plurality of thresholds, wherein each count is a value in the feature vector. In one arrangement, the analytics system counts the number of fragments present in a sample at a region for each cancer type with log-likelihood ratios above one or a plurality of possible threshold values. The analytics system defines a feature vector for each sample, by a count of DNA fragments for each genomic region for each cancer type that provides a calculated log-likelihood ratio for the fragment above a plurality of thresholds, wherein each count is a value in the feature vector. The analytics system uses the defined feature vectors to calculate an informative score for each genomic region describing that genomic region's ability to distinguish between each pair of cancer types. For each pair of cancer types, the analytics system ranks regions based on the informative scores. The analytics system may select regions based on the ranking according to informative scores.

The analytics system calculates 695 an informative score for each region describing that region's ability to distinguish between each pair of cancer types. For each pair of distinct cancer types, the analytics system may specify one type as a positive type and the other as a negative type. In one arrangement, a region's ability to distinguish between the positive type and the negative type is based on mutual information, calculated using the estimated fraction of cfDNA samples of the positive type and of the negative type for which the feature would be expected to be non-zero in the final assay, i.e., at least one fragment of that tier that would be sequenced in a targeted methylation assay. Those fractions are estimated using the observed rates at which the feature occurs in healthy cfDNA, and in high-signal cfDNA and/or tumor samples of each cancer type. For example, if a feature occurs frequently in healthy cfDNA, then it will also be estimated to occur frequently in cfDNA of any cancer type, and would likely result in a low informative score. The analytics system may choose a certain number of regions for each pair of cancer types from the ranking, e.g., 1024.

In additional arrangements, the analytics system further identifies predominantly hypermethylated or hypomethylated regions from the ranking of regions. The analytics system may load the set of fragments in the positive type(s) for a region that was identified as informative. The analytics system, from the loaded fragments, evaluates whether the loaded fragments are predominantly hypermethylated or hypomethylated. If the loaded fragments are predominately hypermethylated or hypomethylated, the analytics system may select probes corresponding to the predominant methylation pattern. If the loaded fragments are not predominantly hypermethylated or hypomethylated, the analytics system may use a mixture of probes for targeting both hypermethylation and hypomethylation. The analytics system may further identify a minimal set of CpG sites that overlap more than some percentage of the fragments.

In other arrangements, the analytics system, after ranking the regions based on informative scores, labels each region with the lowest informative ranking across all pairs of cancer types. For example, if a region was the 10th-most-informative region for distinguishing breast from lung, and the 5th-most-informative for distinguishing breast from colorectal, then it would be given an overall label of "5". The analytics system may design probes starting with the lowest-labeled regions while adding regions to the panel, e.g., until the panel's size budget has been exhausted.

*Off-target genomic regions*

In some arrangements, probes targeting selected genomic regions are further filtered 475 based on the number of their off-target regions. This is for screening probes that pull down too many cfDNA fragments corresponding to, or derived from, off-target genomic regions. Exclusion of probes having many off-target regions can be valuable by decreasing off-target rates and increasing target coverage for a given amount of sequencing.

An off-target genomic region is a genomic region that has sufficient homology to a target genomic region, such that DNA molecules or fragments derived from off-target genomic regions are hybridized to and pulled down by a probe designed to hybridize to a target genomic region. An off-target genomic region can be a genomic region (or a converted sequence of that same region) that aligns to a probe along at least 35bp, 40bp, 45bp, 50bp, 60bp, 70bp, or 80bp with at least an 80%, 85%, 90%, 95%, or 97% match rate. In one arrangement, an off-target genomic region is a genomic region (or a converted sequence of that same region) that aligns to a probe along at least 45bp with at least a 90% match rate. Various methods known in the art can be adopted to screen off-target genomic regions.

Exhaustively searching the genome to find all off-target genomic regions can be computationally challenging. In one arrangement, a k-mer seeding strategy (which can allow one or more mismatches) is combined to local alignment at the seed locations. In this case, exhaustive searching of good alignments can be guaranteed based on k-mer length, number of mismatches allowed, and number of k-mer seed hits at a particular location. This requires doing dynamic programing local alignment at a large number of locations, so this approach is highly optimized to use vector CPU instructions (e.g., AVX2, AVX512) and also can be parallelized across many cores within a machine and also across many machines connected by a network. A person of ordinary skill will recognize that modifications and variations of this approach can be implemented for the purpose of identifying off-target genomic regions.

In some arrangements, probes having sequence homology with off-target genomic regions, or DNA molecules corresponding to, or derived from off-target genomic regions comprising more than a threshold number are excluded (or filtered) from the panel. For example, probes having sequence homology with off-target genomic regions, or DNA molecules corresponding to, or derived from off-target genomic regions from more than 30, more than 25, more than 20, more than 18, more than 15, more than 12, more than 10, or more than 5 off-target regions are excluded.

In some arrangement, probes are divided into 2, 3, 4, 5, 6, or more separate groups depending on the numbers of off-target regions. For example, probes having sequence homology with no off-target regions or DNA molecules corresponding to, or derived from off-target regions are assigned to high-quality group, probes having sequence homology with 1-18 off-target regions or DNA molecules corresponding to, or derived from 1-18 off-target regions, are assigned to low-quality group, and probes having sequence homology with more than 19 off-target regions or DNA molecules corresponding to, or derived from 19 off-target regions, are assigned to poor-quality group. Other cut-off values can be used for the grouping.

In some arrangements, probes in the lowest quality group are excluded. In some arrangements, probes in groups other than the highest-quality group are excluded. In some arrangements, separate panels are made for the probes in each group. In some arrangements, all the probes are put on the same panel, but separate analysis is performed based on the assigned groups.

In some arrangements, a panel comprises a larger number of high-quality probes than the number of probes in lower groups. In some arrangements, a panel comprises a smaller number of poor-quality probes than the number of probes in other group. In some arrangements, more than 95%, 90%, 85%, 80%, 75%, or 70% of probes in a panel are high-quality probes. In some arrangements, less than 35%, 30%, 20%, 10%, 5%, 4%, 3%, 2% or 1% of the probes in a panel are low-quality probes. In some arrangements, less than 5%, 4%, 3%, 2% or 1% of the probes in a panel are poor-quality probes. In some arrangements, no poor-quality probes are included in a panel.

In some arrangements, probes having below 50%, below 40%, below 30%, below 20%, below 10% or below 5% are excluded. In some arrangements, probes having above 30%, above 40%, above 50%, above 60%, above 70%, above 80%, or above 90% are selectively included in a panel.

## Methods of using cancer assay panel

In yet another arrangement, methods of using a cancer assay panel are provided. The methods can comprise steps of treating DNA molecules or fragments to convert unmethylated cytosines to uracils (e.g., using bisulfite treatment), applying a cancer panel (as described herein) to the converted DNA molecules or fragments, enriching a subset of converted DNA molecules or fragments that hybridize (or bind) to the probes in the panel, and detecting the nucleic acid sequence and determining the methylation status thereof, for example, by sequencing the enriched cfDNA fragments. In some arrangements, the sequence reads can be compared to a reference genome (e.g., a human reference genome), allowing for identification of methylation states at a plurality of CpG sites within the DNA molecules or fragments and thus provide information relevant to detecting cancer. While the present disclosure pays particular attention to sequencing based approaches for detecting nucleic acids and determining methylation status thereof (via sequence reads), the disclosure is broad enough to encompass other methods for detecting nucleic acids and determining methylation status thereof (such as other methylation-aware sequencing approaches (e.g., as described in WO 2014/043763), DNA microarrays (e.g., with labeled probes adhered or conjugated to a solid surface or DNA array chip), etc.

*Analysis of sequence reads*

In some arrangements, the sequence reads may be aligned to a reference genome using known methods in the art to determine alignment position information. The alignment position information may indicate a beginning position and an end position of a region in the reference genome that corresponds to a beginning nucleotide base and end nucleotide base of a given sequence read. Alignment position information may also include sequence read length, which can be determined from the beginning position and end position. A region in the reference genome may be associated with a gene or a segment of a gene.

In various arrangements, a sequence read is comprised of a read pair denoted as $R_1$ and $R_2$. For example, the first read $R_1$ may be sequenced from a first end of a nucleic acid fragment whereas the second read $R_2$ may be sequenced from the second end of the nucleic acid fragment. Therefore, nucleotide base pairs of the first read $R_1$ and second read $R_2$ may be aligned consistently (e.g., in opposite orientations) with nucleotide bases of the reference genome. Alignment position information derived from the read pair $R_1$ and $R_2$ may include a beginning position in the reference genome that corresponds to an end of a first read (e.g., $R_1$) and an end position in the reference genome that corresponds to an end of a second read (e.g., $R_2$). In other words, the beginning position and end position in the reference genome represent the likely location within the reference genome to which the nucleic acid fragment corresponds. An output file having SAM (sequence alignment map) format or BAM (binary alignment map) format may be generated and output for further analysis.

From the sequence reads, the location and methylation state for each of CpG site may be determined based on alignment to a reference genome. Further, a methylation state vector for each fragment may be generated specifying a location of the fragment in the reference genome (e.g., as specified by the position of the first CpG site in each fragment, or another similar metric), a number of CpG sites in the fragment, and the methylation state of each CpG site in the fragment whether methylated (e.g., denoted as M), unmethylated (e.g., denoted as U), or indeterminate (e.g., denoted as I). The methylation state vectors may be stored in temporary or persistent computer memory for later use and processing. Further, duplicate reads or duplicate methylation state vectors from a single subject may be removed. In an additional arrangement, it may be determined that a certain fragment has one or more CpG sites that have an indeterminate methylation status. Such fragments may be excluded from later processing or selectively included where downstream data model accounts for such indeterminate methylation statuses.

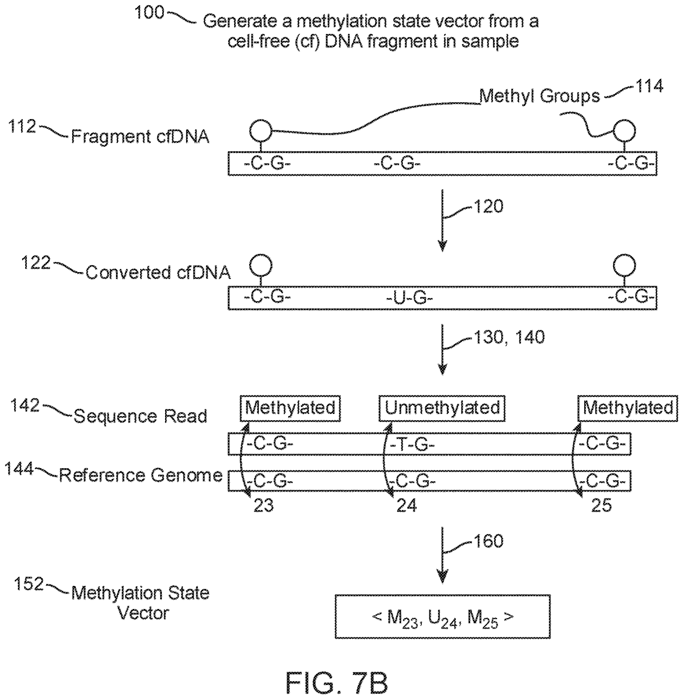

**FIG. 7B** is an illustration of the process 100 of **FIG. 7A** of sequencing a cfDNA fragment to obtain a methylation state vector, according to an arrangement. As an example, the analytics system takes a cfDNA fragment 112. In this example, the cfDNA fragment 112 contains three CpG sites. As shown, the first and third CpG sites of the cfDNA fragment 112 are methylated 114. During the treatment step 120, the cfDNA fragment 112 is converted to generate a converted cfDNA fragment 122. During the treatment 120, the second CpG site which was unmethylated has its cytosine converted to uracil. However, the first and third CpG sites are not converted.

After conversion, a sequencing library 130 is prepared and sequenced 140 generating a sequence read 142. The analytics system aligns 150 the sequence read 142 to a reference genome 144. The reference genome 144 provides the context as to what position in a human genome the fragment cfDNA originates from. In this simplified example, the analytics system aligns 150 the sequence read such that the three CpG sites correlate to CpG sites 23, 24, and 25 (arbitrary reference identifiers used for convenience of description). The analytics system thus generates information both on methylation status of all CpG sites on the cfDNA fragment 112 and which to position in the human genome the CpG sites map. As shown, the CpG sites on sequence read 142 which were methylated are read as cytosines. In this example, the cytosine's appear in the sequence read 142 only in the first and third CpG site which allows one to infer that the first and third CpG sites in the original cfDNA fragment were methylated. The second CpG site is read as a thymine (U is converted to T during the sequencing process), and thus, one can infer that the second CpG site was unmethylated in the original cfDNA fragment. With these two pieces of information, the methylation status and location, the analytics system generates 160 a methylation state vector 152 for the fragment cfDNA 112. In this example, the resulting methylation state vector 152 is $< M_{23}, U_{24}, M_{25} >$, wherein M corresponds to a methylated CpG site, U corresponds to an unmethylated CpG site, and the subscript numbers correspond to positions of each CpG site in the reference genome.

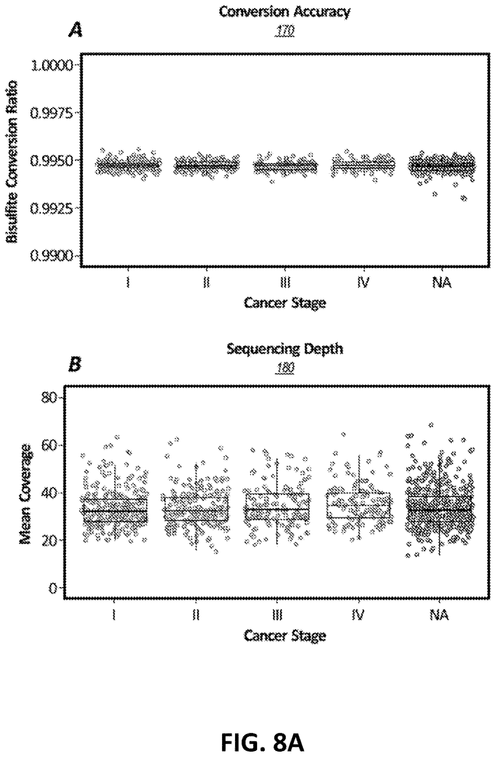

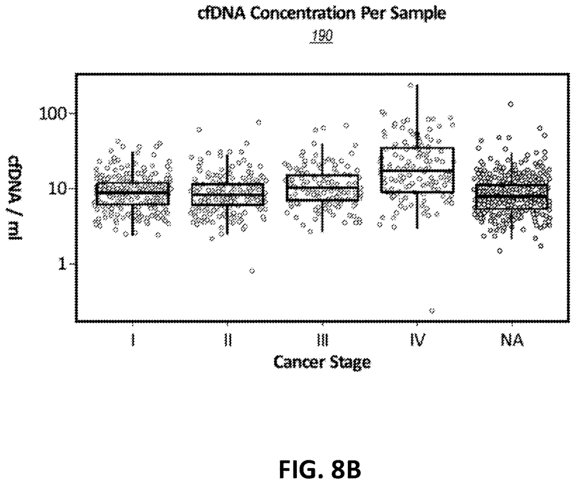

FIGs. 8A & 8B show three graphs of data validating consistency of sequencing from a control group. The first graph 170 shows conversion accuracy of conversion of unmethylated cytosines to uracil (step 120) on cfDNA fragment obtained from a test sample across subjects in varying stages of cancer - stage 0, stage I, stage II, stage III, stage IV, and non-cancer. As shown, there was uniform consistency in converting unmethylated cytosines on cfDNA fragments into uracils. There was an overall conversion accuracy of 99.47% with a precision at $\pm$ 0.024%. The second graph 180 compares coverage (depth of sequencing) over varying stages of cancer. Counting only sequence reads that were confidently mapped to a reference genome, the mean coverage over all groups was ~34. The third graph 190 shows the concentration of cfDNA per sample across varying stages of cancer.

*Detection of cancer*

Sequence reads obtained by the methods provided herein are further processed by automated algorithms. For example, the analytics system is used to receive sequencing data from a sequencer and perform various aspects of processing as described herein. The analytics system can be one of a personal computer (PC), a desktop computer, a laptop computer, a notebook, a tablet PC, a mobile device. A computing device can be communicatively coupled to the sequencer through a wireless, wired, or a combination of wireless and wired communication technologies. Generally, the computing device is configured with a processor and memory storing computer instructions that, when executed by the processor, cause the processor to perform steps as described in the remainder of this document. Generally, the amount of genetic data and data derived therefrom is sufficiently large, and the amount of computational power required so great, so as to be impossible to be performed on paper or by the human mind alone.

The clinical interpretation of methylation status of targeted genomic regions is a process that includes classifying the clinical effect of each or a combination of the methylation status and reporting the results in ways that are meaningful to a medical professional. The clinical interpretation can be based on comparison of the sequence reads with database specific to cancer or non-cancer subjects, and/or based on numbers and types of the cfDNA fragments having cancer-specific methylation patterns identified from a sample. In some arrangements, targeted genomic regions are ranked or classified based on their likeness to be differentially methylated in cancer samples, and the ranks or classifications are used in the interpretation process. The ranks and classifications can include (1) the type of clinical effect, (2) the strength of evidence of the effect, and (3) the size of the effect. Various methods for clinical analysis and interpretation of genome data can be adopted for analysis of the sequence reads. In some other arrangements, the clinical interpretation of the methylation states of such differentially methylated regions can be based on machine learning approaches that interpret a current sample based on a classification or regression method that was trained using the methylation states of such differentially methylated regions from samples from cancer and non-cancer patients with known cancer status, cancer type, cancer stage, TOO, etc.

The clinically meaning information can include the presence or absence of cancer generally, presence or absence of certain types of cancers, cancer stage, or presence or absence of other types of diseases. In some arrangements, the information relates to a presence or absence of one or more cancer types, selected from the group consisting of breast cancer, endometrial cancer, cervical cancer, ovarian cancer, bladder cancer, urothelial cancer of renal pelvis, renal cell carcinoma, prostate cancer, anorectal cancer, colorectal cancer, hepatocellular cancer, cholangiocarcinoma and hepatobiliary cancer, pancreatic cancer, upper GI adenocarcinoma, esophageal squamous cell cancer, head and neck cancer, squamous cell lung cancer, lung adenocarcinoma, small cell lung cancer, neuroendocrine cancer, melanoma, thyroid cancer, sarcoma, multiple myeloma, myeloid neoplasm, lymphoma, and leukemia. In some arrangements, the samples are not cancerous and are from subjects having white blood cell clonal expansion or no cancer.

*Cancer classifier*

In some examples, the assay panel described herein can be used with a cancer type classifier that predicts a disease state for a sample, such as a cancer or non-cancer prediction, a tissue of origin prediction, and/or an indeterminate prediction. In some examples, the cancer type classifier can generate features based on sequence reads by taking into account methylated or unmethylated fragments of DNA at certain genomic areas of interest. For instance, if the cancer type classifier determines that a methylation pattern at a fragment resembles that of a certain cancer type, then the cancer type classifier can set a feature for that fragment as 1, and otherwise if no such fragment is present, then the feature can be set as 0. In this way, the cancer type classifier can produce a set of binary features (merely by way of example, 30,000 features) for each sample. Further, in some examples, all or a portion of the set of binary features for a sample can be input into the cancer type classifier to provide a set of probability scores, such as one probability score per cancer type class and for a non-cancer type class. Furthermore, in some examples, the cancer type classifier can incorporate or otherwise be used in conjunction with thresholding to determine whether a sample is to be called as cancer or non-cancer, and/or indeterminate thresholding to reflect confidence in a specific TOO call. Such methods are described further below.

To train the cancer type classifier, the analytics system (e.g., analytics system 800) can obtain a set of training samples. In some examples, each training sample includes fragment file(s) (e.g., file containing sequence read data), a label corresponding to a type of cancer (TOO) or non-cancer status of the sample, and/or sex of the individual of the sample. The analytics system can utilize the training set to train the cancer type classifier to predict the disease state of the sample.

In some examples, for training, the analytics system divides the genome (e.g., whole genome) or a subset of the genome (e.g., targeted methylation regions) into regions. Merely by way of example, portions of the genome can be separated into "blocks" of CpGs, whereby a new block begins whenever there is a separation between nearest-neighbor CpGs is at least a minimum separation distance (e.g., at least 500 bp). Further, in some examples, each block can be divided into 1000 bp regions and positioned such that neighboring regions have a certain amount (e.g., 50% or 500 bp) of overlap.

Furthermore, in some examples, the analytics system can split the training set into K subsets or folds to be used in a K-fold cross-validation. In some examples, the folds can be balanced for cancer/non-cancer status, tissue of origin, cancer stage, age (e.g., grouped in 10yr buckets), and/or smoking status. In some examples, the training set is split into 5 folds, whereby 5 separate classifiers are trained, in each case training on 4/5 of the training samples and using the remaining 1/5 for validation.

During training with the training set, the analytics system can, for each cancer type (and for healthy cfDNA), fit a probabilistic model to the fragments deriving from the samples of that type. As used herein a "probabilistic model" is any mathematical model capable of assigning a probability to a sequence read based on methylation status at one or more sites on the read. During training, the analytics system fits sequence reads derived from one or more samples from subjects having a known disease and can be used to determine sequence reads probabilities indicative of a disease state utilizing methylation information or methylation state vectors. In particular, in some cases, the analytics system determines observed rates of methylation for each CpG site within a sequence read. The rate of methylation represents a fraction or percentage of base pairs that are methylated within a CpG site. The trained probabilistic model can be parameterized by products of the rates of methylation. In general, any known probabilistic model for assigning probabilities to sequence reads from a sample can be used. For example, the probabilistic model can be a binomial model, in which every site (e.g., CpG site) on a nucleic acid fragment is assigned a probability of methylation, or an independent sites model, in which each CpG's methylation is specified by a distinct methylation probability with methylation at one site assumed to be independent of methylation at one or more other sites on the nucleic acid fragment.

In some examples, the probabilistic model is a Markov model, in which the probability of methylation at each CpG site is dependent on the methylation state at some number of preceding CpG sites in the sequence read, or nucleic acid molecule from which the sequence read is derived. See, e.g., U.S. Pat. Appl. No. 16/352,602 (published as US 2019-0287652 A1), entitled "Anomalous Fragment Detection and Classification," and filed March 13, 2019.

In some examples, the probabilistic model is a "mixture model" fitted using a mixture of components from underlying models. For example, in some embodiments, the mixture components can be determined using multiple independent sites models, where methylation (e.g., rates of methylation) at each CpG site is assumed to be independent of methylation at other CpG sites. Utilizing an independent sites model, the probability assigned to a sequence read, or the nucleic acid molecule from which it derives, is the product of the methylation probability at each CpG site where the sequence read is methylated and one minus the methylation probability at each CpG site where the sequence read is unmethylated. In accordance with this example, the analytics system determines rates of methylation of each of the mixture components. The mixture model is parameterized by a sum of the mixture components each associated with a product of the rates of methylation. A probabilistic model *Pr* of *n* mixture components can be represented as:

$$Pr(fragment|\{\beta_{ki}, f_k\}) = \sum_{k=1}^{n} f_k \prod_i \beta_{ki}^{m_i} (1 - \beta_{ki})^{1-m_i}$$

For an input fragment, $m_i \in \{0,1\}$ represents the fragment's observed methylation status at position $i$ of a reference genome, with 0 indicating unmethylation and 1 indicating methylation. A fractional assignment to each mixture component $k$ is $f_k$, where $f_k \geq 0$ and $\sum_{k=1}^{n} f_k = 1$. The probability of methylation at position $i$ in a CpG site of mixture component $k$ is $\beta_{ki}$. Thus, the probability of unmethylation is $1 - \beta_{ki}$. The number of mixture components $n$ can be 1, 2, 3, 4, 5, 6, 7, 8, 9, 10, etc.

In some examples, the analytics system fits the probabilistic model using maximum-likelihood estimation to identify a set of parameters $\{\beta_{ki}, f_k\}$ that maximizes the log-likelihood of all fragments deriving from a disease state, subject to a regularization penalty applied to each methylation probability with regularization strength *r*. The maximized quantity for N total fragments can be represented as:

$$\sum_{j}^{N} ln\left(Pr\left(fragment_j | \{\beta_{ki}, f_k\}\right)\right) + r \cdot ln\left(\beta_{ki}(1-\beta_{ki})\right)$$

In some examples, the analytics system performs fits separately for each cancer type and for healthy cfDNA. As one of skill in the art would appreciate, other means can be used to fit the probabilistic models or to identify parameters that maximize the log-likelihood of all sequence reads derived from the reference samples. For example, in some examples, Bayesian fitting (using e.g., Markov chain Monte Carlo), in which each parameter is not assigned a single value but instead is associated to a distribution, is used. In some examples, gradient-based optimization, in which the gradient of the likelihood (or log-likelihood) with respect to the parameter values is used to step through parameter space towards an optimum, is used. In still some examples, expectation-maximization, in which a set of latent parameters (such as identities of the mixture component from which each fragment is derived) are set to their expected values under the previous model parameters, and then the model's parameters are assigned to maximize the likelihood conditional on the assumed values of those latent variables. The two-step process is then repeated until convergence.

Further, in some examples, the analytics system can generate features for each sample in the training set. For example, for each sample (regardless of label), in each region, for each cancer type, for each fragment, the analytics system can evaluate the log-likelihood ratio R with the fitted probabilistic models according to:

$$R_{cancer\ type\ A}(fragment) \equiv ln\left(\frac{Pr\left(fragment | cancer\ type\ A\right)}{Pr\left(fragment | healthy\ cfDNA\right)}\right)$$

Next, for each sample, for each region, for each cancer type, for each of a set of "tier" values, the analytics system can count the number of fragments with Rcancer type > tier and assign those counts as non-negative integer-valued features. For example, the tiers include threshold values of 1, 2, 3, 4, 5, 6, 7, 8, and 9, resulting in each region hosting 9 features per cancer type.

In some examples, the analytics system can select certain features for inclusion in a feature vector for each sample. For example, for each pair of distinct cancer types, the analytics system can specify one type as the "positive type" and the other as the "negative type" and rank the features by their ability to distinguish those types. In some cases, the ranking is based on mutual information calculated by the analytics system. For example, the mutual information can be calculated using the estimated fraction of samples of the positive type and negative type (e.g., cancer types A and B) for which the feature is expected to be nonzero in a resulting assay. For instance, if a feature occurs frequently in healthy cfDNA, the analytics system determines the feature is unlikely to occur frequently in cfDNA associated with various types of cancer. Consequently, the feature can be a weak measure in distinguishing between disease states. In calculating mutual information $I$, the variable $X$ is a certain feature (e.g., binary) and variable $Y$ represents a disease state, e.g., cancer type A or B:

$$I(X;Y) = \sum_{y \in Y} \sum_{x \in X} p(x,y)\ log\ log\left(\frac{p(x,y)}{p(x)p(y)}\right)$$

$$I \approx \frac{1}{2}\left(p(1|A) \cdot log\left(\frac{p(1|A)}{\frac{1}{2}\left(p(1|A)+p(1|B)\right)}\right) + p(1|B) \cdot log\left(\frac{p(1|B)}{\frac{1}{2}\left(p(1|A)p(1|B)\right)}\right)\right)$$

$$p(1|A) = f_A + f_H - f_H f_A$$

The joint probability mass function of $X$ and $Y$ is $p(x,y)$ and the marginal probability mass functions are $p(x)$ and $p(y)$. The analytics system can assume that feature absence is uninformative and either disease state is equally likely *a priori*, for example, $p(Y=A) = p(Y=B) = 0.5$. The probability of observing (e.g., in cfDNA) a given binary feature of cancer type A is represented by $p(1|A)$, where $f_A$ is the probability of observing the feature in ctDNA samples from tumor (or high-signal cfDNA samples) associated with cancer type A, and $f_H$ is the probability of observing the feature in a healthy or non-cancer cfDNA sample.

In some examples, only features corresponding to the positive type are included in the ranking, and only when those features' predicted rate of occurrence is greater in the positive type than in the negative type. For example, if "liver" is the positive type and "breast" is the negative type, then only "liver_x" features are considered, and only if their estimated occurrence in liver cfDNA is greater than their estimated occurrence in breast cfDNA. Further, in some examples, for each region, for each cancer type pair (including non-cancer as a negative type), the analytics system keeps only the best performing tier. Further, in some examples, the analytics system transforms feature values by binarization, whereby any feature value greater than 0 is set to 1, such that all features are either 0 or 1.

In some examples, the analytics system trains a multinomial logistic regression classifier on the training data for a fold, and generates predictions for the held-out data. For example, for each of the K folds, one logistic regression can be trained for each combination of hyperparameters. Such hyperparameters can include L2 penalty and/or topK (e.g., the number of high-ranking regions to keep per tissue type pair (including non-cancer), as ranked by the mutual information procedure outlined above). For each set of hyperparameters, performance is evaluated on the cross-validated predictions of the full training set, and the set of hyperparameters with the best performance is selected for retraining on the full training set. In some examples, the analytics system uses log-loss as a performance metric, whereby the log-loss is calculated by taking the negative logarithm of the prediction for the correct label for each sample, and then summing over samples (i.e. a perfect prediction of 1.0 for the correct label would give a log-loss of 0).

To generate predictions for a new sample, feature values are calculated using the same method described above, but restricted to features (region/positive class combinations) selected under the chosen topK value. Generated features are then used to create a prediction using the logistic regression model trained above.

In some examples, the analytics trains a two-stage classifier. For example, the analytics system trains a binary cancer classifier to distinguish between the labels, cancer and non-cancer, based on the feature vectors of the training samples. In this case, the binary classifier outputs a prediction score indicating the likelihood of the presence or absence of cancer. In another example, the analytics system trains a multiclass cancer classifier to distinguish between many cancer types. In this multiclass cancer classifier, the cancer classifier is trained to determine a cancer prediction that comprises a prediction value for each of the cancer types being classified for. The prediction values can correspond to a likelihood that a given sample has each of the cancer types. For example, the cancer classifier returns a cancer prediction including a prediction value for breast cancer, lung cancer, and non-cancer. For example, the cancer classifier may return a cancer prediction for a test sample including a prediction score for breast cancer, lung cancer, and/or no cancer.

The analytics system can train the cancer classifier according to any one of a number of methods. As an example, the binary cancer classifier may be a L2-regularized logistic regression classifier that is trained using a log-loss function. As another example, the multi-cancer (TOO) classifier may be a multinomial logistic regression. In practice either type of cancer classifier may be trained using other techniques. These techniques are numerous including potential use of kernel methods, machine learning algorithms such as multilayer neural networks, etc. In particular, methods as described in PCT/US2019/022122 and U.S. Patent. App. No. 16/352,602 (published as US 2019-0287652 A1). Still further, in some examples, the TOO classifier is trained only on cancer samples that were successfully called as cancer by the binary classifier, thereby ensuring sufficient cancer signal in the cancer sample. On the other hand, in some examples, the binary classifier is trained on the training samples regardless of TOO.

*Exemplary sequencer and analytics system*

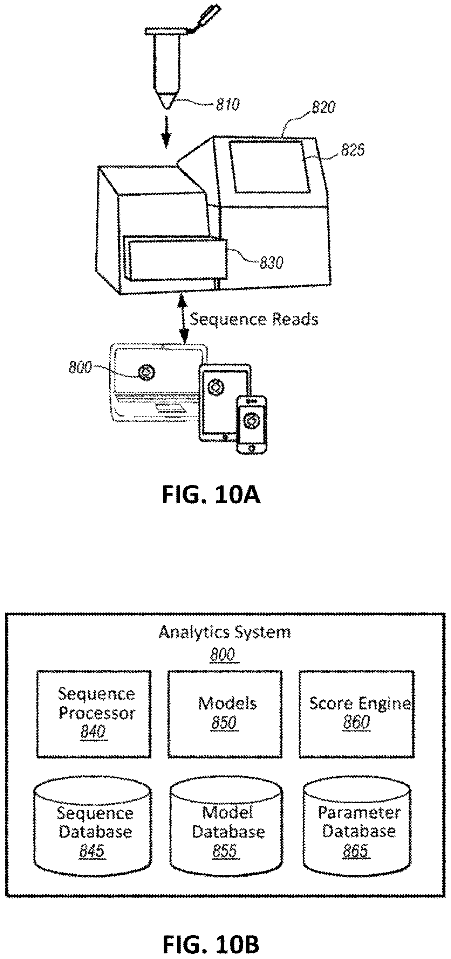

**FIG. 10A** is a flowchart of systems and devices for sequencing nucleic acid samples according to one arrangement. This illustrative flowchart includes devices such as a sequencer 820 and an analytics system 800. The sequencer 820 and the analytics system 800 may work in tandem to perform one or more steps in the processes described herein.

In various arrangements, the sequencer 820 receives an enriched nucleic acid sample 810. As shown in **FIG. 10A,** the sequencer 820 can include a graphical user interface 825 that enables user interactions with particular tasks (e.g., initiate sequencing or terminate sequencing) as well as one more loading stations 830 for loading a sequencing cartridge including the enriched fragment samples and/or for loading necessary buffers for performing the sequencing assays. Therefore, once a user of the sequencer 820 has provided the necessary reagents and sequencing cartridge to the loading station 830 of the sequencer 820, the user can initiate sequencing by interacting with the graphical user interface 825 of the sequencer 820. Once initiated, the sequencer 820 performs the sequencing and outputs the sequence reads of the enriched fragments from the nucleic acid sample 810.

In some arrangements, the sequencer 820 is communicatively coupled with the analytics system 800. The analytics system 800 includes some number of computing devices used for processing the sequence reads for various applications such as assessing methylation status at one or more CpG sites, variant calling or quality control. The sequencer 820 may provide the sequence reads in a BAM file format to the analytics system 800. The analytics system 800 can be communicatively coupled to the sequencer 820 through a wireless, wired, or a combination of wireless and wired communication technologies. Generally, the analytics system 800 is configured with a processor and non-transitory computer-readable storage medium storing computer instructions that, when executed by the processor, cause the processor to process the sequence reads or to perform one or more steps of any of the methods or processes disclosed herein.

In some arrangements, the sequence reads may be aligned to a reference genome using known methods in the art to determine alignment position information. Alignment position may generally describe a beginning position and an end position of a region in the reference genome that corresponds to a beginning nucleotide based and an end nucleotide base of a given sequence read. Corresponding to methylation sequencing, the alignment position information may be generalized to indicate a first CpG site and a last CpG site included in the sequence read according to the alignment to the reference genome. The alignment position information may further indicate methylation statuses and locations of all CpG sites in a given sequence read. A region in the reference genome may be associated with a gene or a segment of a gene; as such, the analytics system 800 may label a sequence read with one or more genes that align to the sequence read. In one embodiment, fragment length (or size) is determined from the beginning and end positions.

In various arrangements, for example when a paired-end sequencing process is used, a sequence read is comprised of a read pair denoted as R_1 and R_2. For example, the first read R_1 may be sequenced from a first end of a double-stranded DNA (dsDNA) molecule whereas the second read R_2 may be sequenced from the second end of the double-stranded DNA (dsDNA). Therefore, nucleotide base pairs of the first read R_1 and second read R_2 may be aligned consistently (e.g., in opposite orientations) with nucleotide bases of the reference genome. Alignment position information derived from the read pair R_1 and R_2 may include a beginning position in the reference genome that corresponds to an end of a first read (e.g., R _1) and an end position in the reference genome that corresponds to an end of a second read (e.g., R_2). In other words, the beginning position and end position in the reference genome represent the likely location within the reference genome to which the nucleic acid fragment corresponds. In one embodiment, the read pair R_1 and R_2 can be assembled into a fragment, and the fragment used for subsequent analysis and/or classification. An output file having SAM (sequence alignment map) format or BAM (binary) format may be generated and output for further analysis.

Referring now to FIG. 14B, FIG. 14B is a block diagram of an analytics system 800 for processing DNA samples according to one arrangement. The analytics system implements one or more computing devices for use in analyzing DNA samples. The analytics system 800 includes a sequence processor 840, sequence database 845, model database 855, models 850, parameter database 865, and score engine 860. In some arrangements, the analytics system 800 performs one or more steps in the processes 300 of **FIG. 3A,** 340 of **FIG. 3B,** 400 of **FIG. 4,** 500 of **FIG. 5,** 600 of **FIG. 6A,** or 680 of **FIG. 6B** and other process described herein.

The sequence processor 840 generates methylation state vectors for fragments from a sample. At each CpG site on a fragment, the sequence processor 840 generates a methylation state vector for each fragment specifying a location of the fragment in the reference genome, a number of CpG sites in the fragment, and the methylation state of each CpG site in the fragment whether methylated, unmethylated, or indeterminate via the process 300 of **FIG. 3A.** The sequence processor 840 may store methylation state vectors for fragments in the sequence database 845. Data in the sequence database 845 may be organized such that the methylation state vectors from a sample are associated to one another.

Further, multiple different models 850 may be stored in the model database 855 or retrieved for use with test samples. In one example, a model is a trained cancer classifier for determining a cancer prediction for a test sample using a feature vector derived from anomalous fragments. The training and use of the cancer classifier is discussed elsewhere herein. The analytics system 800 may train the one or more models 850 and store various trained parameters in the parameter database 865. The analytics system 800 stores the models 850 along with functions in the model database 855.

During inference, the score engine 860 uses the one or more models 850 to return outputs. The score engine 860 accesses the models 850 in the model database 855 along with trained parameters from the parameter database 865. According to each model, the score engine receives an appropriate input for the model and calculates an output based on the received input, the parameters, and a function of each model relating the input and the output. In some use cases, the score engine 860 further calculates metrics correlating to a confidence in the calculated outputs from the model. In other use cases, the score engine 860 calculates other intermediary values for use in the model.

*Application*

In some arrangements, the methods, analytic systems and/or classifier described herein can be used to detect the presence (or absence) of cancer, monitor cancer progression or recurrence, monitor therapeutic response or effectiveness, determine a presence or monitor minimum residual disease (MRD), or any combination thereof. In some arrangements, the analytic systems and/or classifier may be used to identify the tissue or origin for a cancer. For instance, the systems and/or classifiers may be used to identify a cancer as of any of the following cancer types: breast cancer, uterine cancer, cervical cancer, ovarian cancer, bladder cancer, urothelial cancer of renal pelvis, renal cancer other than urothelial, prostate cancer, anorectal cancer, anal cancer, colorectal cancer, hepatobiliary cancer arising from hepatocytes, hepatobiliary cancer arising from cells other than hepatocytes, liver/bile-duct cancer, esophageal cancer, pancreatic cancer, squamous cell cancer of the upper gastrointestinal tract, upper gastrointestinal cancer other than squamous, head and neck cancer, lung cancer, lung adenocarcinoma, small cell lung cancer, squamous cell lung cancer and cancer other than adenocarcinoma or small cell lung cancer, neuroendocrine cancer, melanoma, thyroid cancer, sarcoma, plasma cell neoplasm, multiple myeloma, myeloid neoplasm, lymphoma, and leukemia. For example, as described herein, a classifier can be used to generate a likelihood or probability score (e.g., from 0 to 100) that a sample feature vector is from a subject with cancer. In some arrangements, the probability score is compared to a threshold probability to determine whether or not the subject has cancer. In other arrangements, the likelihood or probability score can be assessed at different time points (e.g., before or after treatment) to monitor disease progression or to monitor treatment effectiveness (e.g., therapeutic efficacy). In still other arrangements, the likelihood or probability score can be used to make or influence a clinical decision (e.g., detection of cancer, treatment selection, assessment of treatment effectiveness, etc.). For example, in one arrangement, if the likelihood or probability score exceeds a threshold, a physician can prescribe an appropriate treatment.

*Detection of cancers*

In some arrangements, the methods and/or classifier of the present invention are used to detect a cancer type in a subject suspected of having cancer. For example, a classifier (as described herein) can be used to determine a likelihood or probability score that a sample feature vector is from a subject that has a cancer type.

In one arrangement, a probability score of greater than or equal to 60 can indicated that the subject has the cancer type. In still other arrangements, a probability score greater than or equal to 65, greater than or equal to 70, greater than or equal to 75, greater than or equal to 80, greater than or equal to 85, greater than or equal to 90, or greater than or equal to 95, indicated that the subject has cancer type. In other embodiments, a probability score can indicate the severity of disease. For example, a probability score of 80 may indicate a more severe form, or later stage, of cancer compared to a score below 80 (e.g., a score of 70). Similarly, an increase in the probability score over time (e.g., at a second, later time point) can indicate disease progression or a decrease in the probability score over time (e.g., at a second, later time point) can indicate successful treatment.

In another arrangement, a cancer log-odds ratio can be calculated for a test subject by taking the log of a ratio of a probability of being a cancer type over a probability of not being the cancer type (i.e., one minus the probability of being the cancer type), as described herein. In accordance with this arrangement, a cancer log-odds ratio greater than 1 can indicate that the subject has a cancer type. In still other arrangements, a cancer type log-odds ratio greater than 1.2, greater than 1.3, greater than 1.4, greater than 1.5, greater than 1.7, greater than 2, greater than 2.5, greater than 3, greater than 3.5, or greater than 4, indicates that the subject has the cancer type. In other arrangements, a cancer log-odds ratio can indicate the severity of disease. For example, a cancer log-odds ratio greater than 2 may indicate a more severe form, or later stage, of a form of cancer compared to a score below 2 (e.g., a score of 1). Similarly, an increase in the cancer log-odds ratio over time (e.g., at a second, later time point) can indicate disease progression or a decrease in the cancer log-odds ratio over time (e.g., at a second, later time point) can indicate successful treatment.

The methods and systems of the present invention can be trained to detect or classify multiple cancer indications. For example, the methods, systems and classifiers of the present invention can be used to detect the presence of one or more, two or more, three or more, five or more, or ten or more different types of cancer.

In some arrangements, the cancer is one or more of head and neck cancer, liver/bile duct cancer, upper GI cancer, pancreatic/gallbladder cancer; colorectal cancer, ovarian cancer, lung cancer, multiple myeloma, lymphoid neoplasms, melanoma, sarcoma, breast cancer, and uterine cancer. In some arrangements, the cancer is one or more of anorectal cancer, bladder or urothelial cancer, or cervical cancer. In some arrangements, the cancer is one or more of breast cancer, uterine cancer, cervical cancer, ovarian cancer, bladder cancer, urothelial cancer of renal pelvis, renal cancer other than urothelial, prostate cancer, anorectal cancer, anal cancer, colorectal cancer, hepatobiliary cancer arising from hepatocytes, hepatobiliary cancer arising from cells other than hepatocytes, liver/bile-duct cancer, esophageal cancer, pancreatic cancer, squamous cell cancer of the upper gastrointestinal tract, upper gastrointestinal cancer other than squamous, head and neck cancer, lung cancer, lung adenocarcinoma, small cell lung cancer, squamous cell lung cancer and cancer other than adenocarcinoma or small cell lung cancer, neuroendocrine cancer, melanoma, thyroid cancer, sarcoma, plasma cell neoplasm, multiple myeloma, myeloid neoplasm, lymphoma, and leukemia.

In some arrangements, the likelihood or probability score can be assessed at different time points (e.g., or before or after treatment) to monitor disease progression or to monitor treatment effectiveness (e.g., therapeutic efficacy). For example, the present disclosure provides methods that involve obtaining a first sample (e.g., a first plasma cfDNA sample) from a cancer patient at a first time point, determining a first likelihood or probability score therefrom (as described herein), obtaining a second test sample (e.g., a second plasma cfDNA sample) from the cancer patient at a second time point, and determine a second likelihood or probability score therefrom (as described herein).

_Treatment (not claimed)_

Information obtained from any method described herein (e.g., the likelihood or probability score) can be used to make or influence a clinical decision (e.g., diagnosis of cancer, treatment selection, assessment of treatment effectiveness, etc.). For example, if the likelihood or probability score exceeds a threshold, a physician can prescribe an appropriate treatment (e.g., a resection surgery, radiation therapy, chemotherapy, and/or immunotherapy). Information such as a likelihood or probability score can be provided as a readout to a physician or subject.

A classifier (as described herein) can be used to determine a likelihood or probability score that a sample feature vector is from a subject that has cancer or a particular type of cancer (e.g., tissue of origin). An appropriate treatment (e.g., resection surgery or therapeutic) can be prescribed when the likelihood or probability exceeds a threshold. For example, if the likelihood or probability score is greater than or equal to 60, one or more appropriate treatments can be prescribed. If the likelihood or probability score is greater than or equal to 65, greater than or equal to 70, greater than or equal to 75, greater than or equal to 80, greater than or equal to 85, greater than or equal to 90, or greater than or equal to 95, one or more appropriate treatments can be prescribed. A cancer log-odds ratio can indicate the effectiveness of a cancer treatment. For example, an increase in the cancer log-odds ratio over time (e.g., at a second, after treatment) can indicate that the treatment was not effective. Similarly, a decrease in the cancer log-odds ratio over time (e.g., at a second, after treatment) can indicate successful treatment. If the cancer log-odds ratio is greater than 1, greater than 1.5, greater than 2, greater than 2.5, greater than 3, greater than 3.5, or greater than 4, one or more appropriate treatments can be prescribed.

In some cases, the treatment is one or more cancer therapeutic agents selected from the group consisting of a chemotherapy agent, a targeted cancer therapy agent, a differentiating therapy agent, a hormone therapy agent, and an immunotherapy agent. For example, the treatment can be one or more chemotherapy agents selected from the group consisting of alkylating agents, antimetabolites, anthracyclines, anti-tumor antibiotics, cytoskeletal disruptors (taxans), topoisomerase inhibitors, mitotic inhibitors, corticosteroids, kinase inhibitors, nucleotide analogs, platinum-based agents and any combination thereof. In some cases, the treatment is one or more targeted cancer therapy agents selected from the group consisting of signal transduction inhibitors (e.g. tyrosine kinase and growth factor receptor inhibitors), histone deacetylase (HDAC) inhibitors, retinoic receptor agonists, proteosome inhibitors, angiogenesis inhibitors, and monoclonal antibody conjugates. In some cases, the treatment is one or more differentiating therapy agents including retinoids, such as tretinoin, alitretinoin and bexarotene. In some cases, the treatment is one or more hormone therapy agents selected from the group consisting of anti-estrogens, aromatase inhibitors, progestins, estrogens, anti-androgens, and GnRH agonists or analogs. In one case, the treatment is one or more immunotherapy agents selected from the group comprising monoclonal antibody therapies such as rituximab (RITUXAN) and alemtuzumab (CAMPATH), non-specific immunotherapies and adjuvants, such as BCG, interleukin-2 (IL-2), and interferon-alfa, immunomodulating drugs, for instance, thalidomide and lenalidomide (REVLIMID). It is within the capabilities of a skilled physician or oncologist to select an appropriate cancer therapeutic agent based on characteristics such as the type of tumor, cancer stage, previous exposure to cancer treatment or therapeutic agent, and other characteristics of the cancer.

## EXAMPLES

The following examples are put forth so as to provide those of ordinary skill in the art with a complete disclosure and description of how to make and use the present description, and are not intended to limit the scope of what the inventors regard as their description nor are they intended to represent that the experiments below are all or the only experiments performed. Efforts have been made to ensure accuracy with respect to numbers used (e.g., amounts, temperature, etc.) but some experimental errors and deviations should be accounted for.

### EXAMPLE 1 - Analysis of probe qualities

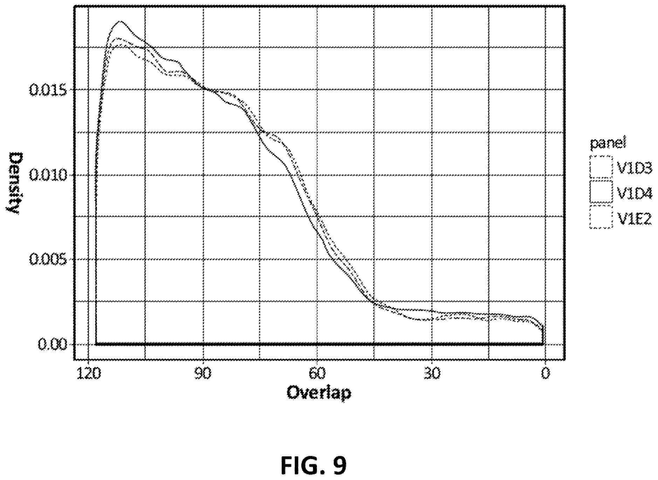

To test how much overlap between a cfDNA fragment and a probe is required to achieve a non-negligible amount of pulldown, various lengths of overlaps were tested using panels designed to include three different types of probes (V1D3, V1D4, V1E2) having various overlaps with 175bp target DNA fragments specific to each probe. Tested overlaps ranged between 0bp and 120bp. Samples comprising 175bp target DNA fragments were applied to the panel and washed, and then DNA fragments bound to the probes were collected. The amounts of the collected DNA fragments were measured and the amounts were plotted as densities over the sizes of overlaps as provided in **FIG. 9.**

There was no significant binding and pull down of target DNA fragments when there were less than 45 bp of overlaps. These results suggest that a fragment-probe overlap of at least 45bp is generally required to achieve a non-negligible amount of pulldown although this number can vary depending on the assay conditions.

Furthermore, it has been suggested that more than a 10% mismatch rate between the probe and fragment sequences in the region of overlap is sufficient to greatly disrupt binding, and thus pulldown efficiency. Therefore, sequences that can align to the probe along at least 45bp with at least a 90% match rate are candidates for off-target pulldown.

Thus, we have performed an exhaustive searching of all genomic regions having 45bp alignments with 90%+ match rate (i.e., off-target regions) for each probe. Specifically, we combined a k-mer seeding strategy (which can allow one or more mismatches) with local alignment at the seed locations. This guaranteed not missing any good alignments based on k-mer length, number of mismatches allowed, and number of k-mer seed hits at a particular location. This involves performing dynamic programing local alignment at a large number of locations, so the implementation was optimized to use vector CPU instructions (e.g., AVX2, AVX512) and parallelized across many cores within a machine and also across many machines connected by a network. This allows exhaustive search which is valuable in designing a high-performance panel (i.e., low off-target rate and high target coverage for a given amount of sequencing).

Following the exhaustive searching, each probe was scored based on the number of off-target regions. The best probes have a score of 1, meaning they match in only one place (high Q). Probes with a low score between 2-19 hits (low Q) were accepted but probes with a poor score more than 20 hits (poor Q) were discarded. Other cutoff values can be used for specific samples.

Numbers of high quality, low quality, and poor quality probes were then counted among probes targeting hypermethylated genomic regions or hypomethylated genomic regions.

**EXAMPLE 2 - Cancer assay panels for detecting specific-cancer types**

**Cancer types:** Cancer-specific panels were designed to detect cancer and/or cancer tissue of origin of fifteen (15) different cancer types. The 15 cancer types include (1) bladder cancer, (2) breast cancer, (3) cervical cancer, (4) colorectal cancer, (5) head and neck cancer, (6) hepatobiliary cancer, (7) lung cancer, (8) melanoma, (9) ovarian cancer, (10) pancreatic cancer, (11) prostate cancer, (12) renal cancer, (13) thyroid cancer, (14) upper gastrointestinal cancer, and (15) uterine cancer (see Lists 1-15). Cancer-specific classification was applied to the samples for relevant classification and labeling.

**Samples used for genomic region selection:** DNA samples for this work came from various sources.

The Circulating Cell-free Genome Atlas Study ("CCGA"; Clinical Trial.gov identifier NCT02889978) is a prospective, multi-center, case-control, observational study with longitudinal follow-up. De-identified biospecimens were collected from approximately 15,000 participants from 142 sites. Samples were selected to ensure a prespecified distribution of cancer types and non-cancers across sites in each cohort, and cancer and non-cancer samples were frequency age-matched by gender.

The Cancer Genome Atlas ("TCGA"; Clinical Trial.gov identifier NCT02889978) is a public resource developed through a collaboration between the National Cancer Institute (NCI) and the National Human Genome Research Institute (NHGRI).

Dissociated tumor cells (DTC) were acquired from Conversant.

Non-cancer cells were provided by Yuval Dor and Ben Glaser (Hebrew University) and originated from human tissue obtained from standard clinical procedures. For example, breast luminal and basal epithelial cells were from breast reduction surgery; colon epithelial cells were from tissue near the site of re-implantation following segmental resection for localized colon pathology; bone marrow cells were from joint replacement surgery; vascular and arterial endothelial cells were from vascular surgery; and head and neck epithelium was from tonsillectomy.

WGBS was performed on more than 1000 genomic DNA samples collected from healthy individuals and individuals diagnosed with cancers of various stages and tissues of origin. The samples included formaldehyde-fixed, paraffin-embedded (FFPE) tissue blocks, disseminated tumor cells (DTC) from cancers of different TOOs, bone marrow mononuclear cells (BMMC), white blood cells (WBC) and peripheral blood mononuclear cells (PBMC). The DTCs were subjected to negative selection to remove WBCs, fibroblasts, and endothelial cells using a negative selection kit (Miltenyi) prior to gDNA isolation. The negative selection yielded purified tumor cells that allowed differentially methylated regions to be more clearly identified.

The TCGA data was collected by hybridization of bisulfite-converted DNA fragments from 8809 samples to methylation-sensitive oligonucleotide arrays. β-values from this study represent the relative abundance of methylation at 480,000 individual CpG sites. 75,000 of these CpG sites were analyzed after excluding CpGs from noisy genomic regions (360,000) and CpG sites with cross-hybridizing probes (45,000). The TCGA data was analyzed using different algorithms because it describes methylation of individual CpG sites, whereas WGBS data reveals the methylation pattern of strings of adjacent CpG sites on DNA fragments.

**Tissue of Origin classes:** Each sample was categorized into one of twenty-five (25) different Tissue of Origin (TOO) classes: breast cancer, uterine cancer, cervical cancer, ovarian cancer, bladder cancer, urothelial cancer of renal pelvis, renal cancer other than urothelial, prostate cancer, anorectal cancer, colorectal cancer, hepatobiliary cancer arising from hepatocytes, hepatobiliary cancer arising from cells other than hepatocytes, pancreatic cancer, squamous cell cancer of the upper gastrointestinal tract, upper gastrointestinal cancer other than squamous, head and neck cancer, lung adenocarcinoma, small cell lung cancer, squamous cell lung cancer and cancer other than adenocarcinoma or small cell lung cancer, neuroendocrine cancer, melanoma, thyroid cancer, sarcoma, multiple myeloma, lymphoma, and leukemia. These TOO classes encompass 97% of the cancer incidence reported by the Surveillance, Epidemiology, and End Results program (SEER; seer.cancer.gov), after filtering out liquid, brain, small intestine, vagina+vulva and penis+testis. Rare incidence cancers like sarcoma, and neuroendocrine cancers were aggregated to guard against misclassification. International Classification of Diseases for Oncology (ICD-O-3) topographical, morphological, and behavioral codes and World Health Organization (WHO) topography designations were used to categorize individual samples into the TOO classes. For example, the 34 TCGA studies were mapped to 25 TOO classes as shown in **TABLE 1.** The TOO classification was iteratively refined against observed classification performance.

### TABLE 1 - Tissue of Origin (TOO) classification of TCGA types

| TOO class | TCGA type | N |
|---|---|---|
| Breast | BRCA | 779 |
| Renal | KIRC, KIRP, KICH | 657 |
| Brain | LLG, GBM | 654 |
| Upper GI | ESCA, STAD | 580 |
| Melanoma | SKCM, UVM | 550 |
| Head and neck | HNSC | 528 |
| Thyroid | THCA | 507 |
| Prostate | PRAD | 498 |
| Uterine | UCEC, UCS | 484 |
| Lung adenocarcinoma | LUAD | 444 |
| Bladder | BLCA | 409 |
| Colorectal | COAD, READ | 382 |
| Hepatobiliary hcc | LIHC | 377 |
| Lung squamous | LUSC | 370 |
| Cervical | CESC | 307 |
| Sarcoma | SARC | 261 |
| Adrenal | ACC, PCPG | 259 |
| Pancreas | PAAD | 184 |
| Leukemia | LAML, LCMI, | 140 |
| Testicular | TGCT | 134 |
| Thymus | THYM | 124 |
| Mesothelioma | MESO | 87 |
| Lymphoma | DLBC | 48 |
| Hepatobiliary biliary | CHOL | 36 |
| Ovarian | OV | 10 |

**Region selection:** For target selection, fragments having abnormal methylation patterns in cancer samples were selected using one or more method as described herein. Use of these methods allowed identification of low noise regions as putative targets. Among the low noise regions, fragments most informative in discriminating cancer types were ranked and selected.

Specifically, in some arrangements, when WGBS data were used, fragment sequences in the database were filtered based on p-value using a non-cancer distribution, and only fragments with $p < 0.001$ were retained, as described herein. In some cases, the selected cfDNAs were further filtered to retain only those that were at least 90% methylated or 90% unmethylated. Next, for each CpG site in the selected fragments, the numbers of cancer samples or non-cancer samples were counted that include fragments overlapping that CpG site. Specifically, P (cancer | overlapping fragment) for each CpG was calculated and genomic sites with high P values were selected as general cancer targets. By design, the selected fragments had very low noise (i.e., few non-cancer fragments overlapping).

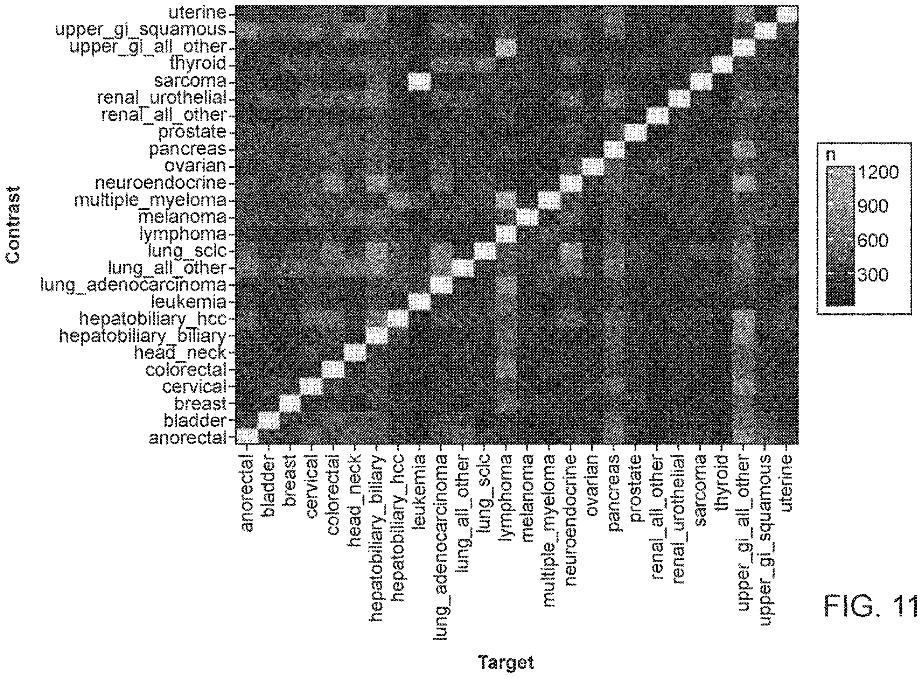

To find cancer type specific targets, similar selection processes were performed. CpG sites were ranked based on their information gain, comparing (i) the numbers of samples of a specific TOO or other samples, including both non-cancer samples and samples of a different TOO, (ii) the numbers of samples of a specific TOO or non-cancer samples, and/or (iii) the numbers of samples of a specific TOO or a different TOO that include fragments overlapping that CpG site. The process was applied to each of the 25 TOOs and the comparison was done for all pairwise combinations for 25 TOOs. For example, P (cancer of a TOO | overlapping fragment) was calculated and then compared with P (cancer of a different TOO | overlapping fragment). An outlier fragment in each TOO having much greater likelihood under cancer of a TOO than under cancer of a different TOO was selected as a target for the TOO. Accordingly, genomic regions selected by the pairwise comparisons included genomic regions differentially methylated to separate a target TOO and a contrast TOO. The numbers of genomic regions for differentiating each target TOO (x-axis) from a contrast TOO (y-axis) are provided in **FIG. 11.**

When TCGA data were used, CpG beta value indicating intensity of methylation was used to identify target genomic regions. This is because array data are not at CpG site levels, and thus they are prone to result in false positives. To avoid false positives, CpG sites were converted into 350 bp bins across the genome. Beta values of each bin were calculated as the mean of CpG beta values in that bin. Bins with less than 2 CpG's were excluded from the analysis. Next, bins were selected with beta difference of> 0.95 between (i) samples of a specific TOO and other samples, including both non-cancer samples and samples of a different TOO, (ii) samples of a specific TOO and non-cancer samples, and/or (iii) samples of a specific TOO and a different TOO that include fragments overlapping that CpG site.

Genomic regions selected as described above were then filtered based on the numbers of their off-target genomic regions as specified herein. Specifically, numbers of genomic locations that have >=45bp alignments with >=90% identity were calculated as the numbers of off-target genomic regions. Genomic regions having off-target genomic regions more than 20 were discarded.

Various lists of target genomic regions selected as described in this section are identified in **TABLE 2** (see Lists 1-15).

**TABLE 2** - **Summary of Lists 1-15**

For each list, the table identifies the cancer type detected, the total number of target genomic regions in the list, a range of SEQ ID NOs corresponding to all target genomic regions in the list to be found in the sequence listing submitted with this application, and a panel size (total of the lengths of all target genomic regions in the list). The sequence listing identifies the chromosomal location of each target genomic region, whether cfDNA fragments to be enriched from the region are hypermethylated genomic region. to a known human is available from also available from or hypomethylated, and the The chromosome numbers and reference genome, hg19. The Genome Reference Consortium Genome Browser provided sequence of the start and sequence of with a reference by Santa Cruz of one DNA stop of the human reference Genomics strand positions are reference number, Institute. of the target are provided relative reference genome, hg19, GRCh37/hg19, and Institute.

| List | Cancer type detected | Target Genomic Regions | SEQ ID NOs First | SEQ ID NOs Last | PanelSize (kb) |
|---|---|---|---|---|---|
| 1 | Bladder | 345 | 1 | 345 | 15.2 |
| 2 | Breast | 881 | 346 | 1226 | 45.0 |
| 3 | Cervical | 8 | 1227 | 1234 | 0.3 |
| 4 | Colorectal | 701 | 1235 | 1935 | 30.8 |
| 5 | Head and neck | 177 | 1936 | 2112 | 8.7 |
| 6 | Hepatobiliary | 335 | 2113 | 2447 | 24.0 |
| 7 | Lung | 491 | 2448 | 2938 | 22.4 |
| 8 | Melanoma | 78 | 2939 | 3016 | 3.4 |
| 9 | Ovarian | 881 | 3017 | 3897 | 37.0 |
| 10 | Pancreatic | 29 | 3898 | 3926 | 1.3 |
| 11 | Prostate | 784 | 3927 | 4710 | 37.7 |
| 12 | Renal | 517 | 4711 | 5227 | 22.6 |
| 13 | Thyroid | 23 | 5228 | 5250 | 1.0 |
| 14 | Upper gastrointestinal | 226 | 5251 | 5476 | 14.9 |
| 15 | Uterine | 240 | 5477 | 5716 | 10.9 |

**EXAMPLE 3 - Cancer Assay Panels for Diagnosing Specific Cancer Types**

Additional cancer assay panels were designed to identify specific cancer types in a manner analogous to that set forth in Example 2. Various lists of target genomic regions selected as described in this section are identified in **TABLE 3** (see Lists 16-49). The target genomic regions of Lists 16-32 contain subsets of the methylation sites of the target genomic regions of Lists 33-49, respectively.

**TABLE 3 - Summary of Lists 16-49**

For each list, the table identifies the cancer type detected, the total number of target genomic regions in the list, a range of SEQ ID NOs corresponding to all target genomic regions in the list to be found in the sequence listing submitted with this application, and a panel size (total of the lengths of all target genomic regions in the list). The sequence listing identifies the chromosomal location of each target genomic region, whether cfDNA fragments to be enriched from the region are hypermethylated or hypomethylated, and the sequence of one DNA strand of the target genomic region. The chromosome numbers and the start and stop positions are provided relative to a known human reference genome, hg19. The sequence of the human reference genome, hg19, is available from Genome Reference Consortium with a reference number, GRCh37/hg19, and also available from Genome Browser provided by Santa Cruz Genomics Institute.

| List | Cancer type detected | Target Genomic Regions | SEQ ID NOs First | SEQ ID NOs Last | Panel Size (kb) |
|------|---------------------|------------------------|-------|------|-----------------|
| 16 | Anorectal | 937 | 5717 | 6653 | 198.8 |
| 17 | Bladder and urothelial | 977 | 6654 | 7630 | 212.6 |
| 18 | Breast | 1201 | 7631 | 8831 | 243.9 |
| 19 | Cervical | 1258 | 8832 | 10089 | 278.1 |
| 20 | Colorectal | 771 | 10090 | 10860 | 143.6 |
| 21 | Head and neck | 1143 | 10861 | 12003 | 236.3 |
| 22 | Liver and bile duct | 1088 | 12004 | 13091 | 256.1 |
| 23 | Lung | 1321 | 13092 | 14412 | 236.5 |
| 24 | Melanoma | 907 | 14413 | 15319 | 244.8 |
| 25 | Ovarian | 853 | 15320 | 16172 | 181.0 |
| 26 | Pancreatic and gallbladder | 1003 | 16173 | 17175 | 193.3 |
| 27 | Prostate | 953 | 17176 | 18128 | 222.2 |
| 28 | Renal | 881 | 18129 | 19009 | 202.8 |
| 29 | Sarcoma | 1014 | 19010 | 20023 | 260.1 |
| 30 | Thyroid | 748 | 20024 | 20771 | 170.8 |
| 31 | Upper gastrointestinal | 793 | 20772 | 21564 | 169.7 |
| 32 | Uterine | 1170 | 21565 | 22734 | 252.9 |
| 33 | Anorectal | 933 | 22735 | 23667 | 669.7 |
| 34 | Bladder and urothelial | 1066 | 23668 | 24733 | 575.9 |
| 35 | Breast | 1272 | 24734 | 26005 | 695.4 |
| 36 | Cervical | 1384 | 26006 | 27389 | 950.7 |
| 37 | Colorectal | 905 | 27390 | 28294 | 708.7 |
| 38 | Head and neck | 1256 | 28295 | 29550 | 770.5 |
| 39 | Liver and bile duct | 1158 | 29551 | 30708 | 814.3 |
| 40 | Lung | 1660 | 30709 | 32368 | 1043.4 |

(continued)

For each list, the table identifies the cancer type detected, the total number of target genomic regions in the list, a range of SEQ ID NOs corresponding to all target genomic regions in the list to be found in the sequence listing submitted with this application, and a panel size (total of the lengths of all target genomic regions in the list). The sequence listing identifies the chromosomal location of each target genomic region, whether cfDNA fragments to be enriched from the region are hypermethylated or hypomethylated, and the sequence of one DNA strand of the target genomic region. The chromosome numbers and the start and stop positions are provided relative to a known human reference genome, hg19. The sequence of the human reference genome, hg19, is available from Genome Reference Consortium with a reference number, GRCh37/hg19, and also available from Genome Browser provided by Santa Cruz Genomics Institute.

| List | Cancer type detected | Target Genomic Regions | SEQ ID NOs | | Panel Size (kb) |
|---|---|---|---|---|---|
| | | | First | Last | |
| 41 | Melanoma | 791 | 32369 | 33159 | 521.7 |
| 42 | Ovarian | 858 | 33160 | 34017 | 354.5 |
| 43 | Pancreatic and gallbladder | 1191 | 34018 | 35208 | 999.6 |
| 44 | Prostate | 895 | 35209 | 36103 | 484.8 |
| 45 | Renal | 865 | 36104 | 36968 | 474.2 |
| 46 | Sarcoma | 951 | 36969 | 37919 | 524.9 |
| 47 | Thyroid | 719 | 37920 | 38638 | 244.6 |
| 48 | Upper gastrointestinal | 854 | 38639 | 39492 | 890.5 |
| 49 | Uterine | 1239 | 39493 | 40731 | 805.1 |

## EXAMPLE 4 - Generation of a mixture model classifier

To maximize performance, the predictive cancer models described in this Example were trained using sequence data obtained from a plurality of samples from known cancer types and non-cancers from both CCGA sub-studies (CCGA1 and CCGA22), a plurality of tissue samples for known cancers obtained from CCGA1, and a plurality of non-cancer samples from the STRIVE study (See Clinical Trail.gov Identifier: NCT03085888 (//clinicaltrials.gov/ct2/show/NCT03085888)). The STRIVE study is a prospective, multi-center, observational cohort study to validate an assay for the early detection of breast cancer and other invasive cancers, from which additional non-cancer training samples were obtained to train the classifier described herein. The known cancer types included from the CCGA sample set included the following: breast, lung, prostate, colorectal, renal, uterine, pancreas, esophageal, lymphoma, head and neck, ovarian, hepatobiliary, melanoma, cervical, multiple myeloma, leukemia, thyroid, bladder, gastric, and anorectal. As such, a model can be a multi-cancer model (or a multi-cancer classifier) for detecting one or more, two or more, three or more, four or more, five or more, ten or more, or 20 or more different types of cancer.

The classifier performance data shown below was reported out for a locked classifier trained on cancer and non-cancer samples obtained from CCGA2, a CCGA sub-study, and on non-cancer samples from STRIVE. The individuals in the CCGA2 sub-study were different from the individuals in the CCGA1 sub-study whose cfDNA was used to select target genomes. From the CCGA2 study, blood samples were collected from individuals diagnosed with untreated cancer (including 20 tumor types and all stages of cancer) and healthy individuals with no cancer diagnosis (controls). For STRIVE, blood samples were collected from women within 28 days of their screening mammogram. Cell-free DNA (cfDNA) was extracted from each sample and treated with bisulfite to convert unmethylated cytosines to uracils. The bisulfite treated cfDNA was enriched for informative cfDNA molecules using hybridization probes designed to enrich bisulfite-converted nucleic acids derived from each of a plurality of targeted genomic regions in an assay panel comprising all of the genomic regions of Lists 1-16. The enriched bisulfite-converted nucleic acid molecules were sequenced using paired-end sequencing on an Illumina platform (San Diego, CA) to obtain a set of sequence reads for each of the training samples, and the resulting read pairs were aligned to the reference genome, assembled into fragments, and methylated and unmethylated CpG sites identified.

*Mixture model based featurization*

For each cancer type (including non-cancer) a probabilistic mixture model was trained and utilized to assign a probability to each fragment from each cancer and non-cancer sample based on how likely it was that the fragment would be observed in a given sample type.

*Fragment-level Analysis*

Briefly, for each sample type (cancer and non-cancer samples), for each region (where each region was used as-is if less than 1 kb, or else subdivided into 1 kb regions in length with a 50% overlap (e.g., 500 base pairs overlap) between adjacent regions), a probabilistic model was fit to the fragments derived from the training samples for each type of cancer and non-cancer. The probabilistic model trained for each sample type was a mixture model, where each of three mixture components was an independent-sites model in which methylation at each CpG is assumed to be independent of methylation at other CpGs. Fragments were excluded from the model if: they had a p-value (from a non-cancer Markov model) greater than 0.01; were marked as duplicate fragments; the fragments had a bag size of greater than 1 (for targeted methylation samples only); they did not cover at least one CpG site; or if the fragment was greater than 1000 bases in length. Retained training fragments were assigned to a region if they overlapped at least one CpG from that region. If a fragment overlapped CpGs in multiple regions, it was assigned to all of them.

*Local Source Models*

Each probabilistic model was fit using maximum-likelihood estimation to identify a set of parameters that maximized the log-likelihood of all fragments deriving from each sample type, subject to a regularization penalty.
Specifically, in each classification region, a set of probabilistic models were trained, one for each training label (i.e., one for each cancer type and one for non-cancer). Each model took the form of a Bernoulli mixture model with three components. Mathematically,

$$(1)\ Pr\left(fragment|\{\beta_{ki}, f_k\}\right) = \sum_{k=1}^{n} f_k \prod_i \beta_{ki}^{m_i}\left(1 - \beta_{ki}\right)^{1-m_i}$$

where $n$ is the number of mixture components, set to 3; $m_i \in \{0, 1\}$ is the fragment's observed methylation at position $i$;

$f_k$ is the fractional assignment to component $k$ (with $f_k \geq 0$ and $\sum f_k = 1$ ); and $\beta_{ki}$ is the methylation fraction in component $k$ at CpG $i$. The product over $i$ included only those positions for which a methylation state could be identified from the sequencing. Maximum-likelihood values of the parameters $\{f_k, \beta_{ki}\}$ of each model were estimated by using the rprop algorithm (e.g., the rprop algorithm as described in Riedmiller M, Braun H. RPROP - A Fast Adaptive Learning Algorithm. Proceedings of the International Symposium on Computer and Information Science VII, 1992) to maximize the total log-likelihood of the fragments of one training label, subject to a regularization penalty on $\beta_{ki}$ that took the form of a beta-distributed prior. Mathematically, the maximized quantity was

$$(2)\ \sum_j\ ln\left(Pr\left(fragment_j|\{\beta_{ki}, f_k\}\right)\right) + \sum_{k,i}\ r\ ln\left(\beta_{ki}(1 - \beta_{ki})\right)$$

where r is the regularization strength, which was set to 1.

*Featurization*

Once the probabilistic models were trained, a set of numerical features was computed for each sample. Specifically, features were extracted for each fragment from each training sample, for each cancer type and non-cancer sample, in each region. The extracted features were the tallies of outlier fragments (i.e., anomalously methylated fragments), which were defined as those whose log-likelihood under a first cancer model exceeded the log-likelihood under a second cancer model or non-cancer model by at least a threshold tier value. Outlier fragments were tallied separately for each genomic region, sample model (i.e., cancer type), and tier (for tiers 1, 2, 3, 4, 5, 6, 7, 8, and 9), yielding 9 features per region for each sample type. In this way, each feature was defined by three properties: a genomic region; a "positive" cancer type label (excluding non-cancer); and the tier value selected from the set {1, 2, 3, 4, 5, 6, 7, 8, 9}. The numerical value of each feature was defined as the number of fragments in that region such that

$$(3)\ ln\left(\frac{Pr(fragment|positive\ cancer\ type)}{Pr(fragment|non-cancer)}\right) > tier$$

where the probabilities were defined by equation (1) using the maximum-likelihood-estimated parameter values corresponding to the "positive" cancer type (in the numerator of the logarithm) or to non-cancer (in the denominator).

*Feature ranking*

For each set of pairwise features, the features were ranked using mutual information based on their ability to distinguish the first cancer type (which defined the log-likelihood model from which the feature was derived) from the second cancer type or non-cancer. Specifically, two ranked lists of features were compiled for each unique pair of class labels: one with the first label assigned as the "positive" and the second as the "negative", and the other with the positive/negative assignment swapped (with the exception of the "non-cancer" label, which was only permitted as the negative label). For each of these ranked lists, only features whose positive cancer type label (as in equation (3)) matched the positive label under consideration were included in the ranking. For each such feature, the fraction of training samples with non-zero feature value was calculated separately for the positive and negative labels. Features for which this fraction was greater in the positive label were ranked by their mutual information with respect to that pair of class labels.
The top ranked 256 features from each pairwise comparison were identified and added to the final feature set for each cancer type and non-cancer. To avoid redundancy, if more than one feature was selected from the same positive type and genomic region (i.e., for multiple negative types), only the one assigned the lowest (most informative) rank for its cancer type pair was retained, breaking ties by choosing the higher tier value. The features in the final feature set for each sample (cancer type and non-cancer) were binarized (any feature value greater than 0 was set to 1, so that all features were either 0 or 1).

*Classifier training*

The training samples were then divided into distinct 5-fold cross-validation training sets, and a two-stage classifier was trained for each fold, in each case training on 4/5 of the training samples and using the remaining 1/5 for validation.
In the first stage of training, a binary (two-class) logistic regression model for detecting the presence of cancer was trained to discriminate the cancer samples (regardless of TOO) from non-cancer. When training this binary classifier, a sample weight was assigned to the male non-cancer samples to counteract sex-imbalance in the training set. For each sample, the binary classifier outputs a prediction score indicating the likelihood of a presence or absence of cancer.
In the second stage of training, a parallel multi-class logistic regression model for determining cancer tissue of origin was trained with TOO as the target label. Only the cancer samples that received a score above the 95th percentile of the non-cancer samples in the first stage classifier were included in the training of this multi-class classifier. For each cancer sample used in training the multi-class classifier, the multi-class classifier outputs prediction values for the cancer types being classified, where each prediction value is a likelihood that the given sample has a certain cancer type. For example, the cancer classifier can return a cancer prediction for a test sample including a prediction score for breast cancer, a prediction score for lung cancer, and/or a prediction score for no cancer.
Both binary and multi-class classifiers were trained by stochastic gradient descent with mini-batches, and in each case, training was stopped early when the performance on the validation fold (assessed by cross-entropy loss) began to degrade. For predicting on samples outside of the training set, in each stage, the scores assigned by the five cross-validated classifiers were averaged. Scores assigned to sex-inappropriate cancer types were set to zero, with the remaining values renormalized to sum to one.
Scores assigned to the validation folds within the training set were retained for use in assigning cutoff values (thresholds) to target certain performance metrics. In particular, the probability scores assigned to the training set non-cancer samples were used to define thresholds corresponding to particular specificity levels. For example, for a desired specificity target of 99.4%, the threshold was set at the 99.4th percentile of the cross-validated cancer detection probability scores assigned to the non-cancer samples in the training set. Training samples with a probability score that exceeded a threshold were called as positive for cancer.
Subsequently, for each training sample determined to be positive for cancer, a TOO or cancer type assessment was made from the multiclass classifier. First, the multi-class logistic regression classifier assigned a set of probability scores, one for each prospective cancer type, to each sample. Next, the confidence of these scores was assessed as the difference between the highest and second-highest scores assigned by the multi-class classifier for each sample. Then, the cross-validated training set scores were used to identify the lowest threshold value such that of the cancer samples in the training set with top-two score differential exceeding the threshold, 90% had been assigned the correct TOO label as their highest score. In this way, the scores assigned to the validation folds during training were further used to determine a second threshold for distinguishing between confident and indeterminate TOO calls.

At prediction time, samples receiving a score from the binary (first-stage) classifier below the predefined specificity threshold were assigned a "non-cancer" label. For the remaining samples, those whose top-two TOO-score differential from the second-stage classifier was below the second predefined threshold were assigned the "indeterminate cancer" label. The remaining samples were assigned the cancer label to which the TOO classifier assigned the highest score.

**EXAMPLE 5 - Classification with the target genomic regions of Lists 16-32**

The discriminatory value of the target genomic regions of Lists 16-32 was evaluated by testing the ability of a cancer classifier to detect cancer and any of 20 different cancer types according to the methylation status of these target genomic regions. Performance was evaluated over a set of 1,532 cancer samples and 1,521 non-cancer samples that were not used to train the classifier, as shown in **TABLE 4.** For each sample, differentially methylated cfDNA was enriched using a bait set comprising all of the target genomic regions of Lists 16-32. The classifier was then constrained to provide cancer determinations based only on the methylation status of the target genomic regions of the List being evaluated.

**TABLE 4**

| Cancer diagnoses of individuals whose cfDNA was used to validate the classifier | | | | | | |
|---|---|---|---|---|---|---|
| Cancer Type | Total | Stage | | | | |
| | | I | II | III | IV | Not Reported |
| Non-cancer | 1521 | - | - | - | - | - |
| Lung | 261 | 60 | 23 | 72 | 106 | 0 |
| Breast | 247 | 102 | 110 | 27 | 8 | 0 |
| Prostate | 188 | 39 | 113 | 19 | 17 | 0 |
| Lymphoid neoplasm | 147 | 15 | 27 | 27 | 39 | 39 |
| Colorectal | 121 | 13 | 22 | 41 | 45 | 0 |
| Pancreas and gallbladder | 95 | 15 | 15 | 19 | 46 | 0 |
| Uterine | 84 | 73 | 3 | 5 | 3 | 0 |
| Upper GI | 67 | 9 | 12 | 19 | 27 | 0 |
| Head and neck | 62 | 7 | 13 | 16 | 26 | 0 |
| Renal | 56 | 37 | 4 | 4 | 11 | 0 |
| Ovary | 37 | 4 | 2 | 25 | 6 | 0 |
| Multiple myeloma | 34 | 10 | 13 | 11 | 0 | 0 |
| Not reported | 29 | 8 | 5 | 7 | 6 | 3 |
| Liver bile duct | 29 | 5 | 7 | 7 | 10 | 0 |
| Sarcoma | 17 | 2 | 4 | 5 | 6 | 0 |
| Bladder and urothelial | 16 | 6 | 7 | 3 | 1 | 0 |
| Anorectal | 14 | 4 | 5 | 5 | 0 | 0 |
| Cervical | 11 | 8 | 1 | 2 | 0 | 0 |
| Melanoma | 7 | 3 | 1 | 0 | 3 | 0 |
| Myeloid neoplasm | 4 | 2 | 1 | 0 | 1 | 0 |
| Thyroid | 4 | 0 | 0 | 0 | 0 | 4 |
| Prediction only | 2 | 0 | 0 | 0 | 2 | 0 |

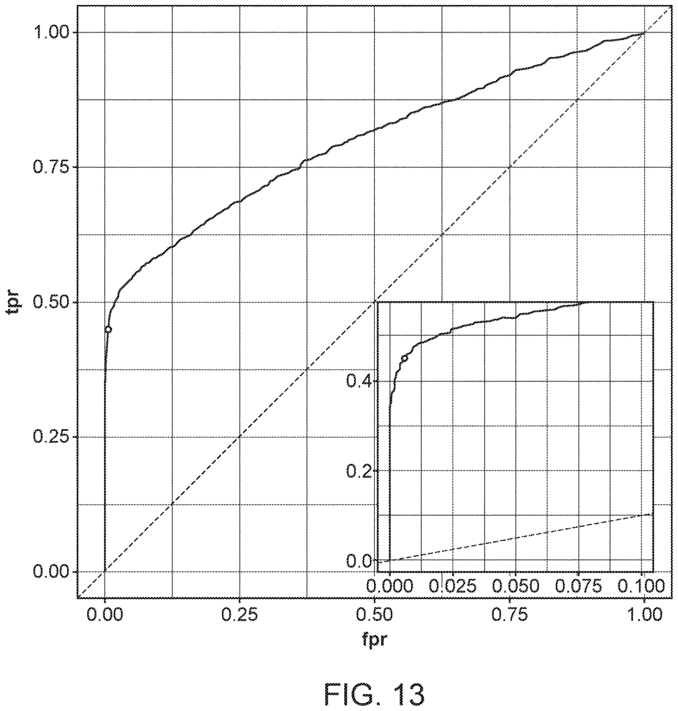

Results from the classifier performance analysis for lists 16-32 are presented in **TABLES 5-8.** An exemplary receiver operator curve (ROC) generated by a trained classifier is shown in **FIGURE 13**. The ROC shows true positive results and false positive results for a determination of cancer or no-cancer based on the methylation status of the target genomic regions of list 23, optimized for lung cancer. The asymmetric shape of the ROC curve illustrates that the classifier was designed to minimize false positive results. Except for list 28 (renal cancer) the areas under the curve are tightly clustered between 0.77 and 0.80, as shown in **TABLE 5.** These results indicate that a determination of cancer is not grossly compromised by using panels optimized for the detection of individual cancer types. Additionally, classifier performance was tested for randomly selected 50% subsets of the target genomic regions of list 20 (colorectal cancer), list 23 (lung cancer) and list 26 (pancreas and gall bladder cancer). The areas under the ROC curve for these subsets of target genomic regions were also tightly clustered between 0.77 and 0.80, indicating that a determination of cancer is not detectably compromised by using smaller panels of less than 400 - 700 target genomic regions having a total panel size of less than 75 - 140 kb.

Once a determination of cancer is made, the classifier assigns the cancer to one of twenty distinct cancer types. The accuracy of these determinations with a specificity of 0.990 is presented in various formats. **TABLE 5** shows true positives, false positives, and false negatives as scored based on the methylation status of each list of target genomic regions optimized for the detection of a specific cancer type. A true positive occurs when the presence of cancer is detected and the cancer type is accurately determined. A false positive occurs for samples from individuals diagnosed with the cancer type that the list was optimized for when the presence of cancer is detected and an inaccurate cancer type is scored. A false negative occurs for samples from individuals diagnosed with a different cancer type than the cancer type that the list was optimized for when the presence of cancer is detected and the cancer type is inaccurately scored as the cancer type for which the list was optimized.

**TABLE 5**

| Cancer detection and cancer type determination using data for lists of target genomic regions optimized for the detection of specific cancer types. | | | | |
|---|---|---|---|---|
| Cancer Type for Target Genomic Regions | AUC | True Positive | False Positive | False Negative |
| Anorectal | 0.78 | 0 | 0 | 6 |
| Bladder & Urothelial | 0.78 | 3 | 0 | 1 |
| Breast | 0.79 | 67 | 5 | 1 |
| Cervical | 0.80 | 0 | 0 | 2 |
| Colorectal | 0.78 | 72 | 2 | 2 |
| Head & Neck | 0.78 | 38 | 16 | 6 |
| Liver & Bile duct | 0.78 | 17 | 2 | 2 |
| Lung | 0.80 | 143 | 11 | 4 |
| Melanoma | 0.79 | 3 | 0 | 0 |
| Ovary | 0.78 | 24 | 1 | 2 |
| Pancreas & Gallbladder | 0.79 | 47 | 2 | 8 |
| Prostate | 0.78 | 14 | 0 | 1 |
| Renal | 0.59 | 0 | 0 | 0 |
| Sarcoma | 0.78 | 6 | 0 | 1 |
| Thyroid | 0.78 | 0 | 1 | 0 |
| Upper GI | 0.77 | 32 | 2 | 1 |
| Uterine | 0.79 | 11 | 0 | 0 |
| Random 50% of Colorectal | 0.77 | 88 | 0 | 7 |
| Random 50% of Lung | 0.79 | 92 | 1 | 8 |
| Random 50% of Pancreas & Gallbladder | 0.78 | 94 | 0 | 9 |

The accuracy of cancer detection by a trained classifier based on the methylation status of lists of target genomic regions selected for specific cancer types is presented for various cancer type lists in **TABLE 6.** When cancer is detected, a cancer type is assigned from one of twenty possible classes of cancer types. The accuracy of cancer type determination is presented in **TABLE 7.** The cancer type determination results are for the accuracy of determining all twenty cancer types, even though the lists of target genomic regions were optimized to detect a single cancer type.

The results in **TABLES 6-7** are segregated for various stages of cancer. Cancer detection and cancer type determination were more accurate for samples from individuals diagnosed with later stages of cancer. This was expected because late stage tumors shed more cfDNA. Nevertheless, the accuracy of detecting cancer and assigning a cancer type for early stage cancers is remarkably high. Furthermore, randomly eliminating 50% of the target genomic regions of list 20 (colorectal cancer), list 23 (lung cancer) and list 26 (pancreas and gall bladder cancer) had essentially no impact on classifier accuracy.

The sensitivity at a specificity of 0.990 for detecting stages I - IV cancers of various cancer types by a classifier acting on the methylation status of target genomic regions in lists selected for the specific cancer type to be detected is presented in **TABLE 8.** For example, when the false positive rate for detecting cancer is limited to 1%, a classifier considering the methylation status of the target genomic regions of list 16 accurately detected anorectal cancer for 50% (2 out of 4) of the samples collected from individuals diagnosed with stage I anorectal cancer. An overall sensitivity for all cancer stages of>70% was achieved for anorectal cancer, head & neck cancer, liver & bile duct cancer, ovarian cancer, pancreatic & gallbladder cancer, and upper gastrointestinal tract cancer. The sensitivity for detecting stage I + II cancers was >50% for anorectal cancer, bladder & urothelial cancer, head & neck cancer, liver & bile duct cancer, and pancreatic & gallbladder cancer. Sensitivity based on the methylation status of a randomly selected 50% of the target genomic regions for colorectal cancer, lung cancer, or pancreas and gall bladder cancer was essentially identical to sensitivity using 100% of the corresponding target genomic regions.

TABLE 6 - Cancer detection accuracy with 99.0% specificity by a classifier using only target genomic regions specific to the indicated cancer type.

| Cancer Stage | Anorectal | | Bladder & Urothelial | | Breast | | Cervical | | Colorectal | | Head & Neck | | Liver & Bile duct | |
|---|---|---|---|---|---|---|---|---|---|---|---|---|---|---|
| | % | Fraction | % | Fraction | % | Fraction | % | Fraction | % | Fraction | % | Fraction | % | Fraction |
| All | 43 | 653/1532 | 45 | 694/1532 | 45 | 684/1532 | 45 | 695/1532 | 42 | 648/1532 | 43 | 665/1532 | 43 | 660/1532 |
| I | 9 | 38/422 | 12 | 49/422 | 11 | 48/422 | 11 | 46/422 | 9 | 36/422 | 10 | 40/422 | 11 | 45/422 |
| II | 31 | 120/388 | 33 | 126/388 | 34 | 130/388 | 33 | 126/388 | 31 | 119/388 | 31 | 121/388 | 31 | 122/388 |
| III | 61 | 192/313 | 65 | 203/313 | 61 | 192/313 | 67 | 210/313 | 59 | 184/313 | 63 | 196/313 | 61 | 191/313 |
| I+II | 20 | 158/810 | 22 | 175/810 | 22 | 178/810 | 21 | 172/810 | 19 | 155/810 | 20 | 161/810 | 21 | 167/810 |
| I+II+III | 31 | 350/1123 | 34 | 378/1123 | 33 | 370/1123 | 34 | 382/1123 | 30 | 339/1123 | 32 | 357/1123 | 32 | 358/1123 |
| III+IV | 71 | 482/676 | 75 | 504/676 | 73 | 492/676 | 75 | 509/676 | 71 | 477/676 | 72 | 489/676 | 72 | 485/676 |
| IV | 80 | 290/363 | 83 | 301/363 | 83 | 300/363 | 82 | 299/363 | 81 | 293/363 | 81 | 293/363 | 81 | 294/363 |

| Cancer Stage | Lung % | Lung Fraction | Melanoma % | Melanoma Fraction | Ovary % | Ovary Fraction | Pancreas & Gallbladder % | Pancreas & Gallbladder Fraction | Prostate % | Prostate Fraction | Renal % | Renal Fraction | Sarcoma % | Sarcoma Fraction |
|---|---|---|---|---|---|---|---|---|---|---|---|---|---|---|
| All | 44 | 675/1532 | 44 | 678/1532 | 44 | 666/1532 | 44 | 678/1532 | 42 | 639/1532 | 44 | 679/1532 | 44 | 666/1532 |
| I | 10 | 43/422 | 11 | 46/422 | 10 | 43/422 | 10 | 44/422 | 8 | 35/422 | 11 | 45/422 | 10 | 40/422 |
| II | 33 | 126/388 | 32 | 125/388 | 30 | 117/388 | 31 | 120/388 | 32 | 125/388 | 33 | 127/388 | 31 | 122/388 |
| III | 63 | 198/313 | 60 | 187/313 | 62 | 195/313 | 63 | 198/313 | 56 | 175/313 | 62 | 193/313 | 61 | 192/313 |
| I+II | 21 | 169/810 | 21 | 171/810 | 20 | 160/810 | 20 | 164/810 | 20 | 160/810 | 21 | 172/810 | 20 | 162/810 |
| I+II+III | 33 | 367/1123 | 32 | 358/1123 | 32 | 355/1123 | 32 | 362/1123 | 30 | 335/1123 | 33 | 365/1123 | 32 | 354/1123 |
| III+IV | 73 | 493/676 | 73 | 491/676 | 73 | 490/676 | 74 | 498/676 | 69 | 469/676 | 72 | 488/676 | 72 | 487/676 |
| IV | 81 | 295/363 | 84 | 304/363 | 81 | 295/363 | 83 | 300/363 | 81 | 294/363 | 81 | 295/363 | 81 | 295/363 |

**TABLE 6 (cont'd)**

| Cancer Stage | Thyroid | | Upper GI | | Uterine | | Random 50% Colorectal | | Random 50% Lung | | Random 50% Pancreas & Gallbladder | |
|---|---|---|---|---|---|---|---|---|---|---|---|---|
| | % | Fraction | % | Fraction | % | Fraction | % | Fraction | % | Fraction | % | Fraction |
| All | 43 | 655/1532 | 43 | 654/1532 | 44 | 668/1532 | 40 | 618/1532 | 44 | 679/1532 | 44 | 669/1532 |
| I | 10 | 43/422 | 10 | 41/422 | 11 | 47/422 | 7 | 31/422 | 10 | 42/422 | 10 | 42/422 |
| II | 30 | 115/388 | 31 | 121/388 | 31 | 121/388 | 27 | 106/388 | 32 | 125/388 | 30 | 117/388 |
| III | 60 | 187/313 | 59 | 186/313 | 62 | 195/313 | 58 | 180/313 | 64 | 201/313 | 63 | 196/313 |
| I+II | 20 | 158/810 | 20 | 162/810 | 21 | 168/810 | 17 | 137/810 | 21 | 167/810 | 20 | 159/810 |
| I+II+III | 31 | 345/1123 | 31 | 348/1123 | 32 | 363/1123 | 28 | 317/1123 | 33 | 368/1123 | 32 | 355/1123 |
| III+IV | 71 | 478/676 | 71 | 478/676 | 72 | 489/676 | 70 | 471/676 | 74 | 500/676 | 73 | 495/676 |
| IV | 80 | 291/363 | 80 | 292/363 | 81 | 294/363 | 80 | 291/363 | 82 | 299/363 | 82 | 299/363 |

TABLE 7 - Accuracy of cancer type determinations with 99.0% specificity by a classifier using only target genomic regions specific to the indicated cancer type.

| Cancer Stage | Anorectal | | Bladder & Urothelial | | Breast | | Cervical | | Colorectal | | Head & Neck | | Liver & Bile duct | |
|---|---|---|---|---|---|---|---|---|---|---|---|---|---|---|
| | % | Fraction | % | Fraction | % | Fraction | % | Fraction | % | Fraction | % | Fraction | % | Fraction |
| All | 89 | 493/552 | 90 | 549/612 | 90 | 534/595 | 89 | 516/577 | 89 | 461/516 | 90 | 514/574 | 89 | 485/546 |
| I | 73 | 19/26 | 74 | 26/35 | 69 | 22/32 | 81 | 21/26 | 76 | 16/21 | 77 | 20/26 | 74 | 23/31 |
| II | 89 | 86/97 | 91 | 100/110 | 89 | 101/113 | 87 | 94/108 | 88 | 85/97 | 89 | 93/104 | 92 | 91/99 |
| III | 89 | 143/161 | 91 | 159/175 | 92 | 153/167 | 91 | 149/163 | 92 | 135/147 | 89 | 150/168 | 88 | 139/158 |
| I+II | 85 | 105/123 | 87 | 126/145 | 85 | 123/145 | 86 | 115/134 | 86 | 101/118 | 87 | 113/130 | 88 | 114/130 |
| I+II+III | 87 | 248/284 | 89 | 285/320 | 89 | 276/312 | 89 | 264/297 | 89 | 236/265 | 88 | 263/298 | 88 | 253/288 |
| III+IV | 90 | 379/420 | 91 | 411/454 | 91 | 398/436 | 90 | 389/431 | 91 | 352/389 | 90 | 389/431 | 89 | 366/410 |
| IV | 91 | 236/259 | 90 | 252/279 | 91 | 245/269 | 90 | 240/268 | 90 | 217/242 | 91 | 239/263 | 90 | 227/252 |

### TABLE 7 (cont'd)

| Cancer Stage | Lung | | Melanoma | | Ovary | | Pancreas & Gallbladder | | Prostate | | Renal | | Sarcoma | |
|---|---|---|---|---|---|---|---|---|---|---|---|---|---|---|
| | % | Fraction | % | Fraction | % | Fraction | % | Fraction | % | Fraction | % | Fraction | % | Fraction |
| All | 89 | 536/600 | 89 | 504/565 | 89 | 457/511 | 90 | 530/589 | 90 | 502/560 | 90 | 517/576 | 90 | 490/546 |
| I | 80 | 24/30 | 79 | 22/28 | 76 | 22/29 | 70 | 19/27 | 68 | 19/28 | 68 | 21/31 | 72 | 18/25 |
| II | 90 | 102/114 | 87 | 89/102 | 89 | 81/91 | 88 | 98/112 | 88 | 90/102 | 91 | 93/102 | 87 | 85/98 |
| III | 90 | 154/172 | 91 | 140/154 | 92 | 133/145 | 92 | 158/171 | 88 | 137/155 | 89 | 144/161 | 90 | 138/154 |
| I+II | 88 | 126/144 | 85 | 111/130 | 86 | 103/120 | 84 | 117/139 | 84 | 109/130 | 86 | 114/133 | 84 | 103/123 |
| I+II+III | 89 | 280/316 | 88 | 251/284 | 89 | 236/265 | 89 | 275/310 | 86 | 246/285 | 88 | 258/294 | 87 | 241/277 |
| III+IV | 90 | 401/446 | 90 | 380/422 | 90 | 345/382 | 92 | 399/436 | 91 | 386/423 | 91 | 387/426 | 91 | 373/409 |
| IV | 90 | 247/274 | 90 | 240/268 | 90 | 212/237 | 91 | 241/265 | 93 | 249/268 | 92 | 243/265 | 92 | 235/255 |

EP 3 921 444 B1

57

| Cancer Stage | Thyroid | | Upper GI | | Uterine | | Random 50% Colorectal | | Random 50% Lung | | Random 50% Pancreas & Gallbladder | |
|---|---|---|---|---|---|---|---|---|---|---|---|---|
| | % | Fraction | % | Fraction | % | Fraction | % | Fraction | % | Fraction | % | Fraction |
| All | 89 | 440/495 | 89 | 463/520 | 90 | 546/608 | 90 | 446/497 | 89 | 533/596 | 90 | 518/579 |
| I | 78 | 18/23 | 71 | 17/24 | 75 | 27/36 | 79 | 15/19 | 77 | 23/30 | 65 | 17/26 |
| II | 93 | 74/80 | 87 | 83/95 | 89 | 99/111 | 89 | 78/88 | 89 | 101/114 | 87 | 91/105 |
| III | 90 | 125/139 | 91 | 131/144 | 90 | 161/178 | 88 | 130/147 | 91 | 157/172 | 93 | 157/169 |
| I+II | 89 | 92/103 | 84 | 100/119 | 86 | 126/147 | 87 | 93/107 | 86 | 124/144 | 82 | 108/131 |
| I+II+III | 90 | 217/242 | 88 | 231/263 | 88 | 287/325 | 88 | 223/254 | 89 | 281/316 | 88 | 265/300 |
| III+IV | 89 | 334/377 | 91 | 352/389 | 91 | 413/453 | 90 | 345/382 | 91 | 401/442 | 92 | 398/434 |
| IV | 88 | 209/238 | 90 | 221/245 | 92 | 252/275 | 92 | 215/235 | 90 | 244/270 | 91 | 241/265 |

TABLE 8 - Sensitivity with 99.0% specificity for the indicated Cancer Type by a classifier using only target genomic regions specific to the indicated cancer type.

| Cancer Stage | Anorectal | | Bladder & Urothelial | | Breast | | Cervical | | Colorectal | | Head & Neck | | Liver & Bile duct | |
|---|---|---|---|---|---|---|---|---|---|---|---|---|---|---|
| | % | Fraction | % | Fraction | % | Fraction | % | Fraction | % | Fraction | % | Fraction | % | Fraction |
| All | 79 | 11/14 | 50 | 8/16 | 29 | 71/247 | 36 | 4/11 | 69 | 83/121 | 84 | 52/62 | 86 | 25/29 |
| I | 50 | 2/4 | 50 | 3/6 | 2 | 2/102 | 13 | 1/8 | 23 | 3/13 | 86 | 6/7 | 60 | 3/5 |
| II | 80 | 4/5 | 57 | 4/7 | 36 | 39/110 | 100 | 1/1 | 41 | 9/22 | 77 | 10/13 | 86 | 6/7 |
| III | 100 | 5/5 | 50 | 1/2 | 82 | 22/27 | 100 | 2/2 | 71 | 29/41 | 81 | 13/16 | 86 | 6/7 |
| I+II | 67 | 6/9 | 54 | 7/13 | 19 | 41/212 | 22 | 2/9 | 34 | 12/35 | 80 | 16/20 | 75 | 9/12 |
| I+II+III | 79 | 11/14 | 53 | 8/15 | 26 | 63/239 | 36 | 4/11 | 54 | 41/76 | 81 | 29/36 | 79 | 15/19 |
| III+IV | 100 | 5/5 | 33 | 1/3 | 86 | 30/35 | 100 | 2/2 | 83 | 71/86 | 86 | 36/42 | 94 | 16/17 |
| IV | n.a. | 0 | 0 | 0/1 | 100 | 8/8 | | | 93 | 42/45 | 89 | 23/26 | 100 | 10/10 |

| Cancer Stage | Lung | | Melanoma | | Ovary | | Pancreas & Gallbladder | | Prostate | | Renal | | Sarcoma | |
|---|---|---|---|---|---|---|---|---|---|---|---|---|---|---|
| | % | Fraction | % | Fraction | % | Fraction | % | Fraction | % | Fraction | % | Fraction | % | Fraction |
| All | 64 | 166/261 | 43 | 3/7 | 84 | 31/37 | 74 | 70/95 | 11.7 | 22/188 | 21.4 | 12/56 | 47 | 8/17 |
| I | 13 | 8/60 | 0 | 0/3 | 25 | 1/4 | 40 | 6/15 | 2.6 | 1/39 | 2.7 | 1/37 | 50 | 1/2 |
| II | 61 | 14/23 | 0 | 0/1 | 0 | 0/2 | 67 | 10/15 | 5.3 | 6/113 | 0.0 | 0/4 | 0 | 0/4 |
| III | 75 | 54/72 | 0 | 0/4 | 96 | 24/25 | 68 | 13/19 | 10.5 | 2/19 | 50.0 | 2/4 | 60 | 3/5 |
| I+II | 27 | 22/83 | 0 | 0/4 | 17 | 1/6 | 53 | 16/30 | 4.6 | 7/152 | 2.4 | 1/41 | 17 | 1/6 |
| I+II+III | 49 | 76/155 | 100 | 3/3 | 81 | 25/31 | 59 | 29/49 | 5.3 | 9/171 | 6.7 | 3/45 | 36 | 4/11 |
| III+IV | 81 | 144/178 | 100 | 3/3 | 97 | 30/31 | 83 | 54/65 | 41.7 | 15/36 | 73.3 | 11/15 | 64 | 7/11 |
| IV | 85 | 90/106 | n.a. | 0 | 100 | 6/6 | 89 | 41/46 | 76.5 | 13/17 | 81.8 | 9/11 | 67 | 4/6 |

**TABLE 8 (cont'd)**

| Cancer Stage | Thyroid | | Upper GI | | Uterine | | Random 50% Colorectal | | Random 50% Lung | | Random 50% Pancreas & Gallbladder | |
|---|---|---|---|---|---|---|---|---|---|---|---|---|
| | % | Fraction | % | Fraction | % | Fraction | % | Fraction | % | Fraction | % | Fraction |
| All | 25 | 1/4 | 70 | 47/67 | 21 | 18/84 | 66 | 80/121 | 64 | 166/261 | 74 | 70/95 |
| I | 0 | 0/2 | 11 | 1/9 | 16 | 12/73 | 15 | 2/13 | 12 | 7/60 | 40 | 6/15 |
| II | 0 | 0/1 | 75 | 9/12 | 33 | 1/3 | 36 | 8/22 | 61 | 14/23 | 67 | 10/15 |
| III | n.a. | 0 | 63 | 12/19 | 60 | 3/5 | 68 | 28/41 | 76 | 55/72 | 63 | 12/19 |
| I+II | 0 | 0/3 | 48 | 10/21 | 17 | 13/76 | 29 | 10/1 | 25 | 21/83 | 53 | 16/30 |
| I+II+III | 0 | 0/3 | 55 | 22/40 | 20 | 16/81 | 50 | 38/76 | 49 | 76/155 | 57 | 28/49 |
| III+IV | 100 | 1/1 | 80 | 37/46 | 63 | 5/8 | 81 | 70/86 | 82 | 145/178 | 83 | 54/65 |
| IV | 100 | 1/1 | 93 | 25/27 | 67 | 2/3 | 93 | 42/45 | 85 | 90/106 | 91 | 42/46 |

**EXAMPLE 6 - Detection of cancer using cancer assay panel**

Blood samples are collected from a group of individuals previously diagnosed with cancer of a TOO ("test group"), and other groups of individuals without cancer or diagnosed with a different type of cancer ("other group"). cfDNA fragments are extracted from the blood samples and treated with bisulfite to convert unmethylated cytosines to uracils. The cancer assay panel described herein is applied to the bisulfite treated samples. Unbound cfDNA fragments are washed and cfDNA fragments bound to the probes are collected. The collected cfDNA fragments are amplified and sequenced. The sequence reads confirm that the probes specifically enrich cfDNA fragments having methylation patterns indicative of cancer of a TOO and samples from the test group include significantly more of the differentially methylated cfDNA fragments compared to the other group.

**Claims**

1. A method of detecting cells of a cancer type in a subject, the method comprising:

   (i) capturing cell-free DNA (cfDNA) fragments from the subject or amplification products thereof with a composition comprising a plurality of different bait oligonucleotides, wherein:

      (a) each bait oligonucleotide in the plurality of different bait oligonucleotides has a length of at least 45 nucleotides, optionally 45 to 300 nucleotides;
      (b) the bait oligonucleotides collectively hybridize to at least 100 target genomic regions that are differentially methylated in a first cancer type of at least 10 cancer types, relative to a different cancer type of the at least 10 cancer types or relative to non-cancer;

   (ii) sequencing the captured cfDNA fragments or amplification products thereof to produce sequencing reads, wherein the cfDNA is converted prior to sequencing by treating the cfDNA to convert unmethylated cytosines to uracils, and
   (iii) for each of the at least 10 cancer types, applying a trained classifier to the sequencing reads, wherein the classifier (a) is constrained to the at least 100 target genomic regions of the bait oligonucleotides for the first cancer type, and (b) assigns a score for each of the at least 10 cancer types; wherein the trained classifier is a classifier trained using converted DNA sequences, wherein converted DNA relates to DNA that has been treated to convert unmethylated cytosines to uracils, and
   (iv) detecting the cells of the cancer type in the subject as the cancer type assigned the highest score;

   wherein the at least 10 cancer types are selected from anorectal cancer, bladder cancer, urothelial cancer, breast cancer, cervical cancer, colorectal cancer, head & neck cancer, liver cancer, bile duct cancer, lung cancer, ovarian cancer, pancreatic cancer, gallbladder cancer, prostate cancer, renal cancer, sarcoma, thyroid cancer, upper GI cancer, and uterine cancer.

2. The method of claim 1, wherein the likelihood of a false positive detection of the cells of the cancer type in the subject is less than 1% and the likelihood of an accurate determination of the cells of the cancer type in the subject is at least 40%.

3. The method of claim 1 or 2, wherein:

   (a) the first cancer type is a stage I cancer type, and the likelihood of an accurate assignment of cancer type is at least 70%;
   (b) the first cancer type is a stage II cancer type, and the likelihood of an accurate assignment of cancer type is at least 85%;
   (c) the first cancer type is a stage I or stage II cancer, and accuracy of assigning the cancer type is at least 75%;
   (d) accuracy of assigning cancer type is at least 80%; or
   (e) the at least 10 cancer types further include esophageal cancer, squamous cell cancer of the upper gastrointestinal tract, lung adenocarcinoma, small cell lung cancer, squamous cell lung cancer, neuroendocrine cancer, melanoma, plasma cell neoplasm, multiple myeloma, myeloid neoplasm, lymphoma, and leukemia.

4. The method of claim 3, wherein:

(a) the first cancer type is anorectal cancer, the target genomic regions are selected from Lists 16 or 33, and the sensitivity for anorectal cancer is at least 65% or 75%;

(b) the first cancer type is bladder & urothelial cancer, the target genomic regions are selected from Lists 1, 17 or 34, and the sensitivity for bladder & urothelial cancer is at least 40%;

(c) the first cancer type is breast cancer, the target genomic regions are selected from Lists 2, 18 or 35, and the sensitivity for breast cancer is at least 20%;

(d) the first cancer type is cervical cancer, the target genomic regions are selected from Lists 3, 19 or 36, and the sensitivity for cervical cancer is at least 25%;

(e) the first cancer type is colorectal cancer, the target genomic regions are selected from Lists 4, 20 or 37, and the sensitivity for colorectal cancer is at least 55%;

(f) the first cancer type is head & neck cancer, the target genomic regions are selected from Lists 5, 21 or 38, and the sensitivity for head & neck cancer is at least 70%;

(g) the first cancer type is liver & bile duct cancer, the target genomic regions are selected from Lists 6, 22, or 39, and the sensitivity for liver & bile duct cancer is at least 75%;

(h) the first cancer type is lung cancer, the target genomic regions are selected from Lists 7, 23 or 40, and the sensitivity for lung cancer is at least 55%;

(i) the first cancer type is melanoma, the target genomic regions are selected from Lists 8, 24 or 41, and the sensitivity for melanoma is at least 30%;

(j) the first cancer type is ovarian cancer, the target genomic regions are selected from Lists 9, 25 or 42, and the sensitivity for ovarian cancer is at least 70%;

(k) the first cancer type is pancreas & gallbladder cancer, the target genomic regions are selected from Lists 10, 26 or 43, and the sensitivity for pancreas & gallbladder cancer is at least 60%;

(l) the first cancer type is sarcoma, the target genomic regions are selected from Lists 29 or 46, and the sensitivity for sarcoma is at least 40%;

(m) the first cancer type is upper gastrointestinal tract cancer, the target genomic regions are selected from Lists 14, 31 or 48, and the sensitivity for upper gastrointestinal tract cancer is at least 60%.

5. The method of claim 1, wherein the bait oligonucleotides collectively hybridize to at least 300 target genomic regions from Lists 1-49, or complements thereof, that are differentially methylated in the first cancer type relative to a different cancer type of the at least 10 cancer types or relative to non-cancer.

6. The method of any one of claims 1-5, wherein:

(i) the total size of the at least 100 target genomic regions is 4 MB to 50 kb;

(ii) the subject has an elevated risk of one or more cancer types;

(iii) the subject manifests symptoms associated with one or more cancer types;

(iv) the subject has not been diagnosed with a cancer; or

(v) the classifier was trained on converted DNA sequences derived from a least 100 subjects with the first cancer type, at least 100 subjects with a second cancer type, and at least 100 subjects with no cancer, and wherein the first cancer type, second cancer type, and third cancer type are selected from the at least 10 cancer types.

7. The method of any one of claims 1-6, wherein the classifier was trained on converted DNA sequences derived from the target genomic regions, and the plurality of different bait oligonucleotides collectively hybridize to at least 100 target genomic regions from each of a plurality of Lists 33-49 or complements thereof; optionally wherein the trained classifier detects the cells of the cancer type in the subject by:

(i) generating a set of features for the sample, wherein each feature in the set of features comprises a numerical value;

(ii) inputting the set of features into the classifier, wherein the classifier comprises a multinomial classifier;

(iii) based on the set of features, determining, at the classifier, a set of probability scores, wherein the set of probability scores comprises one probability score per cancer type class and per non-cancer; and

(iv) thresholding the set of probability scores based on one or more values determined during training of the classifier; and optionally wherein:

(a) the set of features comprises a set of binarized features;

(b) the numerical value comprises a single binary value;

(c) the multinomial classifier comprises a multinomial logistic regression ensemble trained to predict a source tissue for the cancer; and/or

(d) the method further comprising determining the final cancer classification based on a top-two probability score differential relative to a minimum value, wherein the minimum value corresponds to a predefined percentage of training cancer samples that had been assigned the correct cancer type as their highest score during training of the classifier.

8. The method of claim 1, further comprising selecting an anti-cancer therapeutic agent for administration to the subject based on detecting the cells of the cancer type in the subject; optionally wherein the anti-cancer agent is a chemotherapeutic agent selected from the group consisting of alkylating agents, antimetabolites, anthracyclines, antitumor antibiotics, cytoskeletal disruptors (taxans), topoisomerase inhibitors, mitotic inhibitors, corticosteroids, kinase inhibitors, nucleotide analogs, and platinum-based agents.

9. The method of any one of claims 1-8, wherein the total size of the at least 100 target genomic regions is 50 kb to (i) less than 270 kb, (ii) less than 200 kb, (iii) less than 150 kb, or (iv) less than 100 kb.

10. The method of any one of claims 1-9, wherein the at least 100 target genomic regions comprise, for all possible pairs between the first cancer type and the at least 10 other cancer types, at least one target genomic region that is differentially methylated between the pair of cancer types.

11. The method of any one of claims 1-9, wherein the at least 100 target genomic regions comprise:

(a) at least 20% of the target genomic regions of any one of Lists 1-49, or complements thereof;
(b) at least 20% of the target genomic regions of any one of Lists 1-15, or complements thereof;
(c) at least 20% of the target genomic regions of Lists 1-15, or complements thereof;
(d) at least 20% the target genomic regions of any one of Lists 16-32, or complements thereof;
(e) at least 20% of the target genomic regions of Lists 16-32, or complements thereof;
(f) at least 20% of the target genomic regions of any one of Lists 33-49, or complements thereof;
(g) at least 20% of the target genomic regions of Lists 33-49, or complements thereof.

12. The method of any one of claims 1-9, wherein:

(a) the total size of the target genomic regions is less than 1100 kb;
(b) the total number of target genomic regions is less than 10,000;
(c) the bait oligonucleotides each have a length of 45 to 300 nucleotide bases, 75-200 nucleotide bases, 103-150 nucleotide bases, or about 120 nucleotide bases; or
(d) the bait oligonucleotides comprise a plurality of sets of two or more bait oligonucleotides, and further wherein:

(i) each bait oligonucleotide within a set of bait oligonucleotides is configured to bind to the same converted target genomic region or configured to bind to a nucleic acid molecule derived from the target genomic region,
(ii) each set of bait oligonucleotides comprises pairs of bait oligonucleotides, each pair of bait oligonucleotides comprises a first bait oligonucleotide and a second bait oligonucleotide,
(iii) each bait oligonucleotide comprises a 5' end and a 3' end,
(iv) for each pair of bait oligonucleotides a sequence of at least X nucleotide bases at the 3' end of the first bait oligonucleotide is identical to a sequence of X nucleotide bases at the 5' end the second bait oligonucleotide, and
(v) X is at least 25, 30, 35, 40, 45, 50, 60, 70, 75 or 100, and optionally wherein the first bait oligonucleotide comprises a sequence of at least 31, 40, 50 or 60 nucleotide bases that does not overlap a sequence of the second bait oligonucleotide.

13. The method of any one of claims 1-9, wherein:

(a) the at least 100 target genomic regions are human sequences, and each of the bait oligonucleotides is designed to have sequence homology or sequence complementarity with less than 20 off-target human genomic regions;
(b) each of the bait oligonucleotides comprises at least 61 nucleotides;
(c) each of the bait oligonucleotides comprises less than 300 nucleotides;
(d) at least 3% of the bait oligonucleotides comprise no G (Guanine);
(e) each of the bait oligonucleotides comprises multiple binding sites to methylation sites of converted cfDNA molecules, wherein at least 83% of the multiple binding sites comprise exclusively either CpG or CpA; or

(f) each target genomic region comprises at least five methylation sites

14. The method of any one of claims 1-13, wherein:

(i) the bait oligonucleotides comprise an additional set of bait oligonucleotides that collectively hybridize to at least 100 target genomic regions that are differentially methylated in a second cancer type of the at least 10 cancer types, relative to a different cancer type of the at least 10 cancer types or relative to non-cancer;
(ii) the second cancer type is different from the first cancer type; and
(iii) for each of the at least 10 cancer types, applying a second trained classifier to the sequencing reads, wherein the second classifier (a) is constrained to the at least 100 target genomic regions of the additional set of bait oligonucleotides for the second cancer type, and (b) assigns a score for each of the at least 10 cancer types.


**Patentansprüche**

1. Verfahren zum Erkennen von Zellen einer Krebsart in einem Subjekt, das Verfahren umfassend:

(i) Erfassen von zellfreien DNA-Fragmenten (cfDNA-Fragmenten) von dem Subjekt oder von Amplifikationsprodukten davon mit einer Zusammensetzung, umfassend eine Vielzahl von unterschiedlichen Köder-Oligonukleotiden, wobei:

(a) jedes Köder-Oligonukleotid in der Vielzahl von unterschiedlichen Köder-Oligonukleotiden eine Länge von mindestens 45 Nukleotiden, optional 45 bis 300 Nukleotiden, aufweist;
(b) die Köder-Oligonukleotide mit mindestens 100 genomischen Zielbereichen gemeinsam hybridisieren, die bei einer ersten Krebsart von mindestens 10 Krebsarten relativ zu einer unterschiedlichen Krebsart der mindestens 10 Krebsarten oder relativ zu Nicht-Krebs unterschiedlich methyliert sind;

(ii) Sequenzieren der erfassten cfDNA-Fragmente oder Amplifikationsprodukte davon, um Sequenzierungs-Reads zu produzieren, wobei die cfDNA vor dem Sequenzieren durch Behandeln der cfDNA umgewandelt wird, um unmethylierte Cytosine in Uracile umzuwandeln, und
(iii) für jede der mindestens 10 Krebsarten, Anwenden eines trainierten Klassifikators auf die Sequenzierungs-Reads, wobei der Klassifikator (a) auf die mindestens 100 genomischen Zielbereiche der Köder-Oligonukleotide für die erste Krebsart beschränkt ist, und (b) eine Bewertungsziffer für jede der mindestens 10 Krebsarten zuordnet; wobei der trainierte Klassifikator ein Klassifikator ist, der unter Verwendung von umgewandelten DNA-Sequenzen trainiert wird, wobei die umgewandelte DNA sich auf die DNA bezieht, die behandelt wurde, um unmethylierte Cytosine in Uracile umzuwandeln, und
(iv) Erkennen der Zellen der Krebsart in dem Subjekt als die Krebsart, der die höchste Bewertungsziffer zugeordnet wird;

wobei die mindestens 10 Krebsarten aus Anorektalkrebs, Blasenkrebs, Urothelkrebs, Brustkrebs, Gebärmutterhalskrebs, Dickdarmkrebs, Kopf- und Halskrebs, Leberkrebs, Gallengangskrebs, Lungenkrebs, Eierstockkrebs, Bauchspeicheldrüsenkrebs, Gallenblasenkrebs, Prostatakrebs, Nierenkrebs, Sarkom, Schilddrüsenkrebs, Krebs des oberen GI und Gebärmutterkrebs ausgewählt sind.

2. Verfahren nach Anspruch 1, wobei die Wahrscheinlichkeit einer falsch positiven Erkennung der Zellen der Krebsart in dem Subjekt weniger als 1 % beträgt und die Wahrscheinlichkeit einer genauen Bestimmung der Zellen der Krebsart in dem Subjekt mindestens 40 % beträgt.

3. Verfahren nach Anspruch 1 oder 2, wobei:

(a) die erste Krebsart eine Krebsart in einem Stadium I ist, und die Wahrscheinlichkeit einer genauen Zuordnung der Krebsart mindestens 70 % beträgt;
(b) die erste Krebsart eine Krebsart in einem Stadium II ist, und die Wahrscheinlichkeit einer genauen Zuordnung der Krebsart mindestens 85 % beträgt;
(c) die erste Krebsart ein Krebs in einem Stadium I oder Stadium II ist, und die Genauigkeit des Zuordnens der Krebsart mindestens 75 % beträgt;
(d) die Genauigkeit des Zuordnens der Krebsart mindestens 80 % beträgt; oder
(e) die mindestens 10 Krebsarten ferner Speiseröhrenkrebs, Plattenepithelkrebs des oberen Gastrointestinaltrakts, Lungenadenokarzinom, kleinzelliger Lungenkrebs, Plattenepithel-Lungenkrebs, neuroendokriner Krebs, Melanom, Plasmazellneoplasie, Multiples Myelom, myeloische Neoplasie, Lymphom und Leukämie einschließen.

4. Verfahren nach Anspruch 3, wobei:

(a) die erste Krebsart Anorektalkrebs ist, die genomischen Zielbereiche aus den Listen 16 oder 33 ausgewählt sind, und die Empfindlichkeit für Anorektalkrebs mindestens 65 % oder 75 % beträgt;

(b) die erste Krebsart Blasen- und Urothelkrebs ist, die genomischen Zielbereiche aus den Listen 1, 17 oder 34 ausgewählt sind, und die Empfindlichkeit für Blasen- und Urothelkrebs mindestens 40 % beträgt;

(c) die erste Krebsart Brustkrebs ist, die genomischen Zielbereiche aus den Listen 2, 18 oder 35 ausgewählt sind, und die Empfindlichkeit für Brustkrebs mindestens 20 % beträgt;

(d) die erste Krebsart Gebärmutterhalskrebs ist, die genomischen Zielbereiche aus den Listen 3, 19 oder 36 ausgewählt sind, und die Empfindlichkeit für Gebärmutterhalskrebs mindestens 25 % beträgt;

(e) die erste Krebsart Dickdarmkrebs ist, die genomischen Zielbereiche aus den Listen 4, 20 oder 37 ausgewählt sind, und die Empfindlichkeit für Dickdarmkrebs mindestens 55 % beträgt;

(f) die erste Krebsart Kopf- und Halskrebs ist, die genomischen Zielbereiche aus den Listen 5, 21 oder 38 ausgewählt sind, und die Empfindlichkeit für Kopf-und Halskrebs mindestens 70 % beträgt;

(g) die erste Krebsart Leber- und Gallengangskrebs ist, die genomischen Zielbereiche aus den Listen 6, 22 oder 39 ausgewählt sind, und die Empfindlichkeit für Leber- und Gallengangskrebs mindestens 75 % beträgt;

(h) die erste Krebsart Lungenkrebs ist, die genomischen Zielbereiche aus den Listen 7, 23 oder 40 ausgewählt sind, und die Empfindlichkeit für Lungenkrebs mindestens 55 % beträgt;

(i) die erste Krebsart ein Melanom ist, die genomischen Zielbereiche aus den Listen 8, 24 oder 41 ausgewählt sind, und die Empfindlichkeit für Melanom mindestens 30 % beträgt;

(j) die erste Krebsart Eierstockkrebs ist, die genomischen Zielbereiche aus den Listen 9, 25 oder 42 ausgewählt sind, und die Empfindlichkeit für Eierstockkrebs mindestens 70 % beträgt;

(k) die erste Krebsart Bauchspeicheldrüsen- und Gallenblasenkrebs ist, die genomischen Zielbereiche aus den Listen 10, 26 oder 43 ausgewählt sind, und die Empfindlichkeit für Bauchspeicheldrüsen- und Gallenblasenkrebs mindestens 60 % beträgt;

(l) die erste Krebsart ein Sarkom ist, die genomischen Zielbereiche aus den Listen 29 oder 46 ausgewählt sind, und die Empfindlichkeit für das Sarkom mindestens 40 % beträgt;

(m) die erste Krebsart Krebs des oberen Gastrointestinaltrakts ist, die genomischen Zielbereiche aus den Listen 14, 31 oder 48 ausgewählt sind, und die Empfindlichkeit für Krebs des oberen Gastrointestinaltrakts mindestens 60 % beträgt.

5. Verfahren nach Anspruch 1, wobei die Köder-Oligonukleotide mit mindestens 300 genomischen Zielbereichen aus den Listen 1-49 oder Komplementen davon gemeinsam hybridisieren, die in der ersten Krebsart relativ zu einer unterschiedlichen Krebsart der mindestens 10 Krebsarten oder relativ zu Nicht-Krebs unterschiedlich methyliert sind.

6. Verfahren nach einem der Ansprüche 1 bis 5, wobei:

(i) die Gesamtgröße der mindestens 100 genomischen Zielbereiche 4 MB bis 50 kb beträgt;

(ii) das Subjekt ein erhöhtes Risiko für eine oder mehrere Krebsarten aufweist;

(iii) das Subjekt Symptome zeigt, die mit einer oder mehreren Krebsarten verknüpft sind;

(iv) das Subjekt nicht mit einem Krebs diagnostiziert wurde; oder

(v) der Klassifikator auf umgewandelte DNA-Sequenzen trainiert wurde, die von mindestens 100 Subjekten mit der ersten Krebsart, mindestens 100 Subjekten mit einer zweiten Krebsart und mindestens 100 Subjekten ohne Krebs abgeleitet sind, und wobei die erste Krebsart, die zweite Krebsart und die dritte Krebsart aus den mindestens 10 Krebsarten ausgewählt sind.

7. Verfahren nach einem der Ansprüche 1 bis 6, wobei der Klassifikator auf umgewandelte DNA-Sequenzen trainiert wurde, die von den genomischen Zielbereichen abgeleitet sind, und die Vielzahl von unterschiedlichen Köder-Oligonukleotiden mit mindestens 100 genomischen Zielbereichen aus jeder einer Vielzahl von Listen 33-49 oder Komplementen davon gemeinsam hybridisiert; optional wobei der trainierte Klassifikator die Zellen der Krebsart in dem Subjekt erkennt durch:

(i) Erzeugen eines Satzes von Merkmalen für die Probe, wobei jedes Merkmal in dem Satz von Merkmalen einen numerischen Wert umfasst;

(ii) Eingeben des Satzes von Merkmalen in den Klassifikator, wobei der Klassifikator einen multinomialen Klassifikator umfasst;

(iii) basierend auf dem Satz von Merkmalen, Bestimmen eines Satzes von Wahrscheinlichkeitsbewertungsziffern bei dem Klassifikator, wobei der Satz von Wahrscheinlichkeitsbewertungsziffern eine Wahrscheinlichkeitsbewertungsziffer pro Krebsartenklasse und pro Nicht-Krebs umfasst; und

(iv) Schwellenwertbildung des Satzes von Wahrscheinlichkeitsbewertungsziffern basierend auf einem oder mehreren Werten, die während des Trainings des Klassifikators bestimmt werden; und optional wobei:

(a) der Satz von Merkmalen einen Satz von binarisierten Merkmalen umfasst;

(b) der numerische Wert einen einzelnen Binärwert umfasst;

(c) der multinomiale Klassifikator ein multinomiales logistisches Regressionsensemble umfasst, das trainiert ist, um ein Ursprungsgewebe für den Krebs vorherzusagen; und/oder

(d) das Verfahren ferner umfassend das Bestimmen der endgültigen Krebsklassifizierung basierend auf einem Unterschied der Top-Zwei-Wahrscheinlichkeitsbewertungsziffern relativ zu einem Mindestwert, wobei der Mindestwert einem vordefinierten Prozentsatz von Trainingskrebsproben entspricht, die der richtigen Krebsart als deren höchste Bewertungsziffer während des Trainings des Klassifikators zugeordnet wurde.

8. Verfahren nach Anspruch 1, ferner umfassend das Auswählen eines therapeutischen Antikrebsmittels für eine Verabreichung an das Subjekt basierend auf dem Erkennen der Zellen der Krebsart in dem Subjekt; optional wobei das Antikrebsmittel ein chemotherapeutisches Mittel ist, das aus der Gruppe ausgewählt ist, bestehend aus Alkylierungsmitteln, Antimetaboliten, Anthracyclinen, Antitumor-Antibiotika, Zytoskelett-Disruptoren (Taxanen), Topoisomerase-Inhibitoren, Mitose-Inhibitoren, Corticosteroiden, Kinase-Inhibitoren, Nukleotidanalog und Mitteln auf Platinumbasis.

9. Verfahren nach einem der Ansprüche 1 bis 8, wobei die Gesamtgröße der mindestens 100 genomischen Zielbereiche 50 kb bis (i) weniger als 270 kb, (ii) weniger als 200 kb, (iii) weniger als 150 kb, oder (iv) weniger als 100 kb beträgt.

10. Verfahren nach einem der Ansprüche 1 bis 9, wobei die mindestens 100 genomischen Zielbereiche für alle möglichen Paare zwischen der ersten Krebsart und den mindestens 10 anderen Krebsarten mindestens einen genomischen Zielbereich umfassen, der zwischen dem Paar von Krebsarten unterschiedlich methyliert ist.

11. Verfahren nach einem der Ansprüche 1 bis 9, wobei die mindestens 100 genomischen Zielbereiche umfassen:

(a) mindestens 20 % der genomischen Zielbereiche von der beliebigen einen der Listen 1-49 oder Komplementen davon;

(b) mindestens 20 % der genomischen Zielbereiche von der beliebigen einen der Listen 1-15 oder Komplementen davon;

(c) mindestens 20 % der genomischen Zielbereiche der Listen 1-15 oder Komplementen davon;

(d) mindestens 20 % der genomischen Zielbereiche von der beliebigen einen der Listen 16-32 oder Komplementen davon;

(e) mindestens 20 % der genomischen Zielbereiche der Listen 16-32 oder Komplementen davon;

(f) mindestens 20 % der genomischen Zielbereiche von der beliebigen einen der Listen 33-49 oder Komplementen davon;

(g) mindestens 20 % der genomischen Zielbereiche der Listen 33-49 oder Komplementen davon.

12. Verfahren nach einem der Ansprüche 1 bis 9, wobei:

(a) die Gesamtgröße der genomischen Zielbereiche weniger als 1100 kb beträgt;

(b) die Gesamtzahl der genomischen Zielbereiche weniger als 10.000 beträgt;

(c) die Köder-Oligonukleotide jeweils eine Länge von 45 bis 300 Nukleotidbasen, 75-200 Nukleotidbasen, 103-150 Nukleotidbasen oder etwa 120 Nukleotidbasen aufweisen; oder

(d) die Köder-Oligonukleotide eine Vielzahl von Sätzen von zwei oder mehr Köder-Oligonukleotiden umfassen, und ferner wobei:

(i) jedes Köder-Oligonukleotid innerhalb eines Satzes von Köder-Oligonukleotiden konfiguriert ist, um an den gleichen umgewandelten genomischen Zielbereich zu binden, oder konfiguriert ist, um an ein Nukleinsäuremolekül zu binden, das von dem genomischen Zielbereich abgeleitet ist,

(ii) jeder Satz von Köder-Oligonukleotiden Paare von Köder-Oligonukleotiden umfasst, wobei jedes Paar von Köder-Oligonukleotiden ein erstes Köder-Oligonukleotid und ein zweites Köder-Oligonukleotid umfasst,

(iii) jedes Köder-Oligonukleotid ein 5'-Ende und ein 3'-Ende umfasst,

(iv) für jedes Paar von Köder-Oligonukleotiden eine Sequenz von mindestens X Nukleotidbasen an dem 3'-Ende des ersten Köder-Oligonukleotids mit einer Sequenz von X Nukleotidbasen an dem 5'-Ende des zweiten Köder-Oligonukleotids identisch ist, und

(v) X mindestens 25, 30, 35, 40, 45, 50, 60, 70, 75 oder 100 beträgt, und optional wobei das erste Köder-Oligonukleotid eine Sequenz von mindestens 31, 40, 50 oder 60 Nukleotidbasen umfasst, die eine Sequenz des zweiten Köder-Oligonukleotids nicht überlappt.

**13.** Verfahren nach einem der Ansprüche 1 bis 9, wobei:

(a) die mindestens 100 genomischen Zielbereiche menschliche Sequenzen sind, und jedes der Köder-Oligonukleotide gestaltet ist, um Sequenzhomologie oder Sequenzkomplementarität mit weniger als 20 menschlichen Off-Target-Genombereichen aufzuweisen;

(b) jedes der Köder-Oligonukleotide mindestens 61 Nukleotide umfasst;

(c) jedes der Köder-Oligonukleotide weniger als 300 Nukleotide umfasst;

(d) mindestens 3 % der Köder-Oligonukleotide kein G (Guanin) umfassen;

(e) jedes der Köder-Oligonukleotide mehrere Bindungsstellen an Methylierungsstellen von umgewandelten cfDNA-Molekülen umfasst, wobei mindestens 83 % der mehreren Bindungsstellen ausschließlich entweder CpG oder CpA umfassen; oder

(f) jeder genomischer Zielbereich mindestens fünf Methylierungsstellen umfasst

**14.** Verfahren nach einem der Ansprüche 1 bis 13, wobei:

(i) die Köder-Oligonukleotide einen zusätzlichen Satz von Köder-Oligonukleotiden umfassen, die mit mindestens 100 genomischen Zielbereichen gemeinsam hybridisieren, die in einer zweiten Krebsart der mindestens 10 Krebsarten relativ zu einer unterschiedlichen Krebsart der mindestens 10 Krebsarten oder relativ zu Nicht-Krebs unterschiedlich methyliert sind;

(ii) die zweite Krebsart sich von der ersten Krebsart unterscheidet; und

(iii) für jede der mindestens 10 Krebsarten, Anwenden eines zweiten trainierten Klassifikators auf die Sequenzierungs-Reads, wobei der zweite Klassifikator (a) auf die mindestens 100 genomischen Zielbereiche des zusätzlichen Satzes von Köder-Oligonukleotiden für die zweite Krebsart beschränkt ist, und (b) eine Bewertungsziffer für jede der mindestens 10 Krebsarten zuordnet.

## Revendications

**1.** Procédé de détection de cellules d'un type de cancer chez un sujet, le procédé comprenant :

(i) la capture de fragments d'ADN acellulaire (ADNac) auprès du sujet ou de produits d'amplification de ceux-ci avec une composition comprenant une pluralité d'oligonucléotides appâts différents, dans lequel :

(a) chaque oligonucléotide appât dans la pluralité d'oligonucléotides appâts différents a une longueur d'au moins 45 nucléotides, facultativement 45 à 300 nucléotides ;

(b) les oligonucléotides appâts s'hybrident collectivement à au moins 100 régions génomiques cibles qui sont méthylées différentiellement dans un premier type de cancer d'au moins 10 types de cancer, par rapport à un type de cancer différent parmi les au moins 10 types de cancer ou par rapport à une absence de cancer ;

(ii) le séquençage des fragments d'ADNac capturés ou produits d'amplification de ceux-ci pour produire des lectures de séquençage, dans lequel l'ADNac est converti avant séquençage en traitant l'ADNac pour convertir des cytosines non méthylées en uraciles, et

(iii) pour chacun des au moins 10 types de cancer, l'application d'un classificateur entraîné aux lectures de séquençage, dans lequel le classificateur (a) est contraint aux au moins 100 régions génomiques cibles des oligonucléotides appâts pour le premier type de cancer, et (b) attribue un score pour chacun des au moins 10 types de cancer ; dans lequel le classificateur entraîné est un classificateur entraîné à l'aide de séquences d'ADN converti, dans lequel l'ADN converti se rapporte à de l'ADN qui a été traité pour convertir des cytosines non méthylées en uraciles, et

(iv) la détection des cellules du type de cancer chez le sujet en tant que type de cancer auquel est attribué le score le plus élevé ;

dans lequel les au moins 10 types de cancer sont choisis parmi cancer anorectal, cancer de la vessie, cancer urothélial, cancer du sein, cancer du col de l'utérus, cancer colorectal, cancer de la tête et du cou, cancer du foie, cancer des voies biliaires, cancer du poumon, cancer des ovaires, cancer du pancréas, cancer de la vésicule biliaire, cancer de la prostate, cancer du rein, sarcome, cancer de la thyroïde, cancer des voies digestives supérieures, et cancer de l'utérus.

2. Procédé selon la revendication 1, dans lequel la probabilité d'une fausse détection positive des cellules du type de cancer chez le sujet est inférieure à 1 % et la probabilité d'une détermination précise des cellules du type de cancer chez le sujet est d'au moins 40 %.

3. Procédé selon la revendication 1 ou 2, dans lequel :

(a) le premier type de cancer est un type de cancer de stade I, et la probabilité d'une attribution précise de type de cancer est d'au moins 70 % ;
(b) le premier type de cancer est un type de cancer de stade II, et la probabilité d'une attribution précise de type de cancer est d'au moins 85 % ;
(c) le premier type de cancer est un cancer de stade I ou de stade II, et la précision d'attribution du type de cancer est d'au moins 75 % ;
(d) la précision d'attribution de type de cancer est d'au moins 80 % ; ou
(e) les au moins 10 types de cancer comportent en outre cancer de l'œsophage, cancer épidermoïde du tractus gastro-intestinal supérieur, adénocarcinome pulmonaire, cancer du poumon à petites cellules, cancer épidermoïde du poumon, cancer neuroendocrinien, mélanome, néoplasme des cellules plasmatiques, myélome multiple, néoplasme myéloïde, lymphome et leucémie.

4. Procédé selon la revendication 3, dans lequel :

(a) le premier type de cancer est un cancer anorectal, les régions génomiques cibles sont choisies parmi les Listes 16 ou 33, et la sensibilité pour le cancer anorectal est d'au moins 65 % ou 75 % ;
(b) le premier type de cancer est un cancer de la vessie et urothélial, les régions génomiques cibles sont choisies parmi les Listes 1, 17 ou 34, et la sensibilité pour le cancer de la vessie et urothélial est d'au moins 40 % ;
(c) le premier type de cancer est un cancer du sein, les régions génomiques cibles sont choisies parmi les Listes 2, 18 ou 35, et la sensibilité pour le cancer du sein est d'au moins 20 % ;
(d) le premier type de cancer est un cancer du col de l'utérus, les régions génomiques cibles sont choisies parmi les Listes 3, 19 ou 36, et la sensibilité pour le cancer du col de l'utérus est d'au moins 25 % ;
(e) le premier type de cancer est un cancer colorectal, les régions génomiques cibles sont choisies parmi les Listes 4, 20 ou 37, et la sensibilité pour le cancer colorectal est d'au moins 55 % ;
(f) le premier type de cancer est un cancer de la tête et du cou, les régions génomiques cibles sont choisies parmi les Listes 5, 21 ou 38, et la sensibilité pour le cancer de la tête et du cou est d'au moins 70 % ;
(g) le premier type de cancer est un cancer du foie et des voies biliaires, les régions génomiques cibles sont choisies parmi les Listes 6, 22, ou 39, et la sensibilité pour le cancer du foie et des voies biliaires est d'au moins 75 % ;
(h) le premier type de cancer est un cancer du poumon, les régions génomiques cibles sont choisies parmi les Listes 7, 23 ou 40, et la sensibilité pour le cancer du poumon est d'au moins 55 % ;
(i) le premier type de cancer est un mélanome, les régions génomiques cibles sont choisies parmi les Listes 8, 24 ou 41, et la sensibilité pour le mélanome est d'au moins 30 % ;
(j) le premier type de cancer est un cancer des ovaires, les régions génomiques cibles sont choisies parmi les Listes 9, 25 ou 42, et la sensibilité pour le cancer des ovaires est d'au moins 70 % ;
(k) le premier type de cancer est un cancer du pancréas et de la vésicule biliaire, les régions génomiques cibles sont choisies parmi les Listes 10, 26 ou 43, et la sensibilité pour le cancer du pancréas et de la vésicule biliaire est d'au moins 60 % ;
(l) le premier type de cancer est un sarcome, les régions génomiques cibles sont choisies parmi les Listes 29 ou 46, et la sensibilité pour le sarcome est d'au moins 40 % ;
(m) le premier type de cancer est un cancer des voies digestives supérieures, les régions génomiques cibles sont choisies parmi les Listes 14, 31 ou 48, et la sensibilité pour le cancer des voies digestives supérieures est d'au moins 60 %.

**5.** Procédé selon la revendication 1, dans lequel les oligonucléotides appâts s'hybrident collectivement à au moins 300 régions génomiques cibles provenant des Listes 1 à 49, ou des compléments de celles-ci, qui sont méthylées différentiellement dans le premier type de cancer par rapport à un type de cancer différent des au moins 10 types de cancer ou par rapport à une absence de cancer.

**6.** Procédé selon l'une quelconque des revendications 1 à 5, dans lequel :

(i) la taille totale des au moins 100 régions génomiques cibles va de 4 MB à 50 kb ;
(ii) le sujet présente un risque élevé d'un ou plusieurs types de cancer ;
(iii) le sujet manifeste des symptômes associés à un ou plusieurs types de cancer ;
(iv) le sujet n'a pas été diagnostiqué d'un cancer ; ou
(v) le classificateur a été entraîné sur des séquences d'ADN converti dérivées d'au moins 100 sujets avec le premier type de cancer, au moins 100 sujets avec un deuxième type de cancer, et au moins 100 sujets dépourvus de cancer, et dans lequel le premier type de cancer, le deuxième type de cancer, et un troisième type de cancer sont choisis parmi les au moins 10 types de cancer.

**7.** Procédé selon l'une quelconque des revendications 1 à 6, dans lequel le classificateur a été entraîné sur des séquences d'ADN converti dérivées des régions génomiques cibles, et la pluralité d'oligonucléotides appâts différents s'hybrident collectivement à au moins 100 régions génomiques cibles provenant de chacune parmi une pluralité de Listes 33 à 49 ou des compléments de celles-ci ; facultativement dans lequel le classificateur entraîné détecte les cellules du type de cancer chez le sujet par :

(i) la génération d'un ensemble de caractéristiques pour l'échantillon, dans lequel chaque caractéristique dans l'ensemble de caractéristiques comprend une valeur numérique ;
(ii) l'entrée de l'ensemble de caractéristiques dans le classificateur, dans lequel le classificateur comprend un classificateur multinomial ;
(iii) en fonction de l'ensemble de caractéristiques, la détermination, au niveau du classificateur, d'un ensemble de scores de probabilité, dans lequel l'ensemble de scores de probabilité comprend un score de probabilité par classe de type de cancer et par absence de cancer ; et
(iv) seuillage de l'ensemble de scores de probabilité en fonction d'une ou plusieurs valeurs déterminées pendant l'entraînement du classificateur ; et facultativement dans lequel :

(a) l'ensemble de caractéristiques comprend un ensemble de caractéristiques binarisées ;
(b) la valeur numérique comprend une unique valeur binaire ;
(c) le classificateur multinomial comprend un ensemble de régression logistique multinomiale entraîné pour prédire un tissu source pour le cancer ; et/ou
(d) le procédé comprenant en outre la détermination d'une classification finale de cancer en fonction d'un différentiel de score de probabilité des deux premiers par rapport à une valeur minimale, dans lequel la valeur minimale correspond à un pourcentage prédéfini d'échantillons de cancer d'entraînement qui se sont vu attribuer le type de cancer correct en guise de score le plus élevé pendant l'entraînement du classificateur.

**8.** Procédé selon la revendication 1, comprenant en outre la sélection d'un agent thérapeutique anticancéreux pour administration au sujet en fonction de la détection des cellules du type de cancer chez le sujet ; facultativement dans lequel l'agent anticancéreux est un agent chimiothérapeutique choisi dans le groupe constitué par agents alkylants, antimétabolites, anthracyclines, antibiotiques antitumoraux, perturbateurs du cytosquelette (taxanes), inhibiteurs de topoisomérase, inhibiteurs mitotiques, corticostéroïdes, inhibiteurs de kinase, analogues de nucléotides, et agents à base de platine.

**9.** Procédé selon l'une quelconque des revendications 1 à 8, dans lequel la taille totale des au moins 100 régions génomiques cibles va de 50 kb à (i) moins de 270 kb, (ii) moins de 200 kb, (iii) moins de 150 kb, ou (iv) moins de 100 kb.

**10.** Procédé selon l'une quelconque des revendications 1 à 9, dans lequel les au moins 100 régions génomiques cibles comprennent, pour toutes les paires possibles entre le premier type de cancer et les au moins 10 autres types de cancer, au moins une région génomique cible qui est méthylée différentiellement entre la paire de types de cancer.

**11.** Procédé selon l'une quelconque des revendications 1 à 9, dans lequel les au moins 100 régions génomiques cibles comprennent :

(a) au moins 20 % des régions génomiques cibles de l'une quelconque des Listes 1 à 49, ou des compléments de celles-ci ;

(b) au moins 20 % des régions génomiques cibles de l'une quelconque des Listes 1 à 15, ou des compléments de celles-ci ;

(c) au moins 20 % des régions génomiques cibles des Listes 1 à 15, ou des compléments de celles-ci ;

(d) au moins 20 % des régions génomiques cibles de l'une quelconque des Listes 16 à 32, ou des compléments de celles-ci ;

(e) au moins 20 % des régions génomiques cibles des Listes 16 à 32, ou des compléments de celles-ci ;

(f) au moins 20 % des régions génomiques cibles de l'une quelconque des Listes 33 à 49, ou des compléments de celles-ci ;

(g) au moins 20 % des régions génomiques cibles des Listes 33 à 49, ou des compléments de celles-ci.

**12.** Procédé selon l'une quelconque des revendications 1 à 9, dans lequel :

(a) la taille totale des régions génomiques cibles est inférieure à 1100 kb ;

(b) le nombre total de régions génomiques cibles est inférieur à 10 000 ;

(c) les oligonucléotides appâts ont chacun une longueur de 45 à 300 bases nucléotidiques, 75 à 200 bases nucléotidiques, 103 à 150 bases nucléotidiques, ou environ 120 bases nucléotidiques ; ou

(d) les oligonucléotides appâts comprennent une pluralité d'ensembles de deux oligonucléotides appâts ou plus, et dans lequel en outre :

(i) chaque oligonucléotide appât au sein d'un ensemble d'oligonucléotides appâts est configuré pour se lier à la même région génomique cible convertie ou configuré pour se lier à une molécule d'acide nucléique dérivée de la région génomique cible,

(ii) chaque ensemble d'oligonucléotides appâts comprend des paires d'oligonucléotides appâts, chaque paire d'oligonucléotides appâts comprend un premier oligonucléotide appât et un second oligonucléotide appât,

(iii) chaque oligonucléotide appât comprend une extrémité 5' et une extrémité 3',

(iv) pour chaque paire d'oligonucléotides appâts une séquence d'au moins X bases nucléotidiques au niveau de l'extrémité 3' du premier oligonucléotide appât est identique à une séquence de X bases nucléotidiques au niveau de l'extrémité 5' du second oligonucléotide appât, et

(v) X vaut au moins 25, 30, 35, 40, 45, 50, 60, 70, 75 ou 100, et facultativement dans lequel le premier oligonucléotide appât comprend une séquence d'au moins 31, 40, 50 ou 60 bases nucléotidiques qui ne chevauche pas une séquence du second oligonucléotide appât.

**13.** Procédé selon l'une quelconque des revendications 1 à 9, dans lequel :

(a) les au moins 100 régions génomiques cibles sont des séquences humaines, et chacun des oligonucléotides appâts est conçu pour avoir une homologie de séquence ou une complémentarité de séquence avec moins de 20 régions génomiques humaines hors cible ;

(b) chacun des oligonucléotides appâts comprend au moins 61 nucléotides ;

(c) chacun des oligonucléotides appâts comprend moins de 300 nucléotides ;

(d) au moins 3 % des oligonucléotides appâts ne comprennent aucune G (Guanine) ;

(e) chacun des oligonucléotides appâts comprend de multiples sites de liaison à des sites de méthylation de molécules d'ADNac converti, dans lequel au moins 83 % des multiples sites de liaison comprennent exclusivement soit CpG soit CpA ; ou

(f) chaque région génomique cible comprend au moins cinq sites de méthylation

**14.** Procédé selon l'une quelconque des revendications 1 à 13, dans lequel :

(i) les oligonucléotides appâts comprennent un ensemble supplémentaire d'oligonucléotides appâts qui s'hybrident collectivement à au moins 100 régions génomiques cibles qui sont méthylées différentiellement dans un deuxième type de cancer des au moins 10 types de cancer, par rapport à un type de cancer différent des au moins 10 types de cancer ou par rapport à une absence de cancer ;

(ii) le deuxième type de cancer est différent du premier type de cancer ; et

(iii) pour chacun des au moins 10 types de cancer, on applique un second classificateur entraîné aux lectures de séquençage, dans lequel le second classificateur (a) est contraint aux au moins 100 régions génomiques cibles de l'ensemble supplémentaire d'oligonucléotides appâts pour le deuxième type de cancer, et (b) attribue un score pour chacun des au moins 10 types de cancer.

FIG. 1A

FIG. 1B

Binary targets (pull down both types)

OR

semi-binary targets (pull down one type of fragment)

Methylated    Un-methylated

**FIG. 1C**

FIG. 2

Generate data structure for a control group
<u>300</u>

Generate set of methylation state vectors
for a control group
<u>100</u>

For each methylation state vector, subdivide into
strings of methylation sites
<u>310</u>

Tally strings for each position and methylation
state combination
<u>320</u>

Create data structure storing counts of all
possible strings from the control group
<u>330</u>

Validate data structure consistency
<u>340</u>

**FIG. 3A**

Validate data structure consistency
340

Generate set of methylation state vectors
for a validation group
100

Calculate a p-value score for each methylation
state vector with control group data structure
350

Build cumulative density function (CDF) of all p-
value's from the validation group
360

Validate consistency of CDF
370

**FIG. 3B**

Identifying target genomic regions
400

Generate set of methylation state vectors from a sample
100

For each methylation state vector, enumerate all possible methylation state vectors at that position
410

Calculate probabilities for all possible methylation state vectors from control group data structure
420

Use sliding window for methylation state vectors with greater than a threshold number of methylation sites
435

Calculate a p-value score for each methylation state vector based on calculated probabilities
430

Filter set resulting in a subset of anomalously methylated vectors based on p-value scores being below a threshold value
440

Identify fragments indicative of cancer from filtered set
450

Identify genomic regions indicative of cancer from the fragments
460

Identify genomic regions indicative of smoking
465

Remove probes having off-target regions more than a threshold number
475

Design probes targeting the genomic regions
470

## FIG. 4

EP 3 921 444 B1

505

Test Methylation State Vector

Calculate P-Value with 500
Markov Chain Model

$< M_{23}, M_{24}, M_{25}, U_{26} >$

410, 420

| $P$ | $< M_{23}, M_{24}, M_{25}, M_{26} >$ |
|-----|------|
| $P$ | $< M_{23}, M_{24}, M_{25}, U_{26} >$ |

$= P(M_{26} \mid M_{23}, M_{24}, M_{25}) * P(M_{25} \mid M_{23}, M_{24}) * P(M_{24} \mid M_{23}) * P(M_{23})$
$\approx P(M_{26} \mid M_{24}, M_{25}) * P(M_{25} \mid M_{23}, M_{24}) * P(M_{24} \mid M_{23}) * P(M_{23})$

o
o
o

| $P$ | $< U_{23}, U_{24}, U_{25}, U_{26} >$ |
|-----|------|

$= P(U_{26} \mid U_{23}, U_{24}, U_{25}) * P(U_{25} \mid U_{23}, U_{24}) * P(U_{24} \mid U_{23}) * P(U_{23})$
$\approx P(U_{26} \mid U_{24}, U_{25}) * P(U_{25} \mid U_{23}, U_{24}) * P(U_{24} \mid U_{23}) * P(U_{23})$

515 — Probabilities of Possible
Methylation State Vectors

430

| $p$-value | $< M_{23}, M_{24}, M_{25}, U_{26} >$ |
|-----------|------|

$= \sum [\, All\ probabilities \leq P(\, <M_{23}, M_{24}, M_{25}, U_{26}> )\,]$

525 — P-Value of Test Methylation
State Vector

FIG. 5

Training a classifier
600

```
Obtain anomalous fragments from a non-cancer training group
and a cancer training group
605
```

```
For each methylation state vector from the two training groups,
determine if hypomethylated or hypermethylated
610
```

```
For each methylation site in the genome, generate a
hypomethylation score and a hypermethylation score
620
```

```
For each methylation state vector, generate an aggregate
hypomethylation score and an aggregate hypermethylation score
630
```

```
For each subject, rank methylation state vectors by aggregate
hypomethylation score and by aggregate hypermethylation score
640
```

```
For each subject, generate a feature vector based on rankings
650
```

```
Train to distinguish feature vectors from non-cancer training
group and from cancer training group
660
```

```
Compute information gain of each CpG site
670
```

# FIG. 6A

Computing pairwise information gain
680

For each sample, define a feature vector for each cancer type at each region based on count of fragments above a log-likelihood ratio above various thresholds
690

Calculate an informative score for each CpG site describing ability to distinguish between pairs of cancer type
695

# FIG. 6B

Start

105 — Extract nucleic acids from a test sample

110 — Treat extracted nucleic acid to convert unmethylated cytosine to uracil

115 — Prepare library using adapter ligation

120 — Hybridize nucleic acids using hybridization probes

125 — Perform enrichment on nucleic acid fragments

130 — Generate sequence reads of the nucleic acid

140 — Generate methylation state vector for nucleic acids

End

**FIG. 7A**

100 ⟶ Generate a methylation state vector from a cell-free (cf) DNA fragment in sample

Methyl Groups ⟶ 114

112 ⟶ Fragment cfDNA

⟩ -C-G- ⟩ -C-G- ⟩ -C-G-

⟶ 120

122 ⟶ Converted cfDNA

-C-G- -U-G- -C-G-

⟶ 130, 140

142 ⟶ Sequence Read

| Methylated | Unmethylated | Methylated |

-C-G- -T-G- -C-G-

144 ⟶ Reference Genome

-C-G- -C-G- -C-G-

23      24      25

⟶ 160

152 ⟶ Methylation State Vector

$< M_{23}, U_{24}, M_{25} >$

FIG. 7B

FIG. 8A

cfDNA Concentration Per Sample

190

FIG. 8B

FIG. 9

**FIG. 10A**

**FIG. 10B**

FIG. 11

**FIG. 12**

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010037001 A
- WO 2011127136 A
- US 20160340740 A
- WO 2014043763 A
- US 352602
- US 20190287652 A1
- US 2019022122 W

**Non-patent literature cited in the description**

- **LIU, L. et al.** Targeted methylation sequencing of plasma cell-free DNA for cancer detection and classification. *ANNALS OF ONCOLOGY,* 01 June 2018, vol. 29
- **PIDSLEY, R. et al.** Critical evaluation of the Illumina MethylationEPIC BeadChip microarray for whole-genome DNA methylation profiling. *Genome Biology,* 2016, vol. 17, 208
- AJCC Cancer Staging Manual. Springer, 2017
- **RIEDMILLER M ; BRAUN H.** RPROP - A Fast Adaptive Learning Algorithm. *Proceedings of the International Symposium on Computer and Information Science,* 1992, vol. VII